# EUROPEAN PATENT SPECIFICATION

(11) **EP 1 187 816 B1**
(45) Date of publication and mention of the grant of the patent: **20.12.2006**
(21) Application number: 00940569.7
(22) Date of filing: 19.06.2000
(51) Int. Cl.: C07D 239/42, C07D 409/04, C07D 401/04, C07D 403/12, A61K 31/505, A61K 31/506, A61P 9/14

(54) **5-CYANO-2-AMINOPYRIMIDINE DERIVATIVES**
5-CYANO-2-AMINOPYRIMIDINE DERIVATE
DERIVES DE 5-CYANO-2-AMINOPYRIMIDINE

(30) Priority: 18.06.1999 GB 9914258
(43) Date of publication of application: 20.03.2002
(73) Proprietor: Celltech R&D Limited, Slough, Berkshire SL1 3WE (GB)
(72) Inventor: BATCHELOR, Mark, James, Oxfordshire OX9 5QN (GB); MOFFAT, David, Festus, Charles, Berkshire SL6 2YX (GB); DAVIS, Jeremy, Martin, Berkshire RG40 1RE (GB); HUTCHINGS, Martin, Clive, Berkshire RG40 4HN (GB)
(74) Representative: Mercer, Christopher Paul
(86) International application number: PCT/GB2000/002382
(87) International publication number: WO 2000/078731

(56) References cited:
- EP-A- 0 135 472
- WO-A-97/09325

## Description

This invention relates to substituted 5-cyano-2-aminopyrimidines, to processes for their preparation, to pharmaceutical compositions containing them, and to their use in medicine.

Angiogenesis, the growth of capillaries from existing blood vessels, is an essential process in normal embryonic development, tissue repair and some aspects of female reproductive function. It is also associated with the development of several pathological disorders including solid tumour growth, metastasis, psoriasis and rheumatoid arthritis, as well as diabetic retinopathy and age related macular degeneration [Folkman, Nature Medicine, (1995) 1, 27-310].

Several growth factors have been shown to mediate angiogenesis through alteration of vascular permeability, including vascular endothelial growth factor [VEGF; G. Breier *et al.,* Trends in Cell Biology, (1996), 6, 454-6], platelet derived growth factor (PDGF) and acidic and basic fibroblast growth factors (a & b FGF).

VEGF in dimeric form is a ligand that binds to two transmembrane tyrosine kinase associated receptors, expressed exclusively on proliferating endothelial cells, KDR (Flk-1 in mice) also known as VEGFR-2, and Flt-1 also known as VEGFR-1. Binding of VEGF to KDR/Flk and Fit leads to receptor dimerisation, kinase activation, autophosphorylation of the receptor and phosphorylation of intracellular substrates. An analogous series of events ensues after ligand occupancy of the more widely expressed tyrosine kinase associated FGFr receptor by aFGF or bFGF. Thus receptor tyrosine kinase activity initiates a cellular signalling pathway leading to proliferation.

Antagonism of VEGF with antibody completely suppresses neovascularisation and growth of human rhabdomyosarcoma A673 speroids in athymic mice [Borgstrom *et al,* Cancer Res., (1996), 56 4032-4039]. Suppression of bFGF gene expression by interferons a and b inhibits capillary density in mice, leading to pancreatic eyelet tumour suppression [Folkman *et al,* Proc. Natl. Acad.Sci. (1996), 93, 2002 and Singh *et al* Proc.Natl. Acad. Sci. (1995), 92, 10457). Other receptor associated kinases such as PDGF and EGFr may also have some role in mediating angiogenesis.

We have now found a series of substituted 5-cyano-2-aminopyrimidines which are potent and selective inhibitors of receptor tyrosine kinases involved in angiogenesis, especially KDR kinase and/or FGFr kinase. Selective inhibition of these kinases can be expected to have a beneficial effect and the compounds are thus of use in the prophylaxis and treatment of disease states associated with angiogenesis, as described hereinafter.

According to one aspect of the invention, there is provided the use of a compound of formula (1) 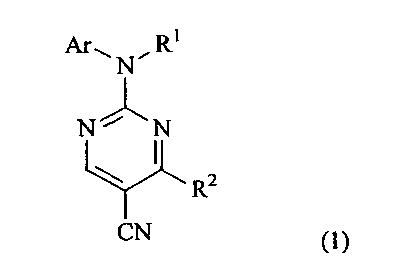 or a salt, solvate, hydrate or N-oxide thereof, for the manufacture of a medicament for the treatment of a disease state associated with angiogenesis
wherein
Ar is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R⁴ or -Alk(R⁴)ₘ;
R¹ is a hydrogen atom or a straight or branched chain C₁₋₆alkyl group;
R² is a -X¹-R³ group where X¹ is a direct bond; and
R³ is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R^{4b} or -Alk(R^{4b})ₘ;
R⁴ and R^{4b} are each a halogen atom, or an amino (-NH₂), -NHR⁵, nitro, cyano, hydroxyl (-OH), -OR⁵, formyl, carboxyl (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵ group, or a C₆₋₁₂aromatic group, a C₁₋₉heteroaromatic group containing one, two, three or four heteroatoms selected from oxygen, sulphur or nitrogen atoms, a C₃₋₁₀cycloaliphatic group, or a C₃₋₁₀heterocycloaliphatic group containing one, two, three or four heteroatoms or heteroatom-containing groups selected from -O- or -S-atoms or -N(R⁶)-, -C(O)-, -C(S)-, -S(O)- or -S(O₂)-, said aromatic or heteroaromatic groups optionally substituted with one, two or three R^{4a} atoms or groups, said cycloaliphatic or heterocycloaliphatic groups optionally substituted with one, two, three or more substituents selected from halogen atoms, hydroxyl, C₁₋₆alkoxy, thiol, C₁₋₆alkylthio, hydroxy, C₁₋₆alkyl, hydroxyethyl, -CN, -NO₂, -NHR⁵ or -N(R⁵)₂ groups, provided that two R⁴ or -Alk(R⁴)ₘ or two R^{4b} or -Alk(R^{4b})ₘ substituents may be linked together to form a C₂₋₆-alkylenedioxy group;
Alk¹ is a straight or branched C₁₋₈alkyl group, a C₆₋₁₂arylC₁₋₈alkyl group, a C₆₋₁₂aryl group, a C₆₋₁₂aryloxyC₁₋₈alkyl group, a C₁₋₈alkanoyloxyC₁₋₈alkyl group, or a C₆₋₁₂aroyloxyC₁₋₈alkyl group, where said groups are optionally substituted with one or more R⁴ substituents;
R⁵ is a -Alk(R⁴)ₘ, C₆₋₁₂aromatic group, a C₁₋₉heteroaromatic group containing one, two, three or four heteroatoms selected from oxygen, sulphur or nitrogen atoms, said aromatic or heteroaromatic group optionally substituted with one, two or three R^{4a} atoms or groups;
Alk is a straight or branched C₁₋₆alkylene, C₂₋₆alkenylene or C₂₋₆alkynylene chain, optionally interrupted by one, two or three -O- or -S- atoms or groups selected from -S(O)-, -S(O)₂- or -N(R⁶)-;
R^{4a} is fluorine, chlorine, bromine or iodine atoms, or C₁₋₆alkyl, C₁₋₆alkylamino, hydroxyC₁₋₆alkyl, hydroxyC₂₋₆alkoxy, C₁₋₆alkylthiol, C₁₋₆alkoxy, C₅₋₇cycloalkoxy, haloC₁₋₆alkyl, amino (-NH₂), aminoC₁₋₆alkyl, C₁₋₆dialkylamino, aminoC₁₋₆alkoxy, C₁₋₆alkylaminoC₁₋₆alkoxy, diC₁₋₆alkylaminoC₁₋₆alkoxy, imido, 1,1,3-trioxobenzo[d]thiazolidino, nitro, cyano, hydroxyl (-OH), formyl [HC(O)-], carboxyl (-CO₂H), -CO₂Alk¹, C₁₋₆ alkanoyl, thiol (-SH), thioC₁₋₆alkyl, -SC(NH₂)NH₂, sulphonyl (-SO₃H), C₁₋₆alkylsulphonyl, aminosulphonyl (-SO₂NH₂), C₁₋₆alkylaminosulphonyl, C₁₋₆dialkylaminosulphonyl, phenylaminosulphonyl, carboxamido (-CONH₂), C₁₋₆alkylaminocarbonyl, C₁₋₆dialkylaminocarbonyl, sulphonylamino (-NHSO₂H), C₁₋₆alkylsulphonylamino, C₁₋₆dialkylsulphonylamino, phenylsulphonylamino, 2-nitrophenylsulphonylamino, aminosulphonylamino (-NHSO₂NH₂), C₁₋₆alkylaminosulphonylamino, C₁₋₆dialkylaminosulphonylamino, phenylaminosulphonylamino, aminocarbonylamino, C₁₋₆alkylaminocarbonylamino C₁₋₆dialkylaminocarbonylamino, phenylaminocarbonylamino, C₁₋₆alkanoylamino, phenylcarbonylamino, C₁₋₆alkanoylaminoC₁₋₆alkyl, C₁₋₆alkoxycarbonylamino, heteroC₃₋₆cycloalkyl, 4-(C₁₋₆alkyl)piperazinyl, heteroC₃₋₆cycloalkylC₁₋₆alkyl, 4-(C₁₋₆alkyl)piperazinylC₁₋₆alkyl, heteroC₃₋₆cycloalkylC₁₋₆alkoxy, heteroC₃₋₆alkylC₁₋₆alkylamino, heteroC₃₋₆cycloalkylamino, tetrazolyl, imidazolyl, triazolyl, imidazolylC₁₋₆alkyl, imidazolylC₁₋₆alkoxy, triazolylC₁₋₆alkoxy, imidazolylaminoC₁₋₆alkoxy, phenylamino, benzylamino, benzyloxy, or pyridylmethylamino group;
R⁶ is a hydrogen atom or a straight or branched chain C₁₋₆alkyl group;
m is zero or an integer 1, 2 or 3.

According to a further aspect of the invention, there is provided a compound of formula (1): 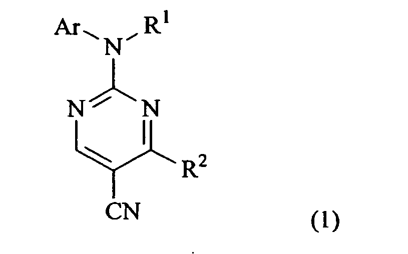 wherein
Ar is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one, two or three substituents, each represented by an atom or group -R⁴ or -Alk(R⁴)ₘ;
R¹ is a hydrogen atom or a straight or branched chain C₁₋₆alkyl group;
R² is a -X¹-R³ group where X¹ is a direct bond; and
R³ is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R^{4b} or -Alk(R^{4b})ₘ;
R⁴ and R^{4b} are each a halogen atom, or an amino (-NH₂), -NHR⁵, nitro, cyano, hydroxyl (-OH), -OR⁵, formyl, carboxyl (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵ group, or a C₆₋₁₂aromatic group, a C₁₋₉heteroaromatic group containing one, two, three or four heteroatoms selected from oxygen, sulphur or nitrogen atoms, a C₃₋₁₀cycloaliphatic group, or a C₃₋₁₀heterocycloaliphatic group containing one, two, three or four heteroatoms or heteroatom-containing groups selected from -O- or -S-atoms or -N(R⁶)-, -C(O)-, -C(S)-, -S(O)- or -S(O₂)-, said aromatic or heteroaromatic groups optionally substituted with one, two or three R^{4a} atoms or groups, said cycloaliphatic or heterocycloaliphatic groups optionally substituted with one, two, three or more substituents selected from halogen atoms, hydroxyl, C₁₋₆alkoxy, thiol, C₁₋₆alkylthio, hydroxy, C₁₋₆alkyl, hydroxyethyl, -CN, -NO₂, -NHR⁵ or -N(R⁵)₂ groups, provided that two R⁴ or -Alk(R⁴)ₘ or two R^{4b} or -Alk(R^{4b})ₘ substituents may be linked together to form a C₂₋₆-alkylenedioxy group;
Alk¹ is a straight or branched C₁₋₈alkyl group, a C₆₋₁₂arylC₁₋₈alkyl group, a C₆₋₁₂aryl group, a C₆₋₁₂aryloxyC₁₋₈alkyl group, a C₁₋₈alkanoyloxyC₁₋₈alkyl group, or a C₆₋₁₂aroyloxyC₁₋₈alkyl group, where said groups are optionally substituted with one or more R⁴ substituents;
R⁵ is a -Alk(R⁴)ₘ, C₆₋₁₂aromatic group, a C₁₋₉heteroaromatic group containing one, two, three or four heteroatoms selected from oxygen, sulphur or nitrogen atoms, said aromatic or heteroaromatic group optionally substituted with one, two or three R^{4a} atoms or groups;
Alk is a straight or branched C₁₋₆alkylene, C₂₋₆alkenylene or C₂₋₆alkynylene chain, optionally interrupted by one, two or three -O- or -S- atoms or groups selected from -S(O)-, -S(O)₂- or -N(R⁶)-;
R^{4a} is fluorine, chlorine, bromine or iodine atoms, or C₁₋₆alkyl, C₁₋₆alkylamino, hydroxyC₁₋₆alkyl, hydroxyC₂₋₆alkoxy, C₁₋₆alkylthiol, C₁₋₆alkoxy, C₅₋₇cycloalkoxy, haloC₁₋₆alkyl, amino (-NH₂), aminoC₁₋₆alkyl, C₁₋₆dialkylamino, aminoC₁₋₆alkoxy, C₁₋₆alkylaminoC₁₋₆alkoxy, diC₁₋₆alkylaminoC₁₋₆alkoxy, imido, 1,1,3-trioxobenzo[d]thiazolidino, nitro, cyano, hydroxyl (-OH), formyl [HC(O)-], carboxyl (-CO₂H), -CO₂Alk¹, C₁₋₆ alkanoyl, thiol (-SH), thioC₁₋₆alkyl, -SC(NH₂)NE₂, sulphonyl (-SO₃H), C₁₋₆alkylsulphonyl, aminosulphonyl (-SO₂NH₂), C₁₋₆alkylaminosulphonyl, C₁₋₆dialkylaminosulphonyl, phenylaminosulphonyl, carboxamido (-CONH₂), C₁₋₆alkylaminocarbonyl, C₁₋₆dialkylaminocarbonyl, sulphonylamino (-NHSO₂H), C₁₋₆alkylsulphonylamino, C₁₋₆dialkylsulphonylamino, phenylsulphonylamino, 2-nitrophenylsulphonylamino, aminosulphonylamino (-NHSO₂NH₂), C₁₋₆alkylaminosulphonylamino, C₁₋₆dialkylaminosulphonylamino, phenylaminosulphonylamino, aminocarbonylamino, C₁₋₆alkylaminocarbonylamino C₁₋₆dialkylaminocarbonylamino, phenylaminocarbonylamino, C₁₋₆alkanoylamino, phenylcarbonylamino, C₁₋₆alkanoylaminoC₁₋₆alkyl, C₁₋₆alkoxycarbonylamino, heteroC₃₋₆cycloalkyl, 4-(C₁₋₆alkyl)piperazinyl, heteroC₃₋₆cycloalkylC₁₋₆alkyl, 4-(C₁₋₆alkyl)piperazinylC₁₋₆alkyl, heteroC₃₋₆cycloalkylC₁₋₆alkoxy, heteroC₃₋₆alkylC₁₋₆alkylamino, heteroC₃₋₆cycloalkylamino, tetrazolyl, imidazolyl, triazolyl, imidazolylC₁₋₆alkyl, imidazolylC₁₋₆alkoxy, triazolylC₁₋₆alkoxy, imidazolylaminoC₁₋₆alkoxy, phenylamino, benzylamino, benzyloxy, or pyridylmethylamino group;
R⁶ is a hydrogen atom or a straight or branched chain C₁₋₆alkyl group;
m is zero or an integer 1, 2 or 3;
and the salts, solvates, hydrates and N-oxides thereof.

According to a further aspect of the invention, there is provided a compound of formula (1) 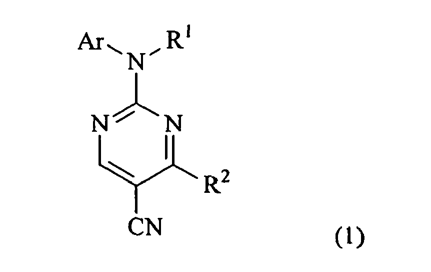 for treating a disease associated with KDR kinase and/or FGFr kinase
wherein
Ar is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R⁴ or -Alk(R⁴)ₘ;
R¹ is a hydrogen atom or a straight or branched chain C₁₋₆alkyl group;
R² is a -X¹-R³ group where X¹ is a direct bond; and
R³ is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R^{4b} or -Alk(R^{4b})ₘ;
R⁴ and R^{4b} are each a halogen atom, or an amino (-NH₂), -NHR⁵, nitro, cyano, hydroxyl (-OH), -OR⁵, formyl, carboxyl (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵ group, or a C₆₋₁₂aromatic group, a C₁₋₉heteroaromatic group containing one, two, three or four heteroatoms selected from oxygen, sulphur or nitrogen atoms, a C₃₋₁₀cycloaliphatic group, or a C₃₋₁₀heterocycloaliphatic group containing one, two, three or four heteroatoms or heteroatom-containing groups selected from -O- or -S-atoms or -N(R⁶)-, -C(O)-, -C(S)-, -S(O)- or -S(O₂)-, said aromatic or heteroaromatic groups optionally substituted with one, two or three R^{4a} atoms or groups, said cycloaliphatic or heterocycloaliphatic groups optionally substituted with one, two, three or more substituents selected from halogen atoms, hydroxyl, C₁₋₆alkoxy, thiol, C₁₋₆alkylthio, hydroxy, C₁₋₆alkyl, hydroxyethyl, -CN, -NO₂, -NHR⁵ or -N(R⁵)₂ groups, provided that two R⁴ or -Alk(R⁴)ₘ or two R^{4b} or -Alk(R^{4b})ₘ substituents may be linked together to form a C₂₋₆-alkylenedioxy group;
Alk¹ is a straight or branched C₁₋₈alkyl group, a C₆₋₁₂arylC₁₋₈alkyl group, a C₆₋₁₂aryl group, a C₆₋₁₂aryloxyC₁₋₈alkyl group, a C₁₋₈alkanoyloxyC₁₋₈alkyl group, or a C₆₋₁₂aroyloxyC₁₋₈alkyl group, where said groups are optionally substituted with one or more R⁴ substituents;
R⁵ is a -Alk(R⁴)ₘ, C₆₋₁₂aromatic group, a C₁₋₉heteroaromatic group containing one, two, three or four heteroatoms selected from oxygen, sulphur or nitrogen atoms, said aromatic or heteroaromatic group optionally substituted with one, two or three R^{4a} atoms or groups;
Alk is a straight or branched C₁₋₆alkylene, C₂₋₆alkenylene or C₂₋₆alkynylene chain, optionally interrupted by one, two or three -O- or -S- atoms or groups selected from -S(O)-, -S(O)₂- or -N(R⁶)-;
R^{4a} is fluorine, chlorine, bromine or iodine atoms, or C₁₋₆alkyl, C₁₋₆alkylamino, hydroxyC₁₋₆alkyl, hydroxyC₂₋₆alkoxy, C₁₋₆alkylthiol, C₁₋₆alkoxy, C₅₋₇cycloalkoxy, haloC₁₋₆alkyl, amino (-NH₂), aminoC₁₋₆alkyl, C₁₋₆dialkylamino, aminoC₁₋₆alkoxy, C₁₋₆alkylaminoC₁₋₆alkoxy, diC₁₋₆alkylaminoC₁₋₆alkoxy, imido, 1,1,3-trioxobenzo[d]thiazolidino, nitro, cyano, hydroxyl (-OH), formyl [HC(O)-], carboxyl (-CO₂H), -CO₂Alk¹, C₁₋₆ alkanoyl, thiol (-SH), thioC₁₋₆alkyl, -SC(NH₂)NH₂, sulphonyl (-SO₃H), C₁₋₆alkylsulphonyl, aminosulphonyl (-SO₂NH₂), C₁₋₆alkylaminosulphonyl, C₁₋₆dialkylaminosulphonyl, phenylaminosulphonyl, carboxamido (-CONH₂), C₁₋₆alkylaminocarbonyl, C₁₋₆dialkylaminocarbonyl, sulphonylamino (-NHSO₂H), C₁₋₆alkylsulphonylamino, C₁₋₆dialkylsulphonylamino, phenylsulphonylamino, 2-nitrophenylsulphonylamino, aminosulphonylamino (-NHSO₂NH₂), C₁₋₆alkylaminosulphonylamino, C₁₋₆dialkylaminosulphonylamino, phenylaminosulphonylamino, aminocarbonylamino, C₁₋₆alkylaminocarbonylamino C₁₋₆dialkylaminocarbonylamino, phenylaminocarbonylamino, C₁₋₆alkanoylamino, phenylcarbonylamino, C₁₋₆alkanoylaminoC₁₋₆alkyl, C₁₋₆alkoxycarbonylamino, heteroC₃₋₆cycloalkyl, 4-(C₁₋₆alkyl)piperazinyl, heteroC₃₋₆cycloalkylC₁₋₆alkyl, 4-(C₁₋₆alkyl)piperazinylC₁₋₆alkyl, heteroC₃₋₆cycloalkylC₁₋₆alkoxy, heteroC₃₋₆alkylC₁₋₆alkylamino, heteroC₃₋₆cycloalkylamino, tetrazolyl, imidazolyl, triazolyl, imidazolylC₁₋₆alkyl, imidazolylC₁₋₆alkoxy, triazolylC₁₋₆alkoxy, imidazolylaminoC₁₋₆alkoxy, phenylamino, benzylamino, benzyloxy, or pyridylmethylamino group;
R⁶ is a hydrogen atom or a straight or branched chain C₁₋₆alkyl group;
m is zero or an integer 1, 2 or 3;
and the salts, solvates, hydrates and N-oxides thereof.

When Ar in the compounds of formula (1) is an aromatic group it may be an optionally substituted monocyclic or bicyclic fused ring C₆₋₁₂aromatic group, such as an optionally substituted phenyl, 1- or 2-naphthyl, 1- or 2-tetrahydronaphthyl, indanyl or indenyl group.

In general, the heteroaromatic group may be for example an optionally substituted monocyclic heteroaromatic, or a bicyclic or tricyclic fused-ring heteroaromatic group. Monocyclic heteroaromatic groups include for example five- or six-membered heteroaromatic groups containing one, two, three or four heteroatoms selected from oxygen, sulphur or nitrogen atoms. Bicyclic heteroaromatic groups include for example nine- to thirteen-membered fused-ring heteroaromatic groups containing one, two or more heteroatoms selected from oxygen, sulphur or nitrogen atoms. Tricyclic heteroaromatic groups include for example ten- to fourteen-membered fused-ring heteroaromatic groups containing one, two or more heteroatoms selected from oxygen, sulphur or nitrogen atoms. The heteroaromatic group may be attached to the remainder of the compound of formula (1) through any of its ring carbon atoms.

Particular examples of heteroaromatic groups represented by Ar include optionally substituted pyrrolyl, furyl, thienyl, imidazolyl, oxazolyl, isoxazolyl, thiazolyl, isothiazolyl, pyrazolyl, 1,2,3-triazolyl, 1,2,4-triazolyt, 1,3,4-triazolyl, 1,2,5-triazolyl, 1,2,3-oxadiazolyl, 1,2,4-oxadiazolyl, 1,2,5-oxadiazolyl, 1,3,4-oxadiazolyl, 1,3,4-thiadiazole, pyridyl, pyrimidinyl, pyridazinyl, pyrazinyl, 1,3,5-triazinyl, 1,2,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, indazolyl, imidazo[1,2-a]pyridyl, benzothiazolyl, benzoxazolyl, quinolizinyl, quinazolinyl, phthalazinyl, quinoxalinyl, cinnolinyl, naphthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

When in the group -Alk(R⁴)ₘ m is an integer 1, 2 or 3, it is to be understood that the substituent or substituents R⁴ may be present on any suitable carbon atom in -Alk. Where more than one R⁴ substituent is present these may be the same or different and may be present on the same or different atom in -Alk or in R⁴ as appropriate. Thus for example, - Alk(R⁴)ₘ may represent a -CH(R⁴)₂ group, such as a -CH(OH)Ar¹ group where Ar¹ is an aryl or heteroaryl group as defined below. Clearly, when m is zero and no substituent R⁴ is present the alkylene, alkenylene or alkynylene chain represented by Alk becomes an alkyl, alkenyl or alkynyl group.

When R⁴ is a halogen atom it may be for example a fluorine, chlorine, bromine, or iodine atom.

Alk¹ is a straight or branched, optionally substituted C₁₋₈ alkyl group such as a methyl, ethyl, n-propyl, i-propyl, n-butyl, i-butyl, s-butyl or t-butyl group; a C₆₋₁₂arylC₁₋₈alkyl group such as an optionally substituted benzyl, phenylethyl, phenylpropyl, 1-naphthylmethyl or 2-naphthylmethyl group; a C₆₋₁₂aryl group such as an optionally substituted phenyl, 1-naphthyl or 2-naphthyl group; a C₆₋₁₂ aryloxyC₁₋₈alkyl group such as an optionally substituted phenyloxymethyl, phenyloxyethyl, 1-naphthyloxymethyl, or 2-naphthyloxymethyl group; an optionally substituted C₁₋₈alkanoyloxyC₁₋₈alkyl group, such as a pivaloyloxymethyl, propionyloxyethyl or propionyloxypropyl group; or a C₆₋₁₂ aroyloxyC₁₋₈alkyl group such as an optionally substituted benzoyloxyethyl or benzoyl-oxypropyl group. Optional substituents present on the Alk¹ group include R⁴ substituents described above.

When Alk is present in or as a substituent, it may be for example a methylene, ethylene, n-propylene, i-propylene, n-butylene, i-butylene, s-butylene, t-butylene, ethenylene, 2-propenylene, 2-butenylene, 3-butenylene, ethynylene, 2-propynylene, 2-butynylene or 3-butynylene chain, optionally interrupted by one, two, or three -O- or -S-, atoms or -S(O)-, -S(O)₂ or -N(R⁶)- (where R⁶ is a hydrogen atom or a straight or branched C₁₋₆alkyl group) groups.

When R⁴ is present in compounds of formula (1) as an optionally substituted cycloaliphatic group it is an optionally substituted C₃₋₁₀ cycloaliphatic group. Particular examples include optionally substituted C₃₋₁₀cycloalkyl, e.g. C₃₋₇cycloalkyl, or C₃₋₁₀cycloalkenyl e.g. C₃₋₇cycloalkenyl groups.

Heterocycloaliphatic groups represented by R⁴ include the aliphatic or cycloaliphatic groups just described for R⁴ but with each group additionally containing one, two, three or four heteroatoms or heteroatom-containing groups selected from -O- or -S- atoms or -N(R⁶)-, -C(O), -C(S)-, -S(O)- or - S(O₂)- groups.

Particular examples of R⁴ cycloaliphatic and heterocycloaliphatic groups include optionally substituted cyclopropyl, cyclobutyl, cyclopentyl, cyclohexyl, cycloheptyl, 2-cyclobuten-1-yl, 2-cyclopenten-1-yl, 3-cyclopenten-1-yl, 2,4-cyclopentadien-1-yl, 3,5,-cyclohexadien-1-yl, tetrahydrofuranyl, pyrroline, e.g. 2- or 3-pyrrolinyl, pyrrolidinyl, dioxolanyl, e.g. 1,3-dioxolanyl, imidazolinyl, e.g. 2-imidazolinyl, imidazolidinyl, pyrazolinyl, e.g. 2-pyrazolinyl, pyrazolidinyl, pyranyl, e.g. 2- or 4-pyranyl, piperidinyl, 1,4-dioxanyl, morpholinyl, 1,4-dithianyl, thiomorpholinyl, piperazinyl, 1,3,5-trithianyl, oxazinyl, e.g. 2H-1,3-, 6H-1,3-, 6H-1,2-, 2H-1,2- or 4H-1,4- oxazinyl, 1,2,5-oxathiazinyl, isoxazinyl, oxathiazinyl, e.g. 1,2,5 or 1,2,6-oxathiazinyl, or 1,3,5-oxadiazinyl groups.

Optional substituents which may be present on R⁴ cycloaliphatic and heterocycloaliphatic groups include one, two, three or more substituents selected from halogen atoms, e.g. fluorine, chlorine, bromine or iodine atoms, or hydroxyl, C₁₋₆alkoxy , e.g. methoxy or ethoxy, thiol, C₁₋₆alkylthio, e.g. methylthio or ethylthio, hydroxy, C₁₋₆alkyl, e.g. hydroxymethyl, hydroxyethyl, -CN, -NO₂, -NHR⁵ or -N(R⁵)₂ groups.

Aryl and heteroaryl groups represented by the groups R⁴, R⁵ or Ar¹ include for example optionally substituted monocyclic or bicyclic C₆₋₁₂ aromatic groups, e.g. phenyl groups, or C₁₋₉ heteroaromatic groups such as those described above in relation to the group Ar. Optional substituents which may be present on these groups include one, two or three R^{4a} atoms or groups described below.

Particularly useful atoms or groups represented by R⁴, -Alk(R⁴)ₘ or R^{4a} as appropriate include fluorine, chlorine, bromine or iodine atoms, or C₁₋₆alkyl, e.g. methyl or ethyl, C₁₋₆alkylamino, e.g. methylamino or ethylamino, hydroxyC₁₋₆alkyl, e.g. hydroxymethyl or hydroxyethyl, hydroxyC₂₋₆alkoxy, e.g. 2-hydroxyethoxy or 3-hydroxyethoxy, C₁₋₆alkylthiol e.g. methylthiol or ethylthiol, C₁₋₆alkoxy, e.g. methoxy or ethoxy, C₅₋₇cycloalkoxy, e.g. cyclopentyloxy, haloC₁₋₆alkyl, e.g. trifluoromethyl, amino (-NH₂), aminoC₁₋₆-alkyl, e.g. aminomethyl or aminoethyl, C₁₋₆dialkylamino, e.g. dimethylamino or diethylamino, aminoC₁₋₆alkoxy, C₁₋₆alkylaminoC₁₋₆alkoxy, diC₁₋₆-alkylaminoC₁₋₆alkoxy, imido, such as phthalimido or naphthalimido, e.g. 1,8-naphthalimido, 1,1,3-trioxobenzo[d]thiazolidino, nitro, cyano, hydroxyl (-OH), formyl [HC(O)-], carboxyl (-CO₂H), -CO₂Alk¹ [where Alk¹ is as defined above], C₁₋₆alkanoyl e.g, acetyl, thiol (-SH), thioC₁₋₆alkyl, e.g. thiomethyl or thioethyl, -SC(NH₂+)NH₂, sulphonyl (-SO₃H), C₁₋₆alkylsulphonyl, e.g. methylsulphonyl, aminosulphonyl (-SO₂NH₂), C₁₋₆alkylaminosulphonyl, e.g. methylaminosulphonyl or ethylaminosulphonyl, C₁₋₆dialkylaminosulphonyl, e.g. dimethylaminosulphonyl or diethylaminosulphonyl, phenylaminosulphonyl, carboxamido (-CONH₂), C₁₋₆alkylaminocarbonyl, e.g. methylaminocarbonyl or ethylaminocarbonyl, C₁₋₆dialkylaminocarbonyl, e.g. dimethylaminocarbonyl or diethylaminocarbonyl, sulphonylamino (-NHSO₂H), C₁₋₆alkylsulphonylamino, e.g. methylsulphonylamino or ethylsulphonylamino, C₁₋₆dialkylsulphonylamino, e.g. dimethylsulphonylamino or diethylsulphonylamino, optionally substituted phenylsulphonylamino, e.g. 2-, 3- or 4-substituted phenylsulphonylamino such as 2-nitrophenylsulphonylamino, aminosulphonylamino (-NHSO₂NH₂), C₁₋₆alkylaminosulphonylamino, e.g. methylaminosulphonylamino or ethylaminosulphonylamino, C₁₋₆dialkylaminosulphonylamino, e.g. dimethylaminosulphonylamino or diethylaminosulphonylamino, phenylaminosulphonylamino, aminocarbonylamino, C₁₋₆alkylaminocarbonylamino e.g. methylaminocarbonylamino or ethylaminocarbonylamino, C₁₋₆dialkylaminocarbonylamino, e.g. dimethylaminocarbonylamino or diethylaminocarbonylamino, phenylaminocarbonylamino, C₁₋₆alkanoylamino, e.g. acetylamino, optionally substituted phenylcarbonylamino, C₁₋₆alkanoylaminoC₁₋₆alkyl, e.g. acetylaminomethyl, C₁₋₆alkoxycarbonylamino, e.g. methoxycarbonylamino, ethoxycarbonylamino or t-butoxycarbonylamino, optionally substituted heteroC₃₋₆cycloalkyl, e.g. piperidinyl, piperazinyl, 4-(C₁₋₆alkyl)piperazinyl, e.g. 4-methylpiperazinyl, homopipeprazinyl, or morpholinyl, optionally substituted heteroC₃₋₆cycloalkylC₁₋₆alkyl, e.g. piperidinylC₁₋₆alkyl, piperazinylC₁₋₆alkyl, 4-(C₁₋₆alkyl)piperazinylC₁₋₆alkyl, e.g. 4-methylpiperazinylmethyl, or morpholinylC₁₋₆alkyl, optionally substituted heteroC₃₋₆cycloalkylC₁₋₆alkoxy, e.g. morpholinylC₁₋₆alkoxy, optionally substituted heteroC₃₋₆alkylC₁₋₆alkylamino, optionally substituted heteroC₃₋₆cycloalkylamino, tetrazolyl, optionally substituted imidazolyl, optionally substituted triazolyl, e.g.1,2,4-, 1,2,3-, 1,3,4- or 1,2,5-triazolyl, optionally substituted imid azolylC₁₋₆alkyl, optionally substituted imidazolylC₁₋₆alkoxy, optionally substituted triazolylC₁₋₆alkoxy, optionally substituted imidazolylaminoC₁₋₆alkoxy, optionally substituted phenylamino, optionally substituted benzylamino, optionally substituted benzyloxy, or optionally substituted pyridylmethylamino group.

Where desired, two R⁴ or -Alk(R⁴)ₘ substituents may be linked together to form a cyclic group such as a cyclic ether, e.g. a C₂₋₆alkylenedioxy group such as ethylenedioxy.

It will be appreciated that where two or more R⁴, -Alk(R⁴)ₘ or R^{4a} substituents are present, these need not necessarily be the same atoms and/or groups.

Especially useful R⁴, -Alk(R⁴)ₘ or R^{4a} substituents include for example fluorine, chlorine, bromine or iodine atoms, or a methylamino, ethylamino, hydroxymethyl, hydroxyethyl, methylthiol, ethylthiol, methoxy, ethoxy, n-propoxy, 2-hydroxyethoxy, 3-hydroxypropoxy, 4-hydroxybutoxy, 2-aminoethoxy, 3-aminopropoxy, 2-(methylamino)ethoxy, 2-(dimethylamino)ethoxy, 3-(dimethyl-amino)propoxy, cyclopentyloxy, cyclohexyl, cyclohexylamino, 2-hydroxycyclohexylamino, trifluoromethyl, trifluoromethoxy, methylamino, ethylamino, amino (-NH)₂, aminomethyl, aminoethyl, dimethylamino, diethylamino, ethyl(methyl) amino, propyl(methyl)amino, 2-hydroxyethylamino, 3-hydroxypropylamino, 4-hydroxybutylamino, 2-aminoethylamino, 3-aminopropylamino, 4-aminobutylamino, 2-(methylamino)ethylamino, 2-(ethylamino)ethylamino, 2-(i-propylamino)ethylamino, 3-(i-propylamino)-propylamino, 2-(dimethylamino)ethylamino, 3-(dimethylamino)propylamino, 2-(diethylamino)ethylamino, 3-(diethylamino)propylamino, 2-(methylamino)-ethyl(methyl)amino, 3-(methylamino)propyl(methyl)amino, 2-(dimethylamino)ethyl(methyl)amino, 2-(dimethylamino)ethyl(ethyl)amino, dimethylaminoethoxy, nitro, cyano, hydroxyl (-OH), formyl [HC(O)-], carboxyl (-CO₂H), -CH₂CO₂H, -OCH₂CO₂H, -CO₂CH₃, -CO₂CH₂CH₃, -CH₂CO₂CH₃, -CH₂CO₂CH₂CH₃, -CH₂CO₂CH₂phenyl, t-butoxycarbonylmethoxy, acetyl, phenacetyl thio (-SH), thiomethyl, thioethyl, -SC(NH)NH₂, sulphonyl (-SO₂H), methylsulphonyl, methylaminosulphonyl, ethylaminosulphonyl, dimethylaminosulphonyl, diethylaminosulphonyl, carboxamido (-CONH₂), methylaminocarbonyl, ethylaminocarbonyl, dimethylaminocarbonyl, diethylaminocarbonyl, methylaminocarbonylmethyl, -NHC(S)NH₂, sulphonylamino (-NHSO₂H), methylsulphonylamino ethylsulphonylamino, dimethylsulphonylamino, diethylsulphonylamino, sulphonylamino (-NHSO₂NH₂), methylaminosulphonylamino, ethylaminosulphonylamino, dimethylaminosulphonylamino, diethylaminosulphonylamino, methylaminocarbonylamino, ethylaminocarbonylamino, dimethylaminocarbonylamino diethylaminocarbonylamino, acetylamino, phenylcarbonylamino, aminomethylcarbonylamino, acetylaminomethyl, methoxycarbonylamino, ethoxycarbonylamino, t-butoxycarbonylamino, pyrrolidinyl, piperidinyl, piperazinyl, 4-methylpiperazinyl, 4-methylpiperazinylC₁₋₆alkylphenylcarbonylamino, homopiperazinyl, morpholinyl, pyrrolidinylC₁₋₆alkyl, piperidinylC₁₋₆alkyl, piperazinylC₁₋₆alkyl, 4-(C₁₋₆alkyl)piperazinylC₁₋₆alkyl, morpholinylC₁₋₆alkyl, morpholinoethoxy, 2-pyrrolidinylethylamino, 2-(1-methylpyrrolidinyl)ethylamino, 1-ethylpyrrolidinylmethylamino, piperidinylamino, 1-benzylpiperidinylamino, imidazolyl, 1,2,4-triazolyl, 1,2,3-triazolyl, 1,3,4-triazolyl, 1,2,5-triazolyl, C₁₋₆alkylimidazolylC₁₋₆alkyl, imidazolylC₁₋₆alkoxy, triazolylC₁₋₆-alkyl, triazolylC₁₋₆alkoxy, imidazolylC₁₋₆alkyl such as imidazlylmethyl or imidazolylethyl, 4-(methoxy)phenylamino, 4-(3-hydroxypropyl)phenylamino, benzylamino, benzyloxy or pyridiylmethylamino group.

In the compounds of formula (1), when the group R¹ or the group R⁶ [when present as -N(R⁶)-] is a straight or branched chain alkyl group it may be for example a C₁₋₆ straight or branched chain alkyl group such as a methyl, ethyl, n-propyl or isopropyl group.

Substituted amino groups include for example groups of formulae -NR⁸R⁹ [where R⁸ is an optionally substituted C₁₋₆alkyl, C₂₋₆alkenyl or C₂₋₆alkynyl group optionally interrupted by one or two heteroatoms or heteroatom-containing groups represented by X³ (where X³ is an atom or group as described above for X¹) and R⁹ is a hydrogen atom or is a group as just defined for R⁸], -N(R⁹)COR⁸, -N(R⁹)CSR⁸, -N(R⁹)SOR⁸, -N(R⁹)SO₂R⁸, -N(R⁹)CONH₂, -N(R⁹)CONR⁸R⁹, -N(R⁹)C(O)OR⁸, -N(R⁹)C(NH)NH₂, -N(R⁹)C(NH)NR⁸NR⁹, -N(R⁹)CSNH₂, -N(R⁹)CSNR⁸R⁹, -N(R⁹)SONH₂, -N(R⁹)SONR⁸R⁹, -N(R⁹)SONH₂, -N(R⁹)SO₂NH₂, -N(R⁹)SONR⁸R⁹ or -N(R⁹)Cyc¹ [where Cyc¹ is an optionally substituted C₃₋₇ monocyclic carbocyclic group optionally containing one or more -O- or -S- atoms or -N(R⁶)-, -C(O)-, -C(S)-, -S(O)- or -S(O₂)- groups].

Particular examples of amino, substituted amino and cyclic amino groups include -NH₂, methylamino, ethylamino, dimethylamino, diethylamino, -NHCyc¹ where Cyc¹ is an optionally substituted cyclopentyl, cyclohexyl, pyrrolidinyl, imidazolidinyl, pyrazolidinyl, piperidinyl, morpholinyl, piperazinyl or thiomorpholinyl group, or -NHet¹ where -NHet¹ is an optionally substituted pyrrolidinyl, imidazolidinyl, pyrazolidinyl, piperidinyl, morpholinyl, piperazinyl or thiomorpholinyl group. Optional substituents which may be present on these groups and substituted and cyclic amino groups in general include one, two or three halogen atoms, e.g. fluorine, chlorine, bromine or iodine atoms, or C₁₋₄alkyl, e.g. methyl or ethyl, -NH2-, -NHCH3-, -N(CH₃)₂, hydroxyl, or C₁₋₄alkoxy, e.g. methoxy or ethoxy groups.

When R³ is present as an aromatic or heteroaromatic group in compounds of formula (1) it may be for example an optionally substituted aromatic or heteroaromatic group as described above in relation to the group Ar. Optional substituents which may be present on these aromatic or heteroaromatic groups include one, two or three R^{4b} or -Alk(R^{4b})ₘ substituents where R^{4b} is an atom or group as described above for R⁴, and Alk and m are as described previously. Particular substituents include optionally substituted C₁₋₆alkyl groups [wherein the optional substituents include one, two or three of those optional substituents described above for R³ when it is an aliphatic group and halogen atoms, e.g. fluorine, chlorine, bromine or iodine atoms or hydroxyl, C₁₋₆alkoxy, e.g. methoxy or ethoxy, thiol, C₁₋₆alkylthio, e.g. methylthio or ethylthio, -SC(NH)NH₂, -CH₂C(NH)NH₂, amino, substituted amino or cyclic amino groups as described above for the optinoal substituents on aliphatic R₃ groups.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isethionates, arylsulphonates, e.g. p-toluenesulphonates, besylates or napsylates, phosphates, sulphates, hydrogen sulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, piperazine, dimethylamine or diethylamine salts.

Particularly useful salts of compounds according to the invention include pharmaceutically acceptable salts, especially acid addition pharmaceutically acceptable salts.

It will be appreciated that depending on the nature of the group Ar and the substituent R², the compounds of formula (1) may exist as tautomers and/or geometrical isomers and/or may have one or more chiral centres so that enantiomers or diasteromers may exist. It is to be understood that the invention extends to all such tautomers and isomers of the compounds of formula (1), and to mixtures thereof, including racemates.

In the compounds according to the invention the aromatic group represented by Ar is preferably a substituted phenyl group. The heteroaromatic group represented by Ar is preferably a substituted five- or six-membered monocyclic heteroaromatic group or a nine- or ten-membered fused-ring heteroaromatic group, each of said groups containing one or two oxygen, sulphur and/or nitrogen atoms. Particularly useful groups of these types include substituted pyridyl, indolyl, benzimidazolyl, indazolyl, benzothiazolyl, quinolyl, isoquinolyl and benzoxazolyl groups.

Substituted quinolyl, indazolyl or benzothiazolyl groups are especially useful. The substituent(s) present on any of the above-mentioned preferred Ar groups may be any of those -R⁴ or -Alk(R⁴)ₘ atoms or groups, particularly one, two or three -R⁴ and/or -Alk(R⁴)ₘ atoms or groups, generally or particularly described above and hereinafter in the Examples. Particularly useful substituents are those which contain one or more basic centres, as described hereinafter. In one preference, at least one -R⁴ or -Alk(R⁴)ₘ substituent will contain a basic centre.

In general in compounds of the invention R¹ is preferably a hydrogen atom.

In one general preference, R² in compounds of formula (1) is a group -X¹R³ in which X¹ is a direct bond. In these compounds R³ is preferably an optionally substituted aromatic group or an optionally substituted heteroaromatic group containing one or two ring oxygen, sulphur and/or nitrogen atoms and is especially a monocyclic heteroaromatic group. Thus in particular R³ may be an optionally substituted phenyl, thienyl, thiazolyl, indolyl or pyridyl group. The pyridyl group may in general be attached to the remainder of the compound of formula (1) through any available ring carbon atom and is in relation to that carbon atom, a 2-, 3- or 4- pyridyl group. Substituted 3-pyridyl groups are especially useful. Substituents which may be present on these R³ aromatic and heteroaromatic groups include one, two or three -R^{4b} or -Alk(R^{4b})ₘ substituents as described in general and in particular above and hereinafter in the Examples. In one preference, at least one -R^{4b} or -Alk(R^{4b})ₘ substituent will contain a basic centre as described hereinafter.

A particularly useful group of compounds according to the invention has the formula (1) wherein Ar is a substituted phenyl, six-membered monocyclic heteroaromatic group or nine- or ten-membered fused-ring heteroaromatic group, each of said groups containing one or two oxygen, sulphur and/or nitrogen atoms; R¹ is a hydrogen atom and R² is an optionally substituted phenyl, thienyl, thiazolyl or monocyclic or bicyclic heteroaromatic group containing one or two oxygen, sulphur and/or nitrogen atoms.

In compounds of this type, Ar is especially a substituted phenyl, pyridyl, indolyl, indazolyl, benzimidazolyl, benzothiazolyl, quinolyl, isoquinolyl or benzoxazolyl group. Substituted phenyl groups are particularly useful. The group R² is preferably an optionally substituted thienyl, phenyl, indolyl or pyridyl group.

The substituents which may be present on Ar or R² groups of these types include one, two or three of those -R⁴, -Alk(R⁴)ₘ, -R^{4b} and/or -Alk(R^{4b})ₘ substituents generally and particularly described above in relation to compounds of formula (1), especially substituents which contain one or more basic centres. Particularly useful substituents containing basic centres include nitrogen containing groups such as amino, substituted amino and cyclic amino groups, as described above in relation to optional substituents present on R³ aliphatic groups, optionally substituted and nitrogen-containing heteroaromatic groups, particularly five- or six-membered nitrogen-containing monocyclic heteroaromatic groups such as optionally substituted imidazolyl groups.

Particular groups containing basic centres include -X^{1a}(Alk^{a})ₚNR^{7a}R^{7b} (where X^{1a} is a direct bond or a linker atom or group, Alk^{a} is as defined above for Alk, p is zero or an integer 1) and R^{7a} and R^{7b} which may be the same or different is each a hydrogen atom or a straight or branched C₁₋₆alkyl group, -X^{1a}(Alk^{a})ₚNHet¹ (where -NHet¹ is an optionally substituted C₃₋₇ cyclic amino group optionally containing one or more other heteroatoms or heteroatom containing groups selected from -O- or -S- atoms or -N(R⁶)-, -C(O), -C(S)-, -S(O)- or -S(O₂)- groups) and -X^{1a}(Alk^{a})ₚAr² (where Ar² is a nitrogen containing heteroaromatic group as described above for Ar). In these groups, NR^{7a}R^{7b} may in particular be -NHCH₃, -N(CH₃)₂, -NHCH₂CH₃, -N(CH₃)(CH₂CH₃), or -N(CH₂CH₃)₂, -NHet¹ may in particular be optionally substituted pyrrolidinyl, piperidinyl, imidazolidinyl, piperazinyl, morpholinyl, thiomorpholinyl or pyrazolidinyl; Ar² may in particular be optionally substituted imidazolyl. X^{1a} when present may in particular be an oxygen atom or a -NH- group.

Linker atoms represented by X^{1a} when present in compounds of formula (1) include -O- or -S- atoms. When X^{1a} is a linker group it may be for example a -C(O)-, -C(S)-, -S(O)-, -S(O)₂-, -N(R⁷)- [where R⁷ is a hydrogen atom or a C₁₋₆ alkyl, e.g. methyl or ethyl, group], -C(R⁷)₂-, -CON(R⁷)-, -OC(O)N(R⁷)-, -CSN(R⁷)-, -N(R⁷)CO-, -N(R⁷)C(O)O-, -N(R⁷)CS-, -SON(R⁷), -SO₂N(R⁷), -N(R⁷)SO₂-, -N(R⁷)CON(R⁷)-, -N(R⁷)CSN(R⁷)-, -N(R⁷)SON(R⁷)- or -N(R⁷)SO₂N(R⁷) group.

Especially useful -R^{4b} and -Alk(R^{4b})ₘ substituents in compounds of the invention include -NH₂, -(CH₂)₂NH₂, -C(CH₃)₂NH₂, -C(CH₃)₂NHCH₃, -C(CH₃)₂N(CH₃)₂, -CH₂N(CH₂CH₃)₂, -CONH(CH₂)₂N(CH₂CH₃)₂, -C(CH₃)₂-pyrrolidinyl, dimethylaminopyrrolidinyl, imidazolyl, imidazolylmethyl, imidazolylethyl and piperidinylethyl groups. Particularly useful -R⁴ and - Alk(R⁴)ₘ substituents include fluorine and chlorine atoms and methyl, ethyl, methoxy, -CF₃, -CH₂F₂, -CH₂F, -OH, -OCF₃, -OCHF₃, -OCHF₂, - OCH₂F, -NO₂, -CN, -NH₂, -NHCH₃, -N(CH₃)₂, Ar^{2a} where Ar^{2a} is imidazolyl, C₁₋₃alkylimidazolyl, triazolyl or C₁₋₃alkyl-triazolyl, -C₁₋₃alkylAr^{2a}, -OC₁₋₃₃alkylAr^{2a}, -NHet^{1a}, where -NHet^{1a} is piperidinyl, C₁₋₃alkylpiperidinyl, morpholinyl, C₁₋₃alkylmorpholinyl, pyrrolidinyl, C₁₋₃alkylpyrrolidinyl, piperazinyl, C₁₋₃alkylpiperazinyl, imidazolidinyl, C₁₋₃alkylimiazolidinyl, pyrazolidinyl or C₁₋₃alkylpyrazolidinyl, -C₁₋₃alkylNHet^{1a}, -OC₁₋₃alkyINHet^{1a}, and -NHCOAr³ where Ar³ is phenyl optionally substituted by Ar^{2a}, -C₁₋₃₃alkylAr^{2a}, -OC₁₋₃alkylAr^{2a}, -NHet¹, -C₁₋₃alkylNHet¹ and -OC₁₋₃alkylNHet¹.

In the above preferred groups the term triazolyl is intended to mean all possible isomers as described above in relation to the group Ar and especially includes 1,2,3- and 1,2,4-triazolyl groups.

Particularly useful compounds of the invention include:
5-Cyano-4-phenyl-N-(3,4,5-trimethoxyphenyl)pyrimidine-2-amine;
5-Cyano-N-[4-(2-imidazol-1-ylethyl)phenyl]-4-(4-methoxcarbonylphenyl) pyrimidine-2-amine;
5-Cyano-4-(4-hydroxymethylphenyl)-N-(3,4,5-trimethoxyphenyl)pyrimidine-2-amine;
5-Cyano-4[(4-N,N-diethylaminomethyl)phenyl]-N-(3,4,5-trimethoxyphenyl) pyrimidine-2-amine;
5-Cyano-4-[2-(3(R)-dimethylaminopyrrolidin-1-yl)pyridin-5-yl]-N-(indazol-5-yl)pyrimidine-2-amine;
4-[4-(1-Amino-1-methylethyl)phenyl]-5-cyano-N-(indazol-5-yl)pyrimidine-2-amine;
4-[4-(1-Amino-1-methylethyl)phenyl]-5-cyano-N-(3,4,5-trimethoxyphenyl) pyrimidine-2-amine;
5-Cyano-N-[4-(2-N,N-diethylaminoethylaminocarboxy)phenyl]-4-phenylpyrimidine-2-amine;
5-Cyano-4-phenyl-N-{4-[2-(2-ethylimidazol-1-yl)ethyl]phenyl}-pyrimidine-2-amine;
4-[4-(1-Amino-1-methylethyl)phenyl]-5-cyano-N-4(1,2,3-triazol-1-yl)-phenyl]pyrimidine-2-amine;
4-[4-(1-Amino-1-methylethyl)phenyl]-5-cyano-N-{4-[2-(2-ethylimidazol-1-yl)ethyl]phenyl}pyrimidine-2-amine;
N-[3-(5-Cyano-4-thiophen-2-ylpyrimidin-2-ylamino)phenyl]-4-(4-methyl piperazin-1-ylmethyl)benzamide;
4-[3-(1-Amino-1-methylethyl)phenyl]-5-cyano-*N*-{4-[2-(2-methylimidazol-1-yl)ethyl]phenyl}pyrimidine-2-amino;
5-Cyano-4-[4-(imiadzol-1-yl)methyl]phenyl-*N*-(3,4,5-trimethoxyphenyl)-pyrimidine-2-amino;
and the salts, solvates, hydrates and N-oxides thereof.

Especially useful compounds according to the invention include:
4-[4-(1-Amino-1-methylethyl)phenyl]-5-cyano-N-[4(1,2,4-triazol-1-yl)phenyl]pyrimidine-2-amine;
5-Cyano-N-[4-(1,2,4-triazol-1-yl)phenyl]-4-[4-(1-dimethylamino-1-methyl ethyl)phenyl] pyrimidine-2-amine;
4-[4-(1-Amino-1-methylethyl)phenyl]-5-cyano-N-(4-fluorophenyl) pyrimidine-2-amine;
4-[4-(1-Amino-1-methylethyl)phenyl]-5-cyano-N-{4-[2-piperidin-1-ylethyl]phenyl}pyrimidine-2-amine;
4-[4-(1-Amino-1-methylethyl)phenyl]-5-cyano-N-[4-(2-imidazol-1-ylethyl)phenyl]pyrimidine-2-amine;
4-[4-(1-Amino-1-methylethyl)phenyl]-5-cyano-N-[4-(2-morpholino ethyl)phenyl]pyrimidine-2-amine;
4-[4-(1-Amino-1-methylethyl)phenyl]-5-cyano-N-[3-(2-morpholino ethyl)phenyl]pyrimidine-2-amine;
5-Cyano-4-[4-(1-methyl-1-pyrrolidin-1-ylethyl)phenyl]-N-(4-fluoro phenyl)pyrimidine-2-amine;
5-Cyano-4-{2-([2-(diethylamino)ethyl]amino)pyridin-5-yl}-N-(4-fluorophenyl) pyrimidine-2-amine;
4-[4-(1-Amino-1-methylethyl)phenyl]-5-cyano-N-(3-fluorophenyl) pyrimidine-2-amine;
and the salts, solvates, hydrates and N-oxides thereof.

Compounds according to the invention are potent and selective inhibitors of KDR and/or FGFr kinases as demonstrated by differential inhibition of these enzymes when compared to inhibition of other protein kinases such as EGFr kinase, p56^{lck} kinase, ZAP-70 kinase, protein kinase C, Csk kinase and p59^{fyn} kinase. The ability of the compounds to act in this way may be simply determined by employing tests such as those described in the Examples hereinafter.

The compounds according to the invention are thus of particular use in the prophylaxis and treatment of diseases in which inappropriate KDR kinase action plays a role, for example in disease states associated with angiogenesis. The compounds are then of use for example in the prophylaxis and treatment of cancer, prosiasis, rheumatoid arthritis, Kaposi's Sarcoma, ischemic heart disease, atherosclerosis and occular diseases, such as diabetic retinopathy, involving retinal vessel proliferation and the invention is to be understood to extend to such uses and to the use of a compound of formula (1) in the preparation of a medicament for the prophylaxis and teatment of such diseases.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Pharmaceutical compositions according to the invention may take a form suitable for oral, buccal, parenteral, nasal, topical or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds for formula (1) may be formulated for parenteral administration by injection, including bolus injection or infusion or particle mediated injection. Formulations for injection may be presented in unit dosage form, e.g. in glass ampoule or multi dose containers, e.g. glass vials or a device containing a compressed gas such as helium for particle mediated administration. The compositions for bolus injection or infusion may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use. For particle mediated administration the complex may be coated on particles such as microscopic gold particles.

In addition to the formulations described above, the compounds of formula (1) may also be formulated as a depot preparation. Such long acting formulations may be administered by implantation or by intramuscular injection. Where desired, the compounds according to the invention may also be conjugated to a polymer, e.g. a naturally occuring polymer such as albumin, to prolong the half life of the compounds when in use. Such conjugates may be formulated and delivered as described above.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichloro-fluoromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular condition will vary depending on the compound chosen, and the condition of the patient to be treated. In general, however, daily dosages may range from around 100ng/kg to 100mg/kg e.g. around 0.01mg/kg to 40mg/kg body weight for oral or buccal administration, from around 10ng/kg to 50mg/kg body weight for parenteral administration and around 0.05mg to around 1000mg e.g. around 0.5mg to around 1000mg for nasal administration or administration by inhalation or insufflation.

The compounds of the invention may be prepared by a number of processes as generally described below and more specifically in the Examples hereinafter. In the following process description, the symbols R¹, R², Alk, Alk¹ and Ar when used in the text or formulae depicted are to be understood to represent those groups described above in relation to formula (1) unless otherwise indicated. In the reactions described below, it may be necessary to protect reactive functional groups, for example hydroxy, amino, thio or carboxy groups, where these are desired in the final product, to avoid their unwanted participation in the reactions.

Conventional protecting groups may be used in accordance with standard practice [see, for example, Green, T. W. in "Protective Groups in Organic Synthesis", John Wiley and Sons, (1991)]. In some instances, deprotection may be the final step in the synthesis of a compound of formula (1) and the processes according to the invention described hereinafter are to be understood to extend to such removal of protecting groups.

A compound of formula (1) may be prepared by reaction of a guanidine of formula (2): 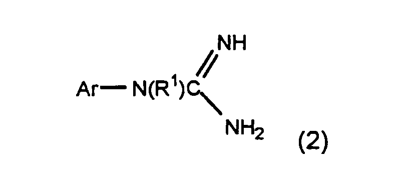 or a salt thereof
with an enaminone of formula (3):

R²COC(CN)CHN(R¹⁰)(R¹¹) (3)

where R¹⁰ and R¹¹, which may be the same or different is each a C₁₋₆ alkyl group.

The reaction may be performed in a solvent, for example a protic solvent such as an alcohol, e.g. ethanol, methoxyethanol or propan-2-ol, optionally in the presence of a base e.g. an alkali metal base, such as sodium hydroxide or potassium carbonate, at an elevated temperature, e.g. the reflux temperature.

Salts of the compounds of formula (2) include acid salts such as inorganic acid salts e.g. hydrochlorides or nitrates.

Intermediate guanidines of formula (2) may be prepared by reaction of the corresponding amine ArNH₂ with cyanamide at an elevated temperature. The reaction may be performed in a solvent such as ethanol at an elevated temperature, e.g. up to the reflux temperature. Where it is desired to obtain a salt of a guanidine of formula (2), the reaction may be performed in the presence of a concentrated acid, e.g. hydrochloric or nitric acid.

The amines ArNH₂ are either known compounds or may be obtained by conventional procedures, for example by hydrogenation of the corresponding nitro derivatives using for example hydrogen in the presence of a metal catalyst in a suitable solvent, for example as more particularly described in the interconversion reactions discussed below. The nitrobenzenes for this particular reaction are either known compounds or may be prepared using similar methods to those used for the preparation of the known compounds.

Intermediate enaminones of formula (3) are either known compounds or may be prepared by reaction of an acetyl derivative R²COCH₂CN with an acetal (R¹⁰)(R¹¹)NCH(OR¹²)₂ (where R¹² is a C₁₋₆alkyl group such as a methyl or ethyl group) at an elevated temperature. The starting materials for this reaction are either known compounds or may be prepared by methods analogous to those used for the preparation of the known compounds.

One particularly useful method for the preparation of acetyl derivatives R²COCH₂CN involves treating a corresponding isoxazole of formula (4): 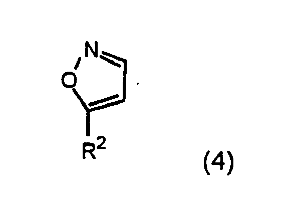 with a base such as an alkoxide, e.g. sodium ethoxide, in a solvent such as an alcohol, e.g.ethanol, at ambient temperature. Intermediate isoxazoles of formula (4) may be obtained by reaction of the corresponding aminopropenone (R²COCHCHN(R¹⁰)(R¹¹) with hydroxylamine in a solvent such as an alcohol, e.g. MeOH at ambient temperature. The aminopropenone starting material for this rection may be obtained by reaction of the corresponding methyl ketone R²COCH₃ with an acetal (R¹⁰)(R¹¹)NCH(OR¹²)₂ as described above.

In another process according to the invention, a compound of formula (1) may be prepared by displacement of a chlorine atom in a pyrimidine of formula (5): 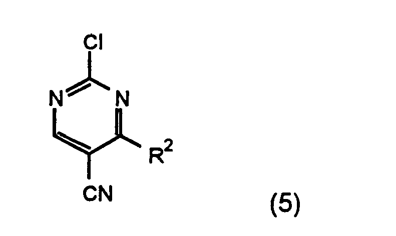 with an amine ArNH₂.

The reaction may be performed at an elevated temperature, for example the reflux temperature, where necessary in the presence of a solvent, for example an alcohol, such as 2-ethoxyethanol or isopopanol, a cyclic ether, e.g. dioxane or a substituted amide such as dimethylformamide, optionally in the presence of a base, for example an organic amine such as pyridine.

Intermediate pyrimidines of formula (5) may be obtained by reaction of a corresponding pyrimidine of formula (6): 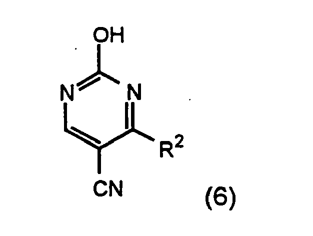 with phosphorous oxychloride optionally in a solvent such as a substituted amide e.g. dimethylformamide at an elevated temperature, for example the reflux temperature.

Intermediates of formula (6) may be prepared from the corresponding amine of formula (7): 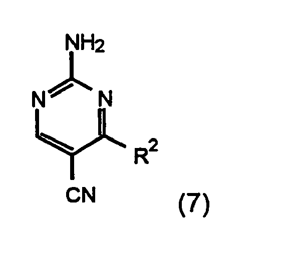 with sodium nitrite in an aqueous acid, e.g. aqueous sulphuric acid at around ambient temperature.

Amines of formula (7) may be prepared by reaction of an enaminone of formula (3) with a guanidine salt, e.g. guanidine carbonate, as described above for the preparation of compounds of formula (1).

Compounds of formula (1) may also be prepared by interconversion of other compounds of formula (1) and it is to be understood that the invention extends to such interconversion processes. Thus, for example, standard substitution approaches employing for example alkylation, arylation, heteroarylation, acylation, thioacylation, sulphonylation, formylation or coupling reactions may be used to add new substitutents to and/or extend existing substituents in compounds of formula (1). Alternatively existing substituents in compounds of formula (1) may be modified by for example oxidation, reduction or cleavage reactions to yield other compounds of formula (1).

The following describes in general terms a number of approaches which can be employed to modify existing Ar and/or R² groups in compounds of formula (1). It will be appreciated that each of these reactions will only be possible where an appropriate functional group exists in a compound of formula (1). Where desired, these reactions may also be performed on intermediates to compounds of formula (1), for example in the preparation of intermediate amines, ArNH₂ or acetyl derivatives R²COCH₂CN, and the description which follows is intended to apply to these intermediates even though only a compound of formula (1) is mentioned.

Thus, for example alkylation, arylation or heteroarylation of a compound of formula (1) may be achieved by reaction of the compound with a reagent AlkL or Ar³L, where Alk is an alkyl group and Ar³ is an aryl or heteroaryl group as defined above in relation to compounds of formula (1) and L is a leaving atom or group such as a halogen atom, e.g. a chlorine or bromine atom, or a sulphonyloxy group, e.g. an arylsulphonyloxy group such as a *p*-toluenesulphonyloxy group.

The alkylation, arylation or heteroarylation reaction may be carried out in the presence of a base, e.g. an inorganic base such as a carbonate, e.g. caesium or potassium carbonate, an alkoxide, e.g. potassium t-butoxide, or a hydride, e.g. sodium hydride, in a dipolar aprotic solvent such as an amide, e.g. a substituted amide such as dimethylformamide or an ether, e.g. a cyclic ether such as tetrahydrofuran, at around 0°C to around 40°C.

In a variation of this process the leaving group L may be alternatively part of the compound of formula (1) and the reaction performed with an appropriate nucleophilic reagent at an elevated temperature. Particular nucleophilic reagents include cyclic amines, such as piperazine or imidazole. Where appropriate the reaction may be performed in a solvent such as an aprotic solvent, e.g. a substituted amide such as dimethylformamide.

In another general example of an interconversion process, a compound of formula (1) may be acylated or thioacylated. The reaction may be performed for example with an acyl halide or anhydride in the presence of a base, such as a tertiary amine e.g. triethylamine in a solvent such as a halogenated hydrocarbon, e.g. dichloromethane or chloroform at for example ambient temperature, or by reaction with a thioester in an inert solvent such as tetrahydrofuran at a low temperature such as around 0°C. Alternatively, the reaction may be performed with an acid, in the presence of a condensing agent, for example a diimide such as 1-(3-dimethylaminopropyl)-3-ethylcarbodiimide, advantageously in the presence of a catalyst such as a N-hydroxy compound, e.g. a N-hydroxytriazole such as 1-hydroxybenzotriazole in the presence of a base, e.g. a cyclic amine such as N-methylmorpholine. The reaction is particularly suitable for use with compounds of formula (1) containing primary or secondary amino groups.

In a further general example of an interconversion process, a compound of formula (1) may be formylated, for example by reaction of the compound with a mixed anhydride HCOOCOCH₃ or with a mixture of formic acid and acetic anhydride.

Compounds of formula (1) may be prepared in another general interconversion reaction by sulphonylation, for example by reaction of the compound with a reagent AlkS(O)₂L, or Ar²S(O)₂L in the presence of a base, for example an inorganic base such as sodium hydride in a solvent such as an amide, e.g. a substituted amide such as dimethylformamide at for example ambient temperature. The reaction may in particular be performed with compounds of formula (1) possessing a primary or secondary amino group.

In further examples of interconversion reactions according to the invention compounds of formula (1) may be prepared from other compounds of formula (1) by modification of existing functional groups in the latter.

Thus in one example, ester groups -CO₂Alk¹ in compounds of formula (1) may be converted to the corresponding acid [-CO₂H] by acid- or base-catalysed hydrolysis or by catalytic hydrogenation depending on the nature of the group Alk¹. Acid- or base-catalysed hydrolysis may be achieved for example by treatment with an organic or inorganic acid, e.g. TFA acid in an aqueous solvent or a mineral acid such as hydrochloric acid in a solvent such as dioxan or an alkali metal hydroxide, e.g. lithium hydroxide in an aqueous alcohol or ether e.g. aqueous MeOH or tetrahydrofuran,. Catalytic hydrogenation may be carried out using for example hydrogen in the presence of a metal catalyst, for example palladium on a support such as carbon in a solvent such as an ether, e.g. tetrahydrofuran or an alcohol, e.g. MeOH.

In a second example, -OAlk² [where Alk² represents an alkyl group such as a methyl group] groups in compounds of formula (1) may be cleaved to the corresponding alcohol -OH by reaction with boron tribromide in a solvent such as a halogenated hydrocarbon, e.g. dichloromethane at a low temperature, e.g. around -78°C.

in another example, alcohol -OH groups in compounds of formula (1) may be converted to a corresponding -OAlk or -OAr group by coupling with a reagent AlkOH or ArOH in a solvent such as tetrahydrofuran in the presence of a phosphine, e.g. triphenylphosphine and an activator such as diethyl-, diisopropyl-, or dimethylazodicarboxylate.

Aminosulphonylamino [-NHSO₂NH₂] groups in compounds of formula (1) may be obtained, in another example, by reaction of a corresponding amine [-NH₂] with sulphamide in the presence of an organic base such as pyridine at an elevated temperature, e.g. the reflux temperature.

In another example of an interconversion process secondary amine groups in compounds of formula (1) may be alkylated using an alcohol, e.g. ethanol and catalytic hydrogenation, employing for example hydrogen in the presence of a metal catalyst such as palladium on a support such as carbon.

In a further example, amine [-NH₂] groups in compounds of formula (1) may be obtained by hydrolysis from a corresponding imide by reaction with hydrazine in a solvent such as an alcohol, e.g. ethanol at ambient temperature. In an alternative, amine groups may also be generated by reduction of the corresponding nitrile, for example using a reducing agent such as a borohydride, e.g. sodium borohydride or cerium trichloride. Alternatively, amine groups may be obtained by Ce^{IV} oxidation of the corresponding p-anisyl- or p-anisylmethylamines using for example ceric ammonium nitrate in a solvent such as acetonitrile.

In another example cyclic amino groups in compounds of formula (1) may be prepared by cyclisation of a corresponding compound containing an amine [-NH₂] group with a reagent L¹AlkL² where L¹ and L² which may be the same or different is each a leaving atom or group as described above L and may for example each be a halogen atom such as a bromine atom. The reaction may advantageously be carried out in the presence of a base e.g. an inorganic base such as potassium carbonate, at an elevated temperature.

In another example, a nitro [-NO₂] group may be reduced to an amine [-NH₂], for example by catalytic hydrogenation as just described, or by chemical reduction using for example a metal, e.g. tin or iron, in the presence of an acid such as hydrochloric acid.

N-oxides of compounds of formula (1) may be prepared for example by oxidation of the corresponding nitrogen base using an oxidising agent such as hydrogen peroxide in the presence of an acid such as acetic acid, at an elevated temperature, for example around 70°C to 80°C, or alternatively by reaction with a peracid such as peracetic acid in a solvent, e.g. dichloromethane, at ambient temperature.

Where salts of compounds of formula (1) are desired, these may be prepared by conventional means, for example by reaction of a compound of formula (1) with an appropriate acid or base in a suitable solvent or mixture of solvents, e.g. an organic solvent such as an ether, e.g. diethylether, or an alcohol, e.g. ethanol.

The following Examples illustrate the invention. In the Examples all ¹Hnmr were run at 300MHz unless specified otherwise. All temperatures are in o C.

The following abbreviations are used:
THF - tetrahydrofuran; DMF - dimethylformamide;
DMSO - dimethylsulphoxide; TFA - trifluoroacetic acid;
DIBAL-H - diisobutylaluminium hydride; MeOH - methanol.

### INTERMEDIATE 1

### 4-[2-(1,2,3-Triazol-1-yl)ethoxy]phenylguanidinium nitrate

The title compound was prepared from 4-[2-(1,2,3-triazol-1-yl)ethoxy]aniline (4.91g, 24.07mmol), cyanamide (1.56g, 40.97mmol) and concentrated HNO₃ (1.58mL, 26.47mmol) in a manner similar to the guanidine of Example 1 to give the desired material (4.7g) as an off-white solid, m.p. >250°. δH (d⁶ DMSO) 9.33 (1H, s), 8.20 (1H, s), 7.74 (1H, s), 7.17-7.14 (6H, m), 7.00-6.97 (2H, m), 4.79 (2H,t, J 4.95Hz) and 4.41 (2H, t, J 4.95Hz).

4-[2-(1,2,3-Triazol-1-yl)ethoxy]aniline was prepared from 4-[2-(1,2,3-triazol-1-yl)ethoxy] nitrobenzene (5.98g, 25.5mmol) and 10% palladium on charcoal (1.5g) in a manner similar to the aniline intermediate of Example 12 to give the desired material (4.91g) as a yellow solid m.p. 141°. δH (CDCl₃) 7.69 (1 H, d, J 0.5Hz), 7.62 (1 H, d J 0.5Hz), 6.65 (2H, d, J 5.8Hz), 6.58 (2H, d, J 5.8Hz), 4.71 (2H, t, J 5.0Hz), 4.24 (2H, t, J 5.0Hz) and 3.43 (2H, s).

4-[2-(1,2,3-Triazol-1-yl)ethoxy]nitrobenzene was prepared from 4-[(2-p-toluenesulphonyl oxy)ethoxy]nitrobenzene (10g, 29.7mmol) and 1,2,3-triazole, sodium salt (2.46mmol) in a manner similar to the analogous reaction of Example 24 to give the desired material (2.25g) as yellow solid, m.p. 123°. δH (d⁶ DMSO) 8.21 (1 H, s), 8.20 (2H, d, J 2.3Hz), 7.74 (1 H, d, J 0.5Hz), 7.14 (2H, d, J 2.4Hz), 4.84 (2H, t, J 4.9Hz) and 4.57 (2H, t, J 4.9Hz). The reaction also yielded 4-[2-(1,2,3-triazol-2-yl)ethoxy]nitrobenzene (4.36g) as a yellow solid, m.p. 111°. δH (d⁶ DMSO) 8.17 (2H, d, J 9.3Hz), 7.80 (2H, s), 7.11 (2H, d, J 9.3Hz), 4.86 (2H, t, J 4.8Hz) and 4.64 (2H, t, J 4.8Hz).

### INTERMEDIATE 2

### 4-[2-(1,2,3-Triazol-2-yl)ethoxy]phenylguanidinium nitrate

The title compound was prepared from 4-[2-(1,2,3-triazol-2-yl)ethoxy]aniline (8.85g,43.4mmol), cyanamide (2.82g, 73.78mmol) and concentrated HN03 (1.58mL, 26.47mmol) in a manner similar to the guanidine of Example 1 to give the desired material (7.95g) as an off-white solid, m.p. >250°. δH (d⁶ DMSO) 9.32 (1 H, s), 7.80 (2H, s), 7.16-7.13 (6H, m), 7.00-6.97 (2H, m), 4.79 (2H, t, J 4.95mmol) and 4.41 (2H, t, J 4.95mmol).

4-[2-(1,2,3-Triazol-2-yl)ethoxy]aniline was prepared from 4-[2-(1,2,3-triazol-2-yl)ethoxy] nitrobenzene (10.5g, 44.8mmol) and 10% palladium on charcoal (1.5g) in a manner similar to the aniline intermediate of Example 12 to give the desired material (4.91g) as a yellow solid m.p. 159°. δH (CDCl₃) 7.62 (2H, s), 6.73-6.58 (4H, m), 4.77 (2H, t, J 5.8Hz), 4.40 (2H, t, J 5.8Hz) 3.43 (2H, s).

### INTERMEDIATE 3

### 4-[2-(1,2,4-Triazol-1-yl)ethoxy]phenylguanidinium nitrate

The title compound was prepared from 4-[2-(1,2,4-triazol-1-yl)ethoxy]aniline (5.30g, 25.9mmol), cyanamide (1.86g, 44.11 mmol) and concentrated HNO₃ (1.88mL, 28.54mmol) in a manner similar to the guanidine of Example 1 to give the desired material (6.62g) as an off-white solid, m.p. 280-282°. δH (d⁶ DMSO) 9.33 (1 H, bs), 8.56 (1H, s), 7.97 (1 H, s), 7.17 (4H, bs), 7.16-7.12 (2H, s), 6.98-6.94 (2H, m), 4.58 (2H, t, J 5.0Hz) and 4.34 (2H, t, J 2.0Hz). 4-[2-(1,2,4-Triazol-1-yl)ethoxy]aniline was prepared from 4-[2-(1,2,4-triazol-1-yl)ethoxy] nitrobenzene (6.28g, 26.8mmol) and 10% palladium on charcoal (0.5g) in a manner similar to the aniline intermediate of Example 12 to give the desired material (5.31 g) as a yellow solid m.p. 85-86°. δH (CDCl₃) 8.21 (1 H, s), 7.94 (1 H, s), 6.69-6.58 (4H, m), 4.51 (2H, t, J 5.0Hz), 4.24 (2H, t, J 5.2Hz) and 3.45 (2H, bs).

4-[2-(1,2,4-Triazol-1-yl)ethoxy]nitrobenzene was prepared from 4-[(2-p-toluenesulphonyl oxy)ethoxy]nitrobenzene (10g, 30.7mmol) and 1,2,4-triazole, sodium salt (3.36g, 36.8mmol) in a manner similar to the analogous reaction of Example 24 to give the desired material (6.45g) as yellow solid, m.p. 118-120°. δH (CDCl₃) 8.21-8.17 (3H, m), 7.97 (1H, s), 6.93-6.90 (2H, m), 4.62 (2H, t, J 5.2Hz) and 4.45 (2H,t, J 5.3Hz).

### INTERMEDIATE 4

### 2-Cyano-3-dimethylamino-1 -pyridin-3-ylpropen-1-one

2-Hydroxy-2-pyridin-3-ylacrylonitrile, sodium salt (1.0g, 5.95mmol) was dissolved in methanol (20mL) and dimethylformamide diethylacetal (1.2mL, 7.0mmol) followed by 1 M hydrochloric acid in diethyl ether (5.95mL) were added. The reaction was stirred at room temperature for 1.5h and then concentrated under reduced pressure. The resulting residue was subjected to column chromatography (silica 3% methanol in dichloromethane) to give the desired product (560mg) as yellow solid. δH (CDCl3) 8.98 (1H, dd, J 2.3, 0.8Hz), 8.69 (1H, dd, 4.8, 1.6Hz), 8.08 (1H, dt, J 7.9, 2.2Hz), 7.98 (1 H, s), 7.37-7.33 (1 H, m), 3.50 (3H,s) and (3H, s).

2-Hydroxy-2-pyridin-3-ylacrylonitrile was prepared by adding a solution of ethyl nicotinate (22.67g, 0.15mol) and acetonitrile (15.6mol, 0.3mol) in toluene (100mL) and DMF (25mL) to a suspension of sodium ethoxide (9.70g, 0.143mol) and the resulting mixture heated at reflux for 2h with vigorous stirring. On cooling the reaction was diluted with ether (400mL) and the resulting precipitate collected and washed further with ether to give the desired material (20.1g) which was used without purification.

### EXAMPLE 1

### 5-Cyano-4-phenyl-N-(3,4,5-trimethoxyphenyl)pyrimidine-2-amine

A mixture of 3,4,5-trimethoxyphenylguanidinium nitrate (1.44g, 5.0mmol), 1-phenyl-2-cyano-3-dimethylaminopropen-1-one (1.0g, 5.0mmol) and sodium hydroxide (0.22g, 5.5mmol) in ethanol (20ml) was heated at reflux for 18h. On cooling the resulting precipitate was collected by filtration, washed with water and diethyl ether, then dried to give the title compound (895mg) as a green solid m.p. 246^{o}. δH (d⁶ DMSO) 10.37 (1H, br s), 8.93 (1 H, s), 7.99 (2H, m), 7.60 (3H, m), 7.25 (2H, m), 3.75 (6H, s) and 3.63 (3H, s).

The propenone used as starting material was prepared by refluxing benzoylacetonitrile (4.50g, 31.0mmol) in dimethylformamide diethylacetal (20ml) for 12h. On cooling the resulting solid was collected and washed with diethyl ether to give the desired product (4.50g) as a beige solid m.p. 98°.

3,4,5-Trimethoxyphenylguanidinium nitrate was prepared by heating a solution of 3,4,5-trimethoxyaniline (5.49g, 30.0mmol), cyanamide [Aldrich, 50% solution in water w/v] (3.50ml, 34.5 mmol) and concentrated HNO₃ (2.1ml, 30.0mmol) in ethanol (30ml). The solid which formed on cooling to room temperature was collected by filtration, washed with ethanol and dried to give the desired material (4.60g) as a grey solid m.p. 187°.

### EXAMPLE 2

### 5-Cyano-N-[4-(2-hydroxyethyl)phenyl]-4-methoxycarbonylphenylpyrimidine-2-amine

In a similar manner to the compound of Example 1, from 4-(2-hydroxyethyl)phenylguanidinium nitrate (1.88g, 7.75mmol), 1-(4-methoxycarbonylphenyl)-2-cyano-3-dimethylaminopropen-1-one (2.07g, 7.75mmol) and sodium hydroxide (310mg, 7.75mmol) to give the title compound (2.40g) as a yellow solid m.p. 194-196°. δH (d⁶ DMSO) 10.52 (1 H, br s), 8.96 (1 H, s), 8.15 (2H, d, J 8.2Hz), 8.04 (2H, d, J 8.2Hz), 7.63 (2H, d, J 7.9Hz), 7.18 (2H, d, J 7.9Hz), 4.61 (1 H, t, J 5.1 Hz), 3.90 (3H, s), 3.57 (2H, m), and 2.68 (2H, d, J 7.0Hz).

The propenone used as starting material in the above process was prepared in a similar manner to the analogous compound of Example 1, to give a yellow solid m.p. 118°.

4-(2-Hydroxyethyl)phenylguanidinium nitrate was prepared in a similar manner to the guanidine of Example 1 as an off-white solid.

### EXAMPLE 3

### 5-Cyano-4-(4-methoxycarbonylphenyl)-N-(3,4,5-trimethoxyphenyl) pyrimidine-2-amine

In a similar manner to the compound of Example 1, from 3,4,5-trimethoxyphenylguanidinium nitrate (1.12g, 3.88mmol), 2-cyano-1-(4-methoxycarbonylphenyl)-3-dimethylaminopropen-1-one (1.0g, 3.9mmol) and sodium hydroxide (258mg,3.88mmol) to give the title compound (760mg) as a yellow solid m.p. 206-208^{o}. δH (d⁶DMSO) 10.46 (1 H, s), 8.97 (1 H, s), 8.15 (4H, m), 7.23 (2H, br s), 3.89 (3H, s), 3.74(6H, s) and 3.62 (3H, s).

### EXAMPLE 4

### 5-Cyano-N-[4-(2-imidazol-1-ylethyl)phenyl]-4-(4-methoxcarbonylphenyl)pyrimidine-2-amine

To a solution of the compound of Example 2 (750mg, 2.0mmol) in pyridine (10ml) was added 4-toluenesulphonyl chloride (458mg, 2.0mmol) and the mixture stirred at ambient temperature for 2.5h. The reaction was diluted with dichloromethane (50ml), washed with 1 M hydrochloric acid (2 x 50ml) followed by saturated Na₂CO₃ (1 x 25ml), dried (MgSO₄) and then concentrated under reduced pressure. The residue was subjected to column chromatography (silica gel; 5% methanol-dichloromethane) to give the desired tosylate (600mg) as a yellow solid. This material was dissolved in dry DMF (15ml) containing imidazole (272mg, 4.0mmol) and the resulting mixture was stirred under a nitrogen atmosphere at 80° for 6h. To the reaction was added saturated brine (150ml) and 2M NaOH (10ml) and this was extracted with dichloromethane (1 x 100ml). The organic phase was dried (MgSO₄) and concentrated under reduced pressure. The residue was recrystallised from ethyl acetate/propan-2-ol (10: 1) to give the title compound (185mg) as a pale yellow solid m.p. 216-218°. δH (d⁶ DMSO) 10.55 (1 H, br s), 8.97 (1 H, s), 8.15 (2H, d, J 8.0Hz), 8.04 (2H, d, J 8.0Hz), 7.64 (2H, d, J 6.9Hz), 7.48 (1 H, s), 7.13 (3H, br s), 6.83 (1 H, s), 4.18 (2H, m), 3.90 (3H, s), and 3.30 (2H, m).

### EXAMPLE 5

### 5-Cyano-4-(4-hydroxymethylphenyl)-N-(3,4,5-trimethoxyphenyl)pyrimidine-2-amine

To a solution of the compound of Example 3 (210mg, 0.5mmol) in dry THF (20ml) at 0°, under a nitrogen atmosphere, was added DIBAL-H (1M in THF) (2.5ml, 2.5mmol) and the reaction was allowed to warm to ambient temperature over 4.5h. The reaction was quenched with 1 M potassium-sodium tartrate (75ml) and extracted with ethyl acetate (2 x 75ml). The organic phase was dried (MgSO₄), concentrated under reduced pressure and the residue subjected to column chromatography (silica gel; 3% methanol-dichloromethane) to give the title compound (150mg) as a yellow solid m.p.188-191°. δH (d⁶DMSO) 10.46 (1H, br s), 8.92 (1H, s), 7.99 (2H, br m), 7.54(2H, d, J 8.0Hz), 7.23 (2H, br s), 5.37 (2H, t, J 4.1 Hz), 4.58 (2H, d, J 4.1Hz), 3.73 (6H, s) and 3.53 (3H, s).

### EXAMPLE 6

### 5-Cyano-4[(4-N,N-diethylaminomethyl)phenyl]-N-(3,4,5-trimethoxyphenyl)pyrimidine-2-amine

The compound of Example 5 was dissolved in chloroform (10mL), thionyl chloride (37µl) added and the resulting solution heated at reflux for 0.1 h. The reaction was concentrated under reduced pressure and the residue taken up in acetonitrile (6ml) to which N,N-diethylamine (150µl) was added. After heating at reflux for 5h the mixture was concentrated under reduced pressure and the residue was subjected to column chromatography (silica gel; 5% methanol-dichloromethane) to give the title compound as a yellow solid m.p. 137°. δH (d⁶ DMSO) 8.68 (1H, s), 8.05 (2H, d, J 8.0Hz), 7.55-7.51 (3H, m), 7.00 (2H, br s), 3.88 (6H,s), 3.85 (3H, s), 2.56 (4H, q, J 7.1 Hz) and 1.07 (6H, t, J 7.1 Hz).

### EXAMPLE 7

### 5-Cyano-4-[2-(3-(R)-dimethylaminopyrrolidin-1-yl)pyridin-5-yl]-N-(indazol-5-yl)pyrimidine-2-amine

4-(2-Chloropyridin-5-yl)-5-cyano-N-(indazol-5-yl)pyrimidine-2-amine (522mg, 1.5mmol) and 3-(R)-dimethylaminopyrrolidine were heated together at 140° in a sealed flask for 2h. On cooling the reaction mixture was triturated with water to give a brown solid which was collected and subjected to column chromatography (silica gel; 1% triethylamine-10% methanol-89% dichloromethane) to give the title compound (417mg) as a yellow solid m.p.249-250^{o}. δH (d⁶ DMSO) 13.00 (1 H, br s), 10.32 (1 H, s), 8.81 (2H, d, J 9.2Hz), 8.15-8.12 (2H, m), 8.04 (1 H, s), 7.59 (1 H, m), 7.52 (1H, d, J 9.8Hz), 6.63 (1H, d, J 9.0Hz), 3.77-3.67 (2H, m), 3.41-3.24 (2H, m), 2.82 (1 H, br m), 2.22 (6H, s), 2.21 (1 H, m) and 1.84 (1 H,m).

The chloropyridine used as starting material was prepared from indazol-5-ylguanidinium nitrate (1.51g ,6.36 mmol), 1-(2-chloropyridin-5-yl)-2-cyano-3-dimethylaminopropen-1-one (1.50g, 6.36 mmol) and sodium hydroxide (254 mg, 6.36 mmol) to give the desired product (1.49g) as a white solid m.p. >285° (decomp).

The propenone was prepared from 3-(2-chloropyridin-5-yl)-3-oxopropionitrile (4.2g, 23.3mmol) and dimethylformamide diethylacetal (13ml) to give the desired material (5.05g) as an off-white solid m.p. 130-132°.

3-(2-Chloropyridin-5-yl)-3-oxopropionitrile was prepared by treating a solution of cyanoacetic acid (9.10g, 53.5mmol) and 2,2'-bipyridyl (5mg) in dry THF (500ml), cooled to -70^{o} under a nitrogen atmosphere, dropwise with n-butyllithium (85.6 ml, 214mmol of a 2.5M solution in hexane). The reaction was allowed to warm to 0° over a period of 1h and then recooled to -70°, at which point a solution of 6-chloronicotinyl chloride (9.42g, 53.5 mmol) in THF (75ml) was added to the resulting red slurry. The reaction was stirred at -70° for a further 1 h upon complete addition, and then allowed to reach 0° where upon 2M hydrochloric acid (250ml) was added. The reaction was extracted with chloroform (2 x 400ml) and the combined organic phases were then washed with saturated aqueous NaHCO₃ (1 x 250ml) and saturated brine (1 x 250ml), dried (MgSO₄) and concentrated under reduced pressure. The resulting solid was triturated with diethyl ether/n-hexane (1:5) to give the desired material (4.20g) as a pale yellow powder m.p. 122-1230.

Indazol-5-ylguanidinium nitrate was prepared from 5-aminoindazole (4.0g,mmol), cyanamide (1.89g, 45.1mmol) and concentrated HNO₃ (2.8ml) to give the desired material as a solid m.p. 252-254°.

### EXAMPLE 8

### 4-[4-(1-Amino-1-methylethyl)phenyl]-5-cyano-N-(indazol-5-yl)pyrimidine-2-amine

A mixture of indazol-5-ylguanidinium nitrate (524mg, 2.2mmol), 1-[4-(1-tert-butoxycarbonylamino-1-methylethyl)phenyl]-2-cyano-3-dimethylaminopropen-1-one (714mg, 2.0mmol) and powdered sodium hydroxide (96mg, 2.4mmol) in propan-2-ol (30ml) was heated at reflux for 6h. The reaction was concentrated *in vacuo* and the residue purified by column chromatography (silica, 60% ethyl acetate in hexane, loading the crude material in dichloromethane) to give 4-[4-(1-tert-butoxycarbonylamino-1-methylethyl)phenyl]-5-cyano-N-(indazol-5-yl)-pyrimidine-2-amine as a yellow solid (850mg). δH (CDCl₃) 8.69 (1 H, s), 8.18 (1 H, s), 8.11 (1 H, s), 8.05 (2H, d, J 8.4Hz), 7.93 (1H, bs), 7.57 (2H, d, J 8.4Hz), 7.50 (2H, m), 5.10 (1 H, bs), 1.66 (6H, s), 1.40 (9H, bs). MS (ESI) 492 (MNa+, 61%), 470 (MH+, 100%), 414 (19%). This product was dissolved in a mixture of TFA acid (20ml) and CH₂Cl₂ (20ml) and was stirred for 30 mins at room temperature before concentrating the reaction *in vacuo.* The residue was dissolved in 10% MeOH in CH₂Cl₂ (200ml) and the organic phase washed with sat. Na₂CO₃ (aq) (50ml), dried (MgSO₄) and concentrated *in vacuo* to give the title compound as a bright yellow solid (541mg) m.p. 267-271° (dec.). δH (d⁶ DMSO) 13.03 (1 H, bs), 10.46 (1 H, s), 8.91 (1H, s), 8.16 (1 H, s), 8.04 (1 H, s), 7.94 (2H, d, J 8.4Hz), 7.74 (2H, d, J 8.4Hz), 7.61 (1H, m), 7.52 (1 H, d, J 8.9Hz), 3.44 (2H, bs), 1.48 (6H, s). MS (ESI) 392 (MNa+, 11%), 370 (MH+, 23%), 353 (100%).

The 1-[4-(11-tert-butoxycarbonylamino-1-methylethyl)phenyl]-2-cyano-3-dimethyl-aminopropen-1-one used in the above process was prepared as follows:

A mixture of 4-acetylbenzonitrile (51.84g, 0.357mol) and N,N-dimethylformamide dimethyl acetal (142ml, 1.07mol) was heated to reflux for 1.5h. The reaction was cooled to room temperature and the resultant crystalline mass collected by filtration and washed with diethyl ether (4 x 100ml) to give 1-(4-cyanophenyl)-3-dimethylaminopropen-1-one as an orange solid (44.56g). An additional crop of this product (11.40g) could be obtained by partially concentrating the filtrate. δH (d⁶ DMSO) 8.01 (2H, d, J 8.2Hz), 7.87 (2H, d, J 8.2Hz), 7.75 (1 H, d, J 12.2Hz), 5.83 (1 H, d, J 12.2Hz), 3.15 (3H, bs), 2.92 (3H, bs). MS (ESI) 201 (MH+, 100%).

Hydroxylamine hydrochloride (21.40g, 308mmol) was added to a suspension of 1-(4-cyanophenyl)-3-dimethylaminopropen-1-one (55.96g, 280mmol) in MeOH (450ml) and the reaction stirred at room temperature for 24h. The reaction was diluted with water (400ml) and the resultant precipitate collected by filtration, washed with water (5 x 150ml) and dried *in vacuo* to give 4-(5-isoxazolyl)- benzonitrile as a pale yellow solid (42.53g) m.p. 148-149°. δH (CDCl₃) 8.35 (1 H, d, J 1.8Hz), 7.91 (2H, d, J 8.3Hz), 7.77, (2H, d, J 8.3Hz), 6.66 (1 H, d, J 1.8Hz).

Cerium trichloride heptahydrate (112.6g, 302mmol) was dried under vacuum (0.6mbar) at 140-150° (oil bath) for 4h in a flask fitted with a large magnetic stirring bar. The flask was refilled with nitrogen, cooled to 0° with an ice bath and anhydrous THF (500ml) introduced with stirring. On complete addition the ice bath was removed and the milky suspension stirred at room temperature for 16h. The reaction was cooled to -78° and methyl lithium (188ml of a 1.6M solution in diethyl ether, 300mmol) added dropwise with stirring. After 45mins a solution of 4-(5-isoxazolyl)-benzonitrile (17.0g, 100mmol) in anhydrous THF (100ml) was added and the reaction mixture left to warm in the cooling bath from -78° to -10° over 3h. The reaction was quenched with 33% ammonium hydroxide (250ml) and filtered through a pad of Celite® to remove the resultant solids. The Celite® pad was washed thoroughly with ethyl acetate (4 x100ml) and the combined filtrates concentrated to approximately 200ml. These filtrates were diluted with brine (200ml) and extracted with ethyl acetate (2 x 150ml), the organic extracts were dried (MgSO₄) and concentrated *in vacuo* to give 1-[4-(5-isoxazolyl)phenyl]-1-methylethylamine as a yellow solid (19.53g). δH (CDCl₃) 8.27 (1H, d, J 1.9Hz), 7.76 (2H, dt, J 8.7, 2.0Hz), 7.62 (2H, d, dt, J 8.7, 2.0Hz), 6.49 (1H, d, J 1.9Hz), 1.94 (2H, bs), 1.53 (6H, s). This compound was used in the following step without purification. A mixture of 1-[4-(5-isoxazolyl)phenyl]-1-methylethylamine (23.87g, 118.2mmol) and di-tert-butyl dicarbonate (28.37g, 130mmol) in toluene (200ml) was heated to reflux for 1h before removing solvent *in vacuo .* The resultant solid was recrystallised from ethanol to give tert-butyl N-{1-[4-(5-isoxazolyl)phenyl]-1-methylethyl} carbamate as bright yellow crystals (24.90g) m.p. 145-146°. δH (CDCl₃) 8.27 (1 H, d, J 1.8Hz), 7.75 (2H, d t, J 8.7, 2.1 Hz), 7.50 (2H, d t, J 8.7, 2.1 Hz), 6.49 (1 H, d, J 1.8Hz), 4.97 (1H, bs), 1.64 (6H, s), 1.37 (9H, bs). MS (ESI) 325 (MNa+,42%), 303 (MH+,56%), 186 (100%).

A freshly prepared solution of sodium ethoxide (3.77g, 164mmol of sodium in 150ml of ethanol) was added to a suspension of tert-butyl N-{1-[4-(5-isoxazolyl) phenyl]-1-methylethyl}carbamate (24.76g, 82mmol) in ethanol (150ml) and the reaction stirred at room temperature for 1h. Ethanol was removed *in vacuo* and the residue partitioned between ethyl acetate (150ml) and cold 1 M hydrochloric acid (250ml). The aqueous layer was re-extracted with ethyl acetate (2 x 80ml) and the combined ethyl acetate extracts washed with brine (100ml), dried (MgSO₄) and concentrated *in vacuo* to give tert-butyl N-{1-[4-(2-cyanoacetyl)phenyl]-1-methylethyl} carbamate as an off-white solid m.p. 122-123°. This crude product was dissolved in THF (150ml), N,N-dimethylformamide diethyl acetal (14.48g, 98.4mmol) added and the mixture heated to 50° for 1 h. Solvent was removed *in vacuo* and the residue purified by column chromatography (silica, 2-4% MeOH in CH₂Cl₂) to give 1-[4-(1-tert-butoxycarbonyl-amino-1-methylethyl)phenyl]-2-cyano-3-dimethylamino-propen-1-one as a pale yellow solid (24.46g) m.p.160-162°. δH (CDCl₃) 7.94 (1H, s), 7.77 (2H, dt, J 8.6,1.9Hz), 7.45 (2H, dt, J 8.6,1.9Hz), 5.08 (1 H, bs), 3.48 (3H, s), 3.28 (3H, s), 1.63 (6H, s), 1.32 (9H, bs). MS (ESI) 380 (MNa+, 63%), 358 (MH+, 32%), 302 (96%), 241 (100%).

### EXAMPLE 9

### 4-[4-(1-Amino-1-methylethyl)phenyl]-5-cyano-N-(3,4,5-trimethoxyphenyl) pyrimidine-2-amine

The title compound was prepared from 3,4,5-trimethoxyphenylguanidinium nitrate (576mg, 2.0mmol), 1-[4-(1-tert-butoxycarbonylamino-1-methylethyl) phenyl]-2-cyano-3-dimethylaminopropen-1-one (660mg, 1.8mmol) and powdered sodium hydroxide (89mg, 2.2mmol) following the method described for the compound of Example 8. This gave the intermediate 4-[4-(1-tert-butoxycabonylamino-1methylethyl)phenyl]-5-cyano-N-(3,4,5-trimethoxyphenyl)pyrimidine-2-amine as a yellow solid (769mg) after column chromatography (silica, 40% ethyl acetate in hexane). This compound was treated with TFA acid in CH₂Cl₂ as descibed for the analogous compound of Example 8 to give the title compound as a pale yellow solid (597mg) m.p.167-168°. δH (d⁶ DMSO) 10.34 (1H, bs), 8.90 (1H, s), 7.96 (2H, bd, J 7.8Hz), 7.73 (2H, d, J 8.2Hz), 7.24 (2H, bs), 3.76 (6H, s), 3.63 (3H, s), 3.18 (2H, bs), 1.42 (6H, s). MS (ESI) 442 (MNa+, 16%), 420 (MH+, 57%), 403 (100%).

Except where otherwise indicated, the following compounds of Examples 10 - 23 and their respective intermediates were prepared in a manner to the compound of Example 8 and its intermediates:

### EXAMPLE 10

### 4-[4-(1-Amino-1-methylethyl)phenyl]-5-cyano-N-[(4-imidazol-1-yl)phenyl] pyrimidine-2-amine

From 4-(imidazol-1-yl)phenylguanidinium nitrate (916mg, 2.8mmol), 1-[4-(1-tert-butoxycarbonylamino-1-methylethyl)phenyl]-2-cyano-3-dimethylaminopropenone (1.0g, 2.8mmol) and powdered sodium hydroxide (224mg, 3.6mmol) to give 4-[4-(1-tert-butoxycarbonylamino-1-methylethyl)phenyl]-5-cyano-N[(4-imidazol-1-yl)phenyl]pyrimidine-2-amine as a yellow solid (675mg) after column chromatography (silica, 2% MeOH in dichloromethane). This compound was treated with TFA acid in CH₂Cl₂ as described for Example 8 to give the title compound as a pale yellow solid (471mg) m.p. 232-233°. δH (d⁶ DMSO) 10.51 (1H, bs), 8.95 (1H, s), 8.19 (1H, s), 7.93-7.89 (4H, m), 7.76-7.61 (5H, m), 7.08 (1H, s), 2.20 (2H, bs) and 1.41 (6H, s).

### EXAMPLE 11

### 4-[4-(1-Amino-1-methylethyl)phenyl]-5-cyano-N-[4-(1,2,4-triazol-1-yl)phenyl]pyrimidine-2-amine

From 4-(1,2,4-triazol-1-yl)phenylguanidinium nitrate (750mg, 2.8mmol), 1-[4-(1-tert-butoxycarbonylamino-1-methylethyl)phenyl]-2-cyano-3-dimethylamino propenone (1.0g, 2.8mmol) and powdered sodium hydroxide (150mg, 3.75mmol) to give 4-[4-(1-tert-butoxycarbonylamino-1-methylethyl)phenyl]-5-cyano-N[4-(1,2,4-triazol-1-yl)phenyl]pyrimidine-2-amine as a yellow solid (380mg) after column chromatography (silica, 2% MeOH in dichloromethane). This compound was treated with TFA acid in CH₂Cl₂ as described for Example 8 to give the title compound as a pale yellow solid (224mg) m.p. 208-209°. δH (d⁶ DMSO) 10.67 (1H, bs), 9.21 (1 H, s), 8.97 (1 H, s), 8.20 (1 H, s), 7.97-7.94 (4H, m), 7.82 (2H, d, J 9.1 Hz), 7.76 (2H, d,J 8.9 Hz), 2.09 (2H, bs) and 1.41 (6H, s).

The guanidine used as starting material in the above process was prepared from 4-(1,2,4-triazol-1yl)aniline (1.60g, 10.0mmol). cyanimide (715mg, 17mmol) and concentrated HNO₃ (725mL, 11 mmol), in a manner similar to the corresponding starting material of Example 1, as a beige solid (1.50g) m.p. >310° (decomp).
4-(1,2,4-triazol-1yl)aniline was prepared by suspending 4-(1,2,4-triazol-1-yl)nitobenzene (2.25g, 11.83mmol) with 10% palladium on charcoal in ethanol (125ml), containing 4M hydrochloric acid (75mL). The resulting mixture was stirred under a hydrogen atmosphere at normal pressure and room temperature for 16h. The reaction was filtered through a pad of Celite® washing thoroughly with ethanol. The filtrate was concentrated to 50mL in volume and 2M NaOH added until the pH was >10. The solution was again concentrated to 50 mL and cooled to 0°. The resulting solid was collected by filtration and washed sparingly with water to give the desired material (1.65g) as an off-white solid, m.p. 150-152°.

The nitrobenzene used in the above process was prepared by heating a mixture of 4-fluornitrobenzene (40g, 28.3 mmol) and 1,2,4-triazole, sodium salt (28.4g, 31.2 mmol) in DMF (250mL) at 80o for 4h. On cooling the reaction was poured into cooled saturated brine (600mL) and 2M NaOH (400mL). The resulting solid was collected by filtration, washed with 2M NaOH (2 x 150mL), water (3 x 100mL) then ethanol (2 x 75mL) and dried under high vacuum. The product was purified by column chromatography (silica 3% MeOH in dichloromethane) to give the desired material as a yellow solid (9.05g).

### EXAMPLE 12

### 4-[4-(1-Amino-1-methylethyl)phenyl]-5-cyano-N-[4-(1,2,3-triazol-1-yl)phenyl]pyrimidine-2-amine

From 4-(1,2,3-triazol-1-yl)phenylguanidinium nitrate (750mg, 2.8mmol), 1-[4-(1-tert-butoxycarbonylamino-1-methylethyl)phenyl]-2-cyano-3-dimethyl aminopropenone (1.0g, 2.8mmol) and powdered sodium hydroxide (150mg, 3.75mmol) to give 4-[4-(1-tert-butoxycarbonylamino-1-methylethyl)phenyl]-5-cyano-N[4-(1,2,4-triazol-1-yl)phenyl]pyrimidine-2-amine as a yellow solid (380mg) after column chromatography (silica, 2% MeOH in dichloromethane). This compound was treated with TFA acid in CH₂Cl₂ as described for Example 8 to give the title compound as a pale yellow solid (224mg) m.p. 208-209°. δH (d⁶ DMSO) 10.67 (1 H, bs), 9.21 (1 H, s), 8.97 (1H, s), 8.20 (1 H, s), 7.97-7.94 (4H, m), 7.82 (2H, d, J 9.1 Hz), 7.76 (2H, d, J 8.9 Hz), 2.09 (2H, bs) and 1.41 (6H, s).

4-(1,2,3-triazol-1-yl)phenylguanidinium nitrate was prepared from 4-(1,2,3-triazol-1-yl)aniline (1.42g, 8.87mmol), cyanamide (635mg, 15.1mmol) and concentrated HNO₃ (645ml, 9.67mmol) in a manner similar to the corresponding starting material of Example 1, as a white solid (1.0g), m.p. >320°.

The aniline used in the above process was prepared by hydrogenation of 4-(1,2,3-triazol-1-yl)nitrobenzene (1.86g, 9.78mmol) in ethanol (75mL) over 10% palladium on charcoal (500mg) at atmospheric pressure and room temperature for 20h. The catalyst was removed by filtration through Celite® and the filtrate concentrated to give the desired product as an off-white solid (1.43g), m.p. 139-140°.

### EXAMPLE 13

### 4-[4-(1-Amino-1-methylethyl)phenyl]-5-cyano-N-(3,5-difluorophenyl) pyrimidine-2-amine

From 3,5-difluorophenylguanidinium nitrate (983mg, 2mmol), 1-[4-(1-tert-butoxycarbonylamino-1-methylethyl)phenyl]-2-cyano-3-dimethylaminopropenone (1.5g, 4.2mmol) and powdered sodium hydroxide (176mg, 4.3mmol) to give 4-[4-(1-tert-butoxycarbonylamino-1-methylethyl)phenyl]-5-cyano-N-[3,5-difluorophenyl] pyrimidine-2-amine as a yellow solid (510mg) after column chromatography (silica, 2% MeOH in dichloromethane). This compound was treated with TFA acid in CH₂Cl₂ as described for Example 8 to give the title compound as a pale yellow solid (332mg) m.p. 209°. δH (d⁶ DMSO) 9.06 (1 H, s), 8.00 (2H, d, J 8.5Hz), 7.78 (2H, d, J 8.5Hz), 7.62-7.54 (2H, m), 6.93-6.86 (1H, m) and 1.60 (6H, s).

### EXAMPLE 14

### 4-[4-(1-Amino-1-methylethyl)phenyl]-5-cyano-N-(3-fluoro-4-methyl phenyl)pyrimidine-2-amine

From 3-fluoro-4-methylphenylguanidinium nitrate (1.15g, 5.0mmol), 1-[4-(1-tert-butoxycarbonylamino-1-methylethyl)phenyl]-2-cyano-3-dimethyl aminopropenone (1.78g, 4.98mmol) and powdered sodium hydroxide (176mg, 4.2 mmol) to give 4-[4-(1-tert-butoxycarbonylamino-1-methylethyl)phenyl]-5-cyano-N-[3-fluoro-4-methylphenyl]pyrimidine-2-amine as a yellow solid (1.47g) after column chromatography (silica, 2% MeOH in dichloromethane). This compound was treated with TFA acid in CH₂Cl₂ as described for Example 8 to give the title compound as a pale yellow solid (1.16) m.p. 209°. δH (d⁶ DMSO) 8.96 (1H, s), 7.91 (2H,d, J 8.5Hz), 7.76 (2H,d, J 8.5Hz), 7.70 (1H,m), 7.46-7.43 (1H, m), 7.27-7.21 (1H, m) and 1.60 (6H, s).

### EXAMPLE 15

### 4-[4-(1-Amino-1-methylethyl)phenyl]-5-cyano-N-(3,4,5-trifluorophenyl) pyrimidine-2-amine

From 3,4,5-trifluorophenylguanidinium nitrate (1.06g, 4.2mmol), 1-[4-(1-tert-butoxycarbonylamino-1-methylethyl)phenyl]-2-cyano-3-dimethylaminopropenone (1.5g, 4.2mmol) and powdered sodium hydroxide (176mg, 4.2mmol) to give 4-[4-(1-tert-butoxycarbonylamino-1-methylethyl)phenyl]-5-cyano-N-[3,4,5-trifluoro-phenyl]pyrimidine-2-amine as a yellow solid (597mg) after column chromatography (silica, 2% MeOH in dichloromethane). This compound was treated with TFA acid in CH₂Cl₂ as described for Example 8 to give the title compound as a pale yellow solid (401 mg) m.p. 220°. δH (d⁶ DMSO) 9.01 (1 H, s), 7.91 (2H, m), 7.78-7.71 (4H, m) and 1.40 (6H, s).

### EXAMPLE 16

### 4-[4-(1-Amino-1-methylethyl)phenyl]-5-cyano-N-4-fluoro-3-trifluoro methylphenyl)pyrimidine-2-amine

From 4-fluoro-3-trifluoromethylphenylguanidinium nitrate (1.19g, 4.2mmol), 1-[4-(1-tert-butoxycarbonylamino-1-methylethyl)phenyl]-2-cyano-3-dimethylamino-propenone (1.5g, 4.2mmol) and powdered sodium hydroxide (176mg, 4.2mmol) to give 4-[4-(1-tert-butoxycarbonylamino-1-methylethyl)phenyl]-5-cyano-N-[4-fluoro-3-trifluoromethylphenyl]pyrimidine -2-amine as a yellow solid (648mg) after column chromatography (silica, 2% MeOH in dichloromethane). This compound (211 mg) was treated with TFA acid in CH₂Cl₂ as described for Example 8 to give the title compound as a pale yellow solid (157mg) m.p. 181°. δH (d⁶ DMSO) 8.91 (1H, s), 8.32 (1H, dd, J 6.5, 2.6Hz), 8.06-8.00 (1H, s), 7.99 (2H, d, J 8.8Hz), 7.77 (2H, d, J 8.8Hz), 7.44 (1 H, m) and 1.47 (6H, s).

### EXAMPLE 17

### 4-[4-(1-Amino-1-methylethyl)phenyl]-5-cyano-N-(2,4-difluorophenyl) pyrimidine-2-amine

From 2,4-difluorophenylguanidinium nitrate (0.98g, 4.2mmol), 1-[4-(1-tert-butoxycarbonylamino-1-methylethyl)phenyl]-2-cyano-3-dimethylamino propenone (1.5g, 4.2mmol) and powdered sodium hydroxide (176mg, 4.3mmol) to give 4-[4-(1-tert-butoxycarbonylamino-1-methylethyl)phenyl]-5-cyano-N-[2,4,-difluoro-phenyl]pyrimidine-2-amine as a yellow solid (648mg) after column chromatography (silica, 2% MeOH in dichloromethane). This compound was treated with TFA acid in CH₂Cl₂ as described for Example 8 to give the title compound as a pale yellow solid (460mg) m.p. 203°. δH (d⁶ DMSO) 8.65 (1H, s), 7.82 (2H, d, J 6.6Hz), 7.70 (2H, d, J 8.3Hz), 7.61-7.55 (1H, m), 7.38-7.32 (1H, m), 7.14-7.09 (1H, m) and 1.39 (6H, s).

### EXAMPLE 18

### 4-[4-(1-Amino-1-methylethyl)phenyl]-5-cyano-N-(3,4-difluorophenyl) pyrimidine-2-amine

From 3,4-difluorophenylguanidinium nitrate (0.98g, 4.2mmol), 1-[4-(1-tert-butoxycarbonylamino-1-methylethyl)phenyl]-2-cyano-3-dimethylamino propenone (1.5g, 4.2mmol) and powdered sodium hydroxide (176mg, 4.3mmol) to give 4-[4-(1-tert-butoxycarbonylamino-1-methylethyl)phenyl]-5-cyano-N-[3,4-difluorophenyl]pyrimidine-2-amine as a yellow solid (631mg) after column chromatography (silica, 2% MeOH in dichloromethane). This compound was treated with TFA acid in CH₂Cl₂ as described for Example 8 to give the title compound as a pale yellow solid (412mg) m.p. 192°. δH (d⁶ DMSO) 10.66 (1H, bs), 8.97 (1H, s), 7.99-7.92 (1H, m), 7.90 (2H, d, J 8.5hz), 7.76 (2H, d, J 8.5hz), 7.53-7.38 (2H, m) and 1.41 (6H, s).

### EXAMPLE 19

### 4-[4-(1-Amino-1-methylethyl)phenyl]-5-cyano-N-(3-chloro-4-fluorophenyl)pyrimidine-2-amine

From 3-chloro-4-fluorophenylguanidinium nitrate (1.05g, 4.2mmol), 1-[4-(1-tert-butoxycarbonylamino-1-methylethyl)phenyl]-2-cyano-3-dimethyl aminopropenone (1.5g, 4.2mmol) and powdered sodium hydroxide (176mg, 4.3 mmol) to give 4-[4-(1-tert-butoxycarbonylamino-1-methylethyl)phenyl]-5-cyano-N-[3-chloro-4-fluorophenyl]pyrimidine-2-amine as a yellow solid (895mg) after column chromatography (silica, 2% MeOH in dichloromethane). This compound was treated with TFA acid, in CH₂Cl₂ as described for Example 8 to give the title compound as a pale yellow solid (501 mg) m.p. 237°. δH (d⁶ DMSO) 10.60 (1H, bs), 8.97 (1H, s), 8.07 (1H, d, J 6.8, 2.5Hz), 7.91 (2H, dapp, J 8.5Hz), 7.75 (2H, d, J 8.5Hz), 7.73-7.69 (1 H, m), 7.41 (1 H, tapp, J 9.1 Hz) and 1.41 (6H, s).

### EXAMPLE 20

### 4-[4-(1-Amino-1-methylethyl)phenyl]-5-cyano-N-(4-fluorophenyl) pyrimidine-2-amine

From 4-fluorophenylguanidinium nitrate (0.91g, 4.2mmol), 1-[4-(1-tert-butoxycarbonylamino-1-methylethyl)phenyl]-2-cyano-3-dimethylamino propenone (1.5g, 4.2mmol) and powdered sodium hydroxide (176mg, 4.3mmol) to give 4-[4-(1-tert-butoxycarbonylamino-1-methylethyl)phenyl]-5-cyano-N-[4-fluorophenyl] pyrimidine-2-amine as a yellow solid (1.28g) after column chromatography (silica, 2% MeOH in dichloromethane). This compound was treated with TFA acid in CH₂Cl₂ as described for Example 8 to give the title compound as a pale yellow solid (607mg) m.p. 228-229 °. δH (d⁶ DMSO) 10.59 (1 H, s), 9.02 (1 H, s), 8.00 (2H, d, J 8.4Hz), 7.86-7.83 (4H, m), 7.32-7.27 (2H, m) and 1.51 (6H, s).

### EXAMPLE 21

### 4-[4-(1-Amino-1-methylethyl)phenyl]-5-cyano-N-(3-triftuoromethyl phenyl)pyrimidine-2-amine

From 3-trifluoromethylphenylguanidinium nitrate (1.12g, 4.2mmol), 1-[4-(1-tert-butoxycarbonylamino-1-methylethyl)phenyl]-2-cyano-3-dimethylamino propenone (1.5g, 4.2mmol) and powdered sodium hydroxide (176mg, 4.3mmol) to give 4-[4-(1-tert-butoxycarbonylamino-1-methylethyl)phenyl]-5-cyano-N-[3-trifluoromethyl] pyrimidine-2-amine as a yellow solid (1.32g) after column chromatography (silica, 2% MeOH in dichloromethane). This compound was treated with TFA in CH₂Cl₂ as described for Example 8 to give the title compound as a pale yellow solid (607mg) m.p. 192°. δH (d⁶ DMSO) 10.70 (1H, bs), 8.93 (1 H, s), 7.92 (1H, d, J 8.3Hz), 7.87 (2H, d, J 8.3Hz), 7.68 (2H, d, J 8.3Hz), 7.51 (1H, t, J 8.0Hz), 7.33 (1H, d, J 7.7Hz) and 1.34 (6H, s).

### EXAMPLE 22

### 4-[4-(1-Amino-1-methylethyl)phenyl]-5-cyano-N-(3-fluorophenyl) pyrimidine-2-amine

From 3-fluorophenylguanidinium nitrate (0.91g, 4.2mmol), 1-[4-(1-tert-butoxycarbonylamino-1-methylethyl)phenyl]-2-cyano-3-dimethylamino propenone (1.5g, 4.2mmol) and powdered sodium hydroxide (176mg, 4.3mmol) to give 4-[4-(1-tert-butoxycarbonylamino-1-methylethyl)phenyl]-5-cyano-N-[3-fluorophenyl] pyrimidine-2-amine as a yellow solid (381 mg) after column chromatography (silica, 2% MeOH in dichloromethane). This compound was treated with TFA acid in CH₂Cl₂ as described for Example 8 to give the title compound as a pale yellow solid (161mg) m.p. 209°. δH (d⁶ DMSO) 10.66 (1 H, s), 8.98 (1H, s), 7.92 (2H, dapp, J 8.5Hz), 7.76 (2H, dapp, J 8.5Hz), 7.74 (1 H, m), 7.56-7.54 (1 H, m), 7.40-7.34 (1 H, m), 6.91-6.86 (1 H, m), 2.48 (2H, bs) and 1.41 (6H, s).

### EXAMPLE 23

### 4-[4-(1-Amino-1-methylethyl)phenyl]-5-cyano-N-(phenyl)pyrimidine-2-amine

From phenylguanidinium nitrate (0.45g, 2.8mmol), 1-[4-(1-tert-butoxycarbonylamino-1-methylethyl)phenyl]-2-cyano-3-dimethylamino propenone (1.0g, 2.8mmol) and powdered sodium hydroxide (112mg, 2.8mmol) to give 4-[4-(1-tert-butoxycarbonylamino-1-methylethyl)phenyl]-5-cyano-N-[phenyl]pyrimidine-2-amine as a yellow solid (820mg) after column chromatography (silica, 2% MeOH in dichloromethane). This compound was treated with TFA acid in CH₂Cl₂ as described for Example 8 to give the title compound as a pale yellow solid (589mg) m.p. 303-304°. δH (d⁶ DMSO) 8.72 (1 H, s), 8.09 (2H, d, J 8.0Hz), 7.69-7.58 (5H, m), 7.43-7.38 (2H, m), 7.15 (1 H, t, J 7.7Hz), 2.32 (2H, bs) and 1.68 (6H, s).

### EXAMPLE 24

### 4-[4-(1-Amino-1-methylethyl)phenyl]-5-cyano-N-{4-[2-piperidin-1-ylethyl]phenyl}pyrimidine-2-amine

4-[4-(1-tert-butoxycarbonylamino-1-methylethyl)phenyl]-5-cyano-N-[4-(2-piperidin-1-ylethyl)phenyl]pyrimidine-2-amine (380mg) was stirred in 50% v/v CH₂Cl₂-TFA (5mL) at room temperature for 30min. The solvent was removed under reduced pressure and the resulting residue was redissolved in CH₂Cl₂, washed with saturated aqueous NaHCO₃, dried (MgSO₄) and evaporated to give the title compound (240mg) as a yellow solid, m.p.150°. δH (CDCl₃) 8.70 (1H, s), 8.01 (2H, d, J 8.0Hz), 7.73 (2H, d, J 8.0Hz), 7.55 (2H, d, J 8.0Hz), 7.22 (2H, d, J 8.0Hz), 2.84 (2H, m), 2.63 (2H, m), 2.52 (4H, m) and 1.51 (6H, s).

The pyrimidine used in the above process was prepared by stirring 4-[4-(1-tert-butoxycarbonylamino-1-methylethyl)phenyl]-5-cyano-N-[4-(2-p-toluene-sulphonyloxyethyl)phenyl]pyrimidine-2-amine (500mg, 0.8mmol) with piperidine (400ml, 4mmol) in DMF (5mL) at 70° for 4h. The solvent was then removed under reduced pressure and the resulting residue was redissolved in CH₂Cl₂, washed with saturated aqueous NaHCO₃, saturated brine, dried (MgSO₄) and concentrated under reduced pressure. The resulting residue was subjected to column chromatography (silica 7% MeOH in dichloromethane) to give the desired material (392mg) as a yellow solid, m.p. 142°. δH (CDCl₃) 8.67 (1H, s), 8.05 (2H, d, J 8.3Hz), 7.58-7.55 (4H, m), 7.22 (2H, d, J 8.5Hz), 5.00 (1 H, s), 2.88-2.82 (2H, m), 2.63-2.52 (6H, m), 1.66 (12H, bs) and 1.48-1.39 (9H, m).

The tosylate used in the above process was prepared by stirring 4-[4-(1-tert-butoxycarbonylamino-1-methylethyl)phenyl]-5-cyano-N-[4-(2-hydroxyethyl)phenyl]pyrimidine-2-amine (4.02g, 8.5mmol), 4-toluenesulphonyl chloride (2.43g, 12.7mmol) and 4-dimethylaminopyridine (150mg) in dichloromethane (70ml) at ambient temperature for 12h. The reaction was diluted with dichloromethane (70mL) and washed with 2M hydrochloric acid (150mL). The organic phase was separated and washed with 2M hydrochloric acid, brine and water, dried (MgSO₄) and concentrated under reduced pressure. The resulting residue was subjected to column chromatography (silica 40% ethyl acetate-hexane) to give the desired material (3.6g) as a yellow solid 134°. δH (CDCl₃) 8.68 (1 H, s), 8.05 (2H, m), 7.72 (2H, d, J 8.4Hz), 7.57 (4H, t, J 8.5Hz), 7.29 (2H, d, J 8.0Hz), 7.15 (2H, d, J 8.5Hz), 5.00 (1 H, s), 4.22 (2H, t, J 7.1Hz), 2.96 (2H, t, J 7.1Hz), 2.42 (3H, s), 1.67 (6H, bs) and 1.56 (9H).

The pyrimidine used in the above process was prepared from 4-(2-hydroxyethyl)phenylguanidinium nitrate (3.73g, 15.4mmol), 1-[4-(1-tert-butoxy-carbonylamino-1-methylethyl)phenyl]-2-cyano-3-dimethylamino propenone (5.0g, 14.0mmol) and powdered sodium hydroxide (672mg 16.8 mmol) to give the desired product (7.7g) as a pale yellow solid, m.p. 114°.

The following compounds of Examples 25-41 and their respective intermediates were prepared in an analogous manner to those in Example 24 except where othewise indicated:

### EXAMPLE 25

### 4-[4-(1-Amino-1-methylethyl)phenyl]-5-cyano-N-[4-(2-imidazol-1-ylethyl)phenyl]pyrimidine-2-amine

From 4-[4-(1-tert-butoxycarbonylamino-1-methylethyl)phenyl]-5-cyano-N-[4-(2-imidazol-1-ylethyl)phenyl]pyrimidine-2-amine (370mg,0.71mmol) to give the title compound (290mg) as a pale yellow solid m.p. 184°. δH (d⁶ DMSO) 10.51 (1H, s), 8.90 (1H, s), 8.02 (2H, d, J 8.0Hz), 7.73 (2H, d, J 8.0Hz), 7.62 (2H, d, J 8.0Hz), 7.41 (1 H, s), 7.13 (3H, m), 6.81 (1 H, s), 4.22 (2H, t, J 7.0Hz), 3.0 (2H, t, J 7.0Hz) and 1.6 (6H, s).

4-[4-(1-tert-Butoxycarbonylamino-1-methylethyl)phenyl]-5-cyano-N-[4-(2-imidazol-1-ylethyl)phenyl]pyrimidine-2-amine was prepared from 4-[4-(1-tert-butoxy carbonyl-amino-1-methylethyl)phenyl]-5-cyano-N-[4-(2-p-toluenesulphonyloxyethyl)phenylpyrimidine-2-amine (500mg,0.8mmol) and imidazole (272mg, 4.0mmol) as a yellow solid (380mg), m.p. 124°. δH (CDCl₃) 8.68 (1 H, s), 8.04 (2H, m), 7.66-7.56 (4H, m), 7.32 (1 H, s), 7.04 (3H, m), 6.85 (1H, t, J 1.3Hz), 5.03 (1 H, s), 4.18 (2H, t, J 7.0Hz), 3.05 (2H, t, J 7.0Hz), 1.66 (6H, bs) and 1.39 (9H, bs).

### EXAMPLE 26

### 4-[4-(1-Amino-1-methylethyl)phenyl]-5-cyano-N-[4-(2-morpholino ethyl)phenyl]pyrimidine-2-amine

From 4-[4-(1-tert-butoxycarbonylamino-1-methylethyl)phenyl]-5-cyano-N-[4-(2-morpholinoethyl)phenyl]pyrimidine-2-amine (430mg) to give the title compound (392mg) as a pale yellow solid m.p. 156°. δH (d⁶ DMSO) 10.40 (1H, s), 8.91 (1 H, s), 7.93 (2H, d, J 8.4Hz), 7.74 (2H, d, J 8.4Hz), 7.64 (2H, d, J 7.8Hz), 7.19 (2H, d, J 8.5Hz), 3.56 (4H, m), 3.36 (8H, bm), 2.70 (2H, t, J 7.5Hz), 2.51-2.46 (2H, m) and 1.49 (6H, s).

4-[4-(1-tert-Butoxycarbonylamino-1-methylethyl)phenyl]-5-cyano-N-[4-(2-morpholinoethyl)phenyl]pyrimidine-2-amine was prepared from 4-[4-(1-tert-butoxy carbonyl-amino-1-methylethyl)phenyl]-5-cyano-N-[4-(2-ptoluenesulphonyloxyethyl)phenyl]pyrimidine-2-amine (500mg, 0.80mmol) and morpholine (350µL, 4.0mmol) as a yellow solid (440mg), m.p. 200°. δH (CDCl₃ ) 8.66 (1H, s), 8.05 (2H, d, J 8.3Hz), 7.69 (1 H, s), 7.58-7.55 (4H, m), 7.21 (2H, d, J 8.5Hz), 5.04 (1H, s), 3.74 (4H, t, J4.7hz), 2.83-2.78 (2H, m), 2.63-2.57 (2H, m), 2.53 (4H, t, J 4.7Hz), 1.65 (6H, bs) and 1.38 (9H, bs).

### EXAMPLE 27

### 4-[4-(1-Amino-1-methylethyl)phenyl]-5-cyano-N-{4-[2-(2-ethylimidazol -1-yl)ethyl]phenyl}pyrimidine-2-amine

From 4-[4-(1-tert-butoxycarbonylamino-1-methylethyl)phenyl]-5-cyano-N-{4-[2-(2-ethylimidazol-1-yl)ethyl]phenyl}pyrimidine-2-amine to give the title compound (310mg) as a yellow solid m.p. 138°. δH (d⁶ DMSO) 10.51 (1 H, s), 8.91 (1 H, s), 8.00 (2H, d, J 8.0Hz), 7.71 (2H, d, J 8.0Hz), 7.62 (2H, d, J 8.0Hz), 7.11 (2H, d, J 8.0Hz), 7.03 (1H, s), 6.71 (1H, s), 4.12 (2H, t, J 7.0Hz), 3.33 (bs), 2.91 (2H, t, J 7.0Hz), 2.40 (2H, t, J 7.0Hz), 1.62 (6H, s) and 1.11 (3H, t, J 7.0Hz).

4-[4-(1-tert-Butoxycarbonylamino-1-methylethyl)phenyl]-5-cyano-N-{4-[2-(2-ethyl-imidazol-1-yl)ethyl]phenyl}pyrimidine-2-amine was prepared from 4-[4-(1-tert-butoxycarbonyl-amino-1-methylethyl)phenyl]-5-cyano-N-[4-(2-p[toluene-sulphonyloxyethyl)phenyl] pyrimidine-2-amine (500mg, 0.80mmol) and 2-ethylimidazole (383mg) to give the title compound (400mg) as a yellow solid, m.p. 210°. δH (CDCl₃) 8.68 (1H, s), 8.05 (2H, d, J 8.4Hz), 7.60-7.56 (5H, bm), 7.05 (2H, d, J 8.4Hz), 6.94 (1 H, d, J 1.3Hz), 6.76 (1 H, d, J 1.3Hz), 5.00 (1 H, s), 4.07 (2H, t, J 7.1 Hz), 3.00 (2H,t, J 7.1 Hz), 2.49 (2H, q, J 7.5Hz), 1.67 (6H, bs), 1.39-1.23 (9H,bm) and 0.88 (3H, t, J 7.0Hz).

### EXAMPLE 28

### 4-[4-(1-Amino-1-methylethyl)phenyl]-5-cyano-N-4-(2-imidazol-1-yl ethoxy)phenylpyrimidine-2-amine

From 4-[4-(1-tert-butoxycarbonylamino-1-methylethyl)phenyl]-5-cyano-N-[4-(2-imidazol-1-ylethoxy)phenyl]pyrimidine-2-amine (857mg,1.59mmol) to give the title compound (566mg) as a yellow solid, m.p. 208-209°. δH (d⁶ DMSO) 10.32 (1 H, bs), 8.86 (1 H, s), 7.89 (2H, d, J 8.5Hz), 7.73 (2H, d, J 8.5Hz), 7.67-7.63 (3H, m), 7.23 (1H, s), 6.94-6.88 (3H, m), 4.33 (2H, t, J 5.0Hz), 4.22 (2H, t, J 5.1 Hz), 2.01 (2H, bs) and 1.40 (6H, s).

4-[4-(1-tert-Butoxycarbonylamino-1-methylethyl)phenyl]-5-cyano-N-[4-(2-imidazol-1-yl-ethoxy)phenyl]pyrimidine-2-amine was prepared from 4-[4-(1-tert-butoxycarbonyl amino-1-methyl-ethyl)phenyl]-5-cyano-N-{4-[(2-p-toluene-sulphonyloxy)ethoxy]phenyl}pyrimidine-2-amine (2.0g, 3.1mmol) and imidazole (1.02g, 15mmol) as a yellow solid (870mg). δH (d⁶ DMSO) 10.34 (1 H, s), 8.88 (1 H, s), 7.90 (2H, d, J 8.5Hz), 7.68-7.64 (3H, m), 7.53 (2H, d, J 8.5Hz), 7.31 (1 H, bs), 7.24 (1H, t, J 1.1Hz), 6.93 (2H, d, J 9.0Hz), 6.89 (1 H, t, J 1.0Hz), 4.35 (2H, t, J 5.2Hz), 4.23 (2H, t, J 5.2Hz), 1.54 (6H, s) and 1.34 (9H, bs).

4-[4-(1-tert-butoxycarbonylamino-1-methylethyl)phenyl]-5-cyano-N-{4-[(2-p-toluenesulphonyloxy)ethoxyl]phenyl]pyrimidine-2-amine was prepared from 4-[4-(1-tert-butoxy-carbonylamino-1-methylethyl)phenyl]-5-cyano-N-[4-(2-hydroxyethoxy)phenyl]-pyrimidine-2-amine (2.1 g, 4.29mmol) and 4-toluene sulphonylchloride (1.24g, 6.5mmol) as a yellow solid (2.10g), m.p. 145-146°.

4-[4-(1-tert-butoxycarbonylamino-1-methylethyl)phenyl]-5-cyano-N-[4-(2-hydroxyethoxy)phenyl]pyrimidine-2-amine was prepared from 1-[4-(1-tert-butoxycarbonyl amino-1-methylethyl)phenyl]-5-cyano-3-dimethylaminopropenone (2.16g, 8.0mmol), 4-(2-hydroxyethoxy)phenylguanidinium nitrate (2.08g, 8.0mmol) and sodium hydroxide (320mg, 8.0mmol) as a pale green solid (2.28g), m.p. 126-127°. δH (CDCl₃) 8.64 (1 H, s), 8.06-8.02 (2H, m), 7.58-7.51 (5H, m), 6.97-6.94 (2H, m), 5.03 (1H, bs), 4.14 (2H,t, J 5.2Hz), 4.00-3.95 (2H, bm), 2.12 (1 H, bs), 1.68 (6H, s) and 1.38 (9H, bs).

The guanidine used in the above process was prepared by heating a solution of 4-(2-hydroxyethoxy)aniline (38.0g, 0.25mmol), cyanamide (17.67g, 0.421g) in 25mL water, and concentrated HNO₃ (17.8mL, 0.27mmol) in ethanol (350mL) for 24h. The reaction was cooled to 0° and diluted with ether (350mL). The resulting solid was collected by filtration and dried to give the desired material as a purple solid (42.45g). δH (d⁶ DMSO) 9.34 (1H, s), 7.18 (4H, bs), 7.16-7.12 (2H, m), 7.01-6.96 (2H, m), 4.85 (1 H, t, J 5.3Hz), 3.98 (2H, t, J 5.2Hz) and 3.70 (2H, t, J 5.0Hz).

### EXAMPLE 29

### 4-[4-(1-Amino-1-methylethyl)phenyl]-5-cyano-N-[3-(2-morpholino ethyl)phenyl]pyrimidine-2-amine

From 4-[4-(1-tert-butoxycarbonylamino-1-methylethyl)phenyl]-5-cyano-N-[3-(2-morpholinoethyl)phenyl]pyrimidine-2-amine (400mg, 0.73mmol) to give the title compound (250mg) as a pale yellow solid m.p. 166-167°. δH (CDCl₃) 8.69 (1 H, s), 8.05 (2H, d, J 8.5Hz), 7.68 (2H, d, J 8.7Hz), 7.61 (1 H, bs), 7.53-7.48 (2H, m), 7.30 (1 H, t, J 7.8Hz), 7.00 (1 H, d, J 7.5Hz), 3.76-3.73 (4H, m), 2.87-2.81 (2H, m), 2.67-2.61 (2H, m), 2.55-2.52 (4H, m), 1.83 (2H, bs) and 1.54 (6H, s).

4-[4-(1-tert-butoxycarbonylamino-1-methylethyl)phenyl]-5-cyano-N-[3-(2-morpholinoethyl)phenyl]pyrimidine-2-amine was prepared from 4-[4-(1-tert-butoxy carbonyl-amino-1-methylethyl)phenyl]-5-cyano-N-[3-(2-p-toluenesuphonyloxyethyl)phenyl]pyrimidine-2-amine (500mg, 0.84mmol) and morpholine (293µL, 3.36mmol) as a yellow solid (413mg). δH (CDCl₃) 8.68 (1H, s), 8.06 (2H, d, J 8.2Hz), 7.58-7.51. (5H, m), 7.30 (1 H, t, J 7.8Hz), 7.00 (1H, d, J 7.7Hz), 5.04 (1 H, bs), 3.76-3.73 (4H, m), 2.86-2.81 (2H, m), 2.66-2.61 (2H, m), 2.55-2.52 (4H, m), 1.65 (6H, s) and 1.39 (9H, bs).

4-[4-(1-tert-butoxycarbonylamino-1-methylethyl)phenyl]-5-cyano-N-[3-(2-p-toluenesuphonyloxyethyl)phenyl]pyrimidine-2-amine was prepared from 4-[4-(1-tert-butoxy-carbonylamino-1-methylethyl)phenyl]-5-cyano-N-[3-(2-hydroxyethyl phenyl]pyrimidine-2-amine (880mg) and 4-toluenesulphonylchloride (572mg, 3.0mmol) as yellow solid (945mg). δH (CDCl₃) 8.68 (1 H, s), 8.06 (2H, d, J 7.9Hz), 8.06 (2H, d, J 7.9Hz), 7.60-7.53 (4H, m), 7.43 (1 H, bs), 7.29-7.24 (3H, m), 6.90 (1 H, d, J 7.0Hz), 5.04 (1 H, bs), 4.26 (2H, t, J 6.8Hz), 2.98 (2H, t, J 7.0Hz), 2.39 (3H, s), 1.66 (6H, s) and 1.39 (9H, bs).

4-[4-(1-tert-butoxycarbonylamino-1-methylethyl)phenyl]-5-cyano-N-[3-(2-hydroxyethyl)phenyl]pyrimidine-2-amine was prepared from 4-[4-(1-tert-butoxycarbonyl amino-1-methyl-ethyl)phenyl]-5-cyano-N-[3-(hydroxyethoxy)phenyl] pyrimidine-2-amine (2.0g, 5.6mmol), 3-(2-hydroxyethyl)-phenylguanidinium nitrate (1.6g, 5.6mmol) and sodium hydroxide (336mg, 8.4mmol) as a yellow solid (980mg), m.p. 164-164°. δH (CDCl₃) 8.66 (1 H, s), 8.05 (2H, d, J 8.4Hz), 7.71 (1H, bs), 7.59-7.51 (4H, m), 7.32 (1H, t, J 7.9Hz), 7.01 (1H, d, J 7.7Hz), 5.06 (1H, bs), 3.89 (2H, t, J 6.5Hz), 2.89 (2H, t, J 6.5Hz), 1.65 (6H, s) and 1.39 (9H, bs).

### EXAMPLE 30

### 5-Cyano-4-phenyl-N-[4-(2-piperidin-1-ylethyl)phenyl]pyrimidine-2-amine

From 5-cyano-4-phenyl-N-[4-(2-p-toluenesulphonyloxyethyl)phenyl] pyrimidine-2-amine (800mg, 1.70mmol) and piperidine (0.84mL,8.5mmol) to give the title compound (367mg) as a pale yellow solid, m.p. 124-125°. δH (CDCl₃) 8.69 (1 H, s), 8.05 (2H, d, J 6.8Hz), 7.60-7.51 (6H, m), 7.22 (2H, d, J 8.4Hz), 2.87-2.83 (2H, m), 2.61-2.50 (6H, m), 1.66 (4H, bs) and 1.48-1.43 (2H, m).

5-Cyano-4-phenyl-N-[4-(2-p-toluenesulphonyloxyethyl)phenyl]pyrimidine-2-amine was prepared from 5-cyano-4-phenyl-N-[4-(2-hydroxyethyl)-phenyl]pyrimidine-2-amine (3.30g, 10.43mmol) and 4-toluenesulphonylchloride (2.19g, 11.47mmol) as a pale yellow solid (3.91 g) m.p. 134-135°. δH (d⁶ DMSO) 8.69 (1 H, s), 8.05 (2H, dd, J 6.0, 2.0Hz), 7.72 (2H, d, J 8.3Hz), 7.61-7.51 (6H, m), 7.29 (2H, d, J 8.1 Hz), 7.14 (2H, d, J 8.5Hz), 4.21(2H, t, J 7.0Hz), 2.95 (2H, t, J 7.0Hz) and 2.41 (3H, s). 5-Cyano-4-phenyl-N-[4-(2-hydroxyethyl)phenyl]pyrimidine-2-amine was prepared from 1-phenyl-2-cyano-3-dimethylaminopropen-1-one (4.0g, 20mmol), 4-(2-hydroxyethyl)phenyl-guanidinium nitrate (4.24g,20mmol) and sodium hydroxide (800mg, 20.0mmol) as a yellow solid (3.50g), m.p. 142-143°. δH (CDCl₃) 8.68 (1H, s), 8.03 (2H, dd, J 6.0, 2.0Hz), 7.66 (1H, bs), 7.59-7.51 (5H, m), 7.23 (2H, m), 3.88 (2H, t, J 6.5Hz), 2.87 (2H, t, J 6.5Hz) and 1.62 (2H, bs).

### EXAMPLE 31

### 5-Cyano-4-phenyl-N-[4-(2-imidazol-1-ylethyl)phenyl]pyrimidine-2-amine

From 5-cyano-4-phenyl-N-[4-(2-p-toluenesulphonyloxyethyl)phenyl]-pyrimidine-2-amine (800mg, 1.70mmol) and imidazole (578mg, 8.50mmol) to give the title compound (378mg) as a yellow solid m.p. 210-211°. δH (CDCl₃/d⁶ DMSO) 10.28 (1 H, bs), 8.74 (1 H, s), 7.99 (1 H, s), 7.94 (1 H, d, J 6.7Hz), 7.65 (2H, d, J 8.74Hz), 7.57-7.49 (2H, d, J 6.7Hz), 7.03 (2H, d, J 8.4Hz), 6.98 (1H, s), 6.85 (1H, t, J 0.9Hz), 4.16 (2H, t, J 7.1 Hz) and 2.97 (2H, t, J 7.1 Hz).

### EXAMPLE 32

### 5-Cyano-4-phenyl-N-{4-[2-(2-ethylimidazol-1-yl)ethyl]phenyl}-pyrimidine-2-amine

From 5-cyano-4-phenyl-N-[4-(2-p-toluenesulphonyloxyethyl)phenyl]-pyrimidine-2-amine (800mg, 1.70mmol) and 2-ethylimidazole (817mg, 8.5mmol) to give the title compound (480mg) as a yellow solid, m.p. 190-192°. δH (CDCl₃) 8.72 (1H, s), 8.07 (2H, dd, J 5.4, 1.4Hz), 7.75 (1H, bs), 7.62-7.54 (5H, m), 7.06 (2H, d, J 8.5Hz), 6.98 (1 H, d, J 1.3Hz), 6.79 (1 H, d, J 1.3Hz), 4.09 (2H, t, J 7.0Hz), 3.02 (2H, t, J 7.0Hz), 2.51 (2H, q, J 7.6Hz) and 1.27 (3H, t, J 7.6Hz).

### EXAMPLE 33

### 5-Cyano-4-phenyl-N-{4-[2-(1,2,4-triazol-1-ylethyl)phenyl}pyrimidine-2-amine

From 5-cyano-4-phenyl-N-[4-(2-p-toluenesulphonyloxyethyl)phenyl]-pyrimidine-2-amine (500mg,1.12mmol) and 1,2,4-triazole, sodium salt (122mg, 1.35mmol) to give the title compound (188mg) as a yellow solid, m.p. 217-218°. δH (d⁶ DMSO) 10.48 (1H, s), 8.94 (1H, s), 8.33 (1H, s), 7.96 (3H, m), 7.68-7.59 (5H, m), 7.11 (2H, d, J 8.5Hz), 4.42 (2H, t, J 7.1 Hz) and 3.08 (2H, t, J 7.1 Hz).

### EXAMPLE 34

### 5-Cyano-4-phenyl-N-[4-(2-imidazol-1-ylethoxy)phenyl]pyrimidine-2-amine

From 5-cyano-4-phenyl-N-[4-(2-p-toluenesulphonyloxyethoxy)phenyl]-pyrimidine-2-amine (1.54g,3.17mmol) and imidazole (1.08g, 15.8mmol) to give the title compound (790mg) as a yellow solid, m.p. 185°. δH (CDCl₃) 8.67 (1 H, s), 8.04 (2H, d, J 8.1Hz), 7.60 (1 H, s), 7.58-7.52 (5H, m), 7.45 (1H, s), 7.07 (1 H, s), 7.05 (1 H, t, J 1.2Hz), 6.91 (1 H, d, J 2.2Hz), 6.88 (1 H, d, J 2.2Hz), 4.35 (2H, t, J 5.0Hz) and 4.23 (2H, t, J 5.0Hz).

5-cyano-4-phenyl-N-[4-(2-p-toluenesulphonyloxyethoxy)phenyl] pyrimidine-2-amine was prepared from 5-cyano-4-phenyl-N-[4-(2-hydroxyethoxy)phenyl]-pyrimidine-2-amine (1.42g,4.28mmol) and 4-toluenesulphonyl chloride (1.23g, 6.4mmol) as a yellow solid, m.p. 147°. δH (CDCl₃) 8.67 (1H, s), 8.05-8.03 (2H, m), 7.83 (2H, dd, J 6.5, 1.8Hz), 7.58-7.49 (6H, m), 7.35 (2H, dd, J 8.6, 0.9Hz), 6.83 (2H, d, J 0.9Hz), 4.38-4.36 (2H, m), 4.18-4.16 (2H, m) and 2.45 (3H, s).

5-cyano-4-phenyl-N-[4-(2-hydroxyethoxy)phenyl]pyrimidine-2-amine was prepared from 1-phenyl-2-cyano-3-dimethylaminopropen-1-one (1.41g, 7.0mmol), 4-(2-hydroxy-ethoxy) phenylguanidinium nitrate (2.0g, 7.7mmol) and sodium hydroxide (340mg, 8.4mmol) to give a yellow solid (1.54g), m.p. 151°. δH (CDCl) 8.67 (1H, s), 8.04 (2H, d, J 7.7Hz), 7.58-7.52 (5H, m), 7.45 (1H, s), 6.95 (2H, dd, J 6.7, 2.3Hz), 4.12-4.09 (2H, m), 4.00-3.97 (2H, m) and 2.05-2.01 (1 H, m).

### EXAMPLE 35

### 5-Cyano-N-{4-[2-(2-ethylimidazol-1-yl)ethoxy]phenyl}-4-thien-2-yl Pyrimidine-2-amine

From 5-cyano-N-[4-(2-p-toluenesulphonyloxyethoxy)phenyl]-4-thien-2-yl pyrimidine-2-amine (1.5g, 3.0mmol) and 2-ethylimidazole (1.46g, 15.2mmol) to give the title compound (0.94g) as a pale yellow solid, m.p. 205°. δH (CDCl₃) 8.59 (1 H, s), 8.41 (1 H, d, J 4.0Hz), 7.63 (1 H, d, J 5.0Hz), 7.53 (2H, m), 7.37 (1 H, s), 7.22 (1 H, m), 6.97 (2H, d, J 6.5Hz), 6.88 (2H, d, J 9.0Hz), 4.24 (4H, dd, J 6.8, 4.3Hz), 2.78 (2H, q, J 7.8Hz) and 1.39 (3H, t, J 7.5Hz).

5-cyano-N-[4-(2-p-toluenesulphonyloxyethoxy)phenyl]-4-thien-2-ylpyrimidin-2-amine was prepared from 5-cyano-N-[4-(2-hydroxyethoxy)-phenyl]-4-thien-2-ylpyrimidine-2-amine (8.02g, 23.7mmol) and 4-toluenesulphonylchloride (9.0g, 47.4mmol) to give a yellow solid (4.97g), m.p. 160°. δH (d⁶ DMSO) 10.32 (1H, s), 8.84 (1H, s), 8.26 (1H, d, J 3.9Hz), 7.99 (1 H, d, J 5.0Hz), 7.80 (2H, d, J 8.3Hz), 7.64 (2H, m), 7.47 (2H, d, J 8.3hz), 7.33 (1H, t, J 4.5Hz), 6.85 (2H, d, J 9.0Hz), 4.33 (2H, t, J 4.0Hz), 4.15 (2H, m) and 2.41 (3H, s).

### EXAMPLE 36

### 5-Cyano-N-[4-{2-(2-methylimidazol-1-ylethoxy}phenyl]-4-thien-2-ylpyrimidine-2-amine

From 5-cyano-N-[4-(2-p-toluenesulphonyloxyethoxy)phenyl]-4-thien-2-yl pyrimidin-2-amine (2.0g, 4.07mmol) and 2-methylimidazole (1.67g, 5mmol) to give the title compound (0.87g) as a yellow solid, m.p. 190°. δH (d⁶ DMSO) 10.30 (1H, bs), 8.83 (1 H, s), 8.25 (1 H, d, J 3.8Hz), 7.97 (1 H, d, J 5.0Hz), 7.65 (1H, bs), 7.32 (1H, t, J 4.0hz), 7.10 (1H, s), 6.93 (2H, d, J 8.7Hz), 6.72 (1 H, s), 4.23 (4H, m) and 2.32 (3H, s).

### EXAMPLE 37

### 5-Cyano-N-[4-(2-imidazol-1-ylethoxy)phenyl]-4-thien-2-ylpyrimidine-2-amine

From 5-cyano-N-[4-(2-p-toluenesulphonyloxyethoxy)phenyl]-4-thien-2-yl pyrimidin-2-amine (2.45g,5.0mmol) and imidazole (1.7g, 25mmol) to give the title compound (1.0g) as a yellow solid, m.p. 199-200°. δH (d⁶ DMSO) 9.90 (1 H, bm), 8.76 (1 H, d, J 1.0Hz), 8.26 (1 H, m), 7.92 (1H, dd, J 5.0, 1.1Hz), 7.65 (3H, m), 7.31 (1H, dd, J 5.0, 3.8Hz), 7.20 (1H, t, J 1.2Hz), 6.94 (3H, m), 4.36 (2H, m) and 4.30 (2H, m).

### EXAMPLE 38

### 5-Cyano-N-[4-(2-(1,2,4-triazol-1-yl)ethoxy)phenyl]-4-thien-2-ylpyrimidine-2-amine

From 5-cyano-N-[4-(2-p-toluenesulphonyloxyethoxy)pheny[l-4-thien-2-yl pyrimidin-2-amine (1.29g, 3.6mmol) and 1,2,4-triazole sodium salt (990mg, 10.9mmol) to give the title compound (500mg) as a yellow solid, m.p. 180-182°. δH (d⁶ DMSO) 8.84 (1 H, s), 8.56 (1 H, s), 8.24 (1H, d, J 4.0Hz), 7.98-7.99 (2H, m), 7.63 (2H, bs), 7.32 (1 H, dd, J 4.0Hz), 6.92 (2H, d, J 8.8Hz), 4.57 (2H, t, J 5.1 Hz) and 4.34 (2H, t, J 5.1 Hz).

### EXAMPLE 39

### 5-Cyano-N-[4-(2-(1,3,4-triazol-1-yl)ethoxy)phenyl]-4-thien-2-ylpyrimidine-2-amine

From the same reactants in Example 38 and produced as as a side product the title compound was obtained (100mg) as a yellow solid, m.p. 228°. δH (d⁶ DMSO) 8.84 (1H, s), 8.56 (2H, s), 8.25 (1 H, d, J 3.9Hz), 7.98 (1 H, d, J 4.9Hz), 7.65 (2H, bs), 7.33 (1 H, t, J 4.5Hz), 6.95 (2H, d, J 8.9Hz), 4.44 (2H, t, J 5.0Hz) and 4.26 (2H, t, J 5.0Hz).

### EXAMPLE 40

### 5-Cyano-N-{4-[2-(1H-imidazol-2-ylamino)ethoxy]phenyl}-4-thien-2-ylpyrimidine-2-amine

From 5-cyano-N-[4-(2-p-toluenesulphonyloxyethoxy)phenyl]-4-thien-2-yl pyrimidin-2-amine (500mg, 0.78mmol), and 2-aminoimidazole sulphate (500mg, 3.78mmol) and potassium carbonate (522mg, 3.78mmol) to give the title compound (170mg) as a yellow solid, m.p. 140°. δH 8.84 (1 H, s), 8.25 (1H, d, J 4.0Hz), 7.98 (1H, d, J 5.0Hz), 7.76-7.60 (2H, m), 7.35-7.32 (1 H, m), 6.93 (2H, d, J 8.7Hz), 6.64 (1 H, s), 6.36 (1H, s), 5.34 (1 H, m) and 4.08 (4H, m).

### EXAMPLE 41

### 5-Cyano-N-[4-(2-imidazol-1-ylethylphenyl]-4-thien-2-ylpyrimidine-2-amine

From 5-cyano-[4-thien-2-yl-N-[4-(2-p-toluenesulphonyloxyethyl)phenyl] pyrimidine-2-amine (550mg, 1.15mmol) and imidazole (393mg,5.77mmol) to give the title compound (300mg) as a yellow solid, m.p. 187°. δH (CDCl₃) 8.62 (1 H, s), 8.43 (1 H, dd, J 3.2, 1.0Hz), 7.65 (1 H, dd, J 5.9, .0Hz), 7.59 (2H, d, J 8.2Hz), 7.51 (1H, s), 7.35 (1H, s), 7.24 (1 H, dd, J 4.0,1.0Hz), 7.10-7.05 (3H, m), 6.85 (1H, s), 4.19 (2H, t, J 7.0Hz) and 3.06 (2H, t, J 7.0Hz).

5-Cyano-4-thien-2-yl-N-[4-(2-p-toluenesulphonyloxyethyl)phenyl] pyrimidine-2-amine was prepared from 5-cyano-N-[4-(2-hydroxyethyl)-phenyl]-4-thien-2-ylpyrimidine-2-amine (1.39g, 4.30mmol) and 4-toluenesulphonylchloride (1.23g, 6.5mmol) as a yellow solid (1.15g), m.p. 190°. δH (CDCl₃) 8.62 (1 H, s), 8.43 (1H, dd, J 3.1, 1.0Hz), 7.66-7.61 (3H, m), 7.45 (1 H, s), 7.28-7.22 (3H, m), 3.73 (2H, t, J 7.3Hz) and 3.09 (2H, t, J 7.3Hz).

5-cyano-N-[4-(2-hydroxyethyl)phenyl]-4-thien-2-ylpyrimidine-2-amine was prepared from 1-thien-2-yl-2-cyano-3-dimethylaminopropen-1one (2.17g, 10.5mmol), 4-(2-hydroxy-ethyl)phenylguanidinium nitrate (2.8g, 11.6mmol) and powdered sodium hydroxide (505mg) as a yellow solid, m.p. 178°. δH (d⁶ DMSO) 10.36 (1 H, s), 8.86 (1 H, s), 8.26 (1 H, m), 8.00 (1 H, d, J 4.1 Hz), 7.64 (2H, m), 7.33 (1 H, dd, J 4.0, 1.0Hz), 7.20 (2H, d, J 8.4Hz), 4.60 (1 H, t, J 5.2Hz), 3.58 (2H, t, J 7.0Hz) and 2.69 (2H, t, J 7.0Hz).

The compounds following Examples 42-71 were prepared in a similar manner to the compound of Example 1:

### EXAMPLE 42

### 5-Cyano-N-[4-(imidazol-1-yl)phenyl]-4-phenylpyrimidine-2-amine

From 1-phenyl-2-cyano-3-dimethylaminopropen-1-one (1.5g, 7.49mmol), 4-imidazol-1-ylphenylguanidinium nitrate (2.45g,7.40mmol) and sodium hydroxide (600mg, 15mmol) to give the title compound (1.40g) as a yellow solid, m.p. 278-280°. δH 10.69 (1H, bs), 9.00 (1H, s), 8.21 (1H, s), 7.98-7.91 (4H, m), 7.70-7.60 (6H, m) and 7.10 (1 H, s).

### EXAMPLE 43

### 5-Cyano-N-[4-(2-dimethylaminoethoxy)phenyl]-4-phenyl pyrimidine-2-amine

From 1-phenyl-2-cyano-3-dimethylaminopropen-1-one (250mg,1.25mmol), 4-(2-dimethylaminoethoxy)phenylguanidinium nitrate (523mg,1 .25mmol) and sodium hydroxide (108mg, 2.7mmol) to give the title compound (230mg) as a yellow solid, m.p. 152-153°. δH (d⁶ DMSO) 10.37 (1H, s), 8.40 (1H, s), 7.94 (2H, m), 7.62 (5H, m), 6.94 (2H, dt, J 9.0, 2.0Hz), 4.02 (2H, t, J 5.8Hz), 2.61 (2H, t, J 5.8Hz) and 2.21 (6H, s).

### EXAMPLE 44

### 5-Cyano-N-[3,5-dimethyl-4-(2-morpholinoethoxy)phenyl]-4-phenylpyrimidine-2-amine

From 1-phenyl-2-cyano-3-dimethylaminopropen-1-one (250mg, 1.25mmol), 3,5-dimethyl-4-(2-morpholinoethoxy)phenylguanidinium nitrate (523mg, 1.25mmol) and sodium hydroxide (108mg, 2.7mmol) to give the title compound (317mg) as a yellow solid, m.p. 160-162°. δH (d⁶ DMSO) 10.32 (1H, s), 8.92 (1H, s), 7.96 (2H, m), 7.64-7.59 (3H, m), 7.42 (2H, bs), 3.83 (2H, t, J 5.7Hz), 3.59 (4H, t, J 4.6Hz), 2.68 (2H, t, J 5.7Hz), 2.50 (4H, m) and 2.23 (6H, s).

### EXAMPLE 45

### 5-Cyano-N-[3,5-dimethoxyphenyl]-4-phenylpyrimidine-2-amine

From 1-phenyl-2-cyano-3-dimethylaminopropen-1-one (250mg,1.25mmol), 3,5-dimethoxyphenylguanidinium nitrate (321 mg, 1.4mmol) and sodium hydroxide (56mg, 1.4mmol) to give the title compound (280mg) as a yellow solid m.p. 206-207°. δH (d⁶ DMSO) 10.47 (1H, s), 8.98 (1 H, s), 7.99 (2H, m), 7.63 (3H, m), 7.13 (2H, bs), 6.25 (1 H, t, J 2.2Hz) and 3.78 (3H, s).

### EXAMPLE 46

### 5-Cyano-N-[3,4-dimethoxyphenyl]-4-phenylpyrimidine-2-amine

From 1-phenyl-2-cyano-3-dimethylaminopropen-1-one (250mg, 1.25mmol), 3,4-dimethoxyphenylguanidinium nitrate (348mg, 1.25mmol) and sodium hydroxide (56mg) to give the title compound (107mg) as an orange solid, m.p. 155-157°. δH (d⁶ DMSO) 9.78 (1H, bs), 8.80 (1H, s), 8.00 (2H, m), 7.63-7.51 (3H, m), 7.25 (1 H, dd, J 8.7, 2.5mmol), 6.94 (1 H, d, J 8.7mmol), 3.79 (3H, s) and 3.78 (3H, s).

### EXAMPLE 47

### 5-Cyano-4-phenyl-N-[phenyl]pyrimidine-2-amine

From 1-phenyl-2-cyano-3-dimethylaminopropen-1-one (1.0g, 5.0mmol), phenylguanidinium nitrate (830mg, 2.5mmol) and sodium hydroxide (200mg, 5.0mmol) to give the title compound (660mg) as a yellow solid, m.p. 160-161°. δH (CDCl₃) 8.71 (1H, s), 8.08-8.05 (2H, m), 7.66-7.51 (6H, m), 7.42-7.36 (2H, m) and 7.19-7.13 (1 H, m).

### EXAMPLE 48

### 5-Cyano-4-indol-3-yl-N-(3,4,5-trimethoxyphenyl)pyrimidine-2-amine

From 1-indol-3-yl-2-cyano-3-dimethylaminopropen-1-one (1.0g, 4.18mmol), 3,4,5-trimethoxyphenylguanidinium nitrate (1.20g, 4.18mmol) and sodium hydroxide (167mg, 4.18mmol) to give the title compound (475mg) as a yellow solid, m.p. 200-201°. δH (d⁶ DMSO) 12.04 (1H, bs), 10.04 (1H, s), 8.77 (1 H, s), 8.52 (1 H, s), 8.50 (1 H, bs), 7.52 (1H, d, J 8.1 Hz), 7.24 (2H, t, J 7.4Hz), 7.12 (2H, bs), 3.70 (6H, s) and 3.66 (3H, s).

1-indol-3-yl-2-cyano-3-dimethylaminopropen-1-one was prepared from 3-(cyanoacetyl) indole (4.0g, mmol) and dimethylformamide dimethylacetal (4.1 mL, 23.9mmol) as a yellow solid (2.4g), m.p. 187-188°. δH (d⁶ DMSO) 11.73 (1 H, bs), 8.26 (1 H, s), 8.12 (1 H, dd, J 6.8, 1.3Hz), 7.98 (1 H, s), 7.47-7.44 (1 H, m), 7.20-7.09 (2H, m), 3.35 (3H, s) and 3.26 (3H, s).

3-(Cyanoacetyl)indole was prepared by suspending indole (11.71g, 0.10mmol) and potassium cyanoacetate (24.6g, 0.2mmol) in acetonitrile (300mL), and to this methanesulphonylchloride (7.7mL, 0.1mmol) was added. The resulting mixture was stirred at ambient temperature for 1 h, and then sodium carbonate (10g in 50mL water) was added. After 5 min, the organic phase was separated, dried (Na₂SO₄) and concentrated under reduced pressure. The residue was recrystallised from ethanol (100mL) to give the desired material as an orange solid (4.31g), m.p. 226-227°. δH (d⁶ DMSO) 12.20 (1 H, bs), 8.38 (1 H, d, J 2.8Hz), 8.14-8.10 (1 H, m), 7.53-7.50 (1 H, m), 7.25-7.21 (2H, m) and 4.49 (2H, s).

### EXAMPLE 49

### 5-Cyano-4-indol-3-yl-N-[4-(1,2,4-triazol-1-yl)phenyl]pyrimidine-2-amine

From 1-indol-3-yl-2-cyano-3-dimethylaminopropen-1-one (289mg, 1.2 mmol), 4-(1,2,4-triazol-1-yl)phenylguanidinium nitrate (320mg,1.2mmol) and sodium hydroxide (48mg, mmol) to give the title compound (270mg) as a yellow solid, m.p. 329-330°. δH (d⁶ DMSO) 12.00 (1H, bs), 10.39 (1 H, bs), 9.26 (1 H, s), 8.84 (1 H, s), 8.55 (2H, bm), 8.28-8.21 (1H, m), 7.96 (2H, d, J 8.8Hz), 7.86 (2H, d, J 8.8Hz), 7.56-7.52 (1H, m), 7.28-7.15 (2H, m) and 3.30 (1 H, bs).

### EXAMPLE 50

### 5-Cyano-4-thiazol-2-yl-N-(3,4,5-trimethoxyphenyl)pyrimidine-2-amine

From 2-cyano-3-dimethylamino-1-thiazol-2-ylpropen-1-one (300mg, 13.8 mmol), 3,4,5-trimethoxyphenylguanidinium nitrate (436mg, 15.2mmol) and sodium hydroxide (61 mg, 15.2mmol) to give the title compound (324mg) as a yellow solid, m.p. 223°. δH (d⁶ DMSO) 10.52 (1H, bs), 9.03 (1H, s), 8.28 (1 H, d, J 3.0Hz), 8.22 (1 H, d, J 3.0Hz), 7.23 (2H, s), 3.88 (6H, s) and 3.72 (3H, s).

2-Cyano-3-dimethylamino-1-thiazol-2-ylpropen-1-one was prepared from 2-cyanoacetyl thiazole (400mg,2.94mmol) and dimethylformamide dimethylacetal (1.5mL) as a yellow solid, m.p. 126° δH (CDCl₃) 8.89 (1 H, s), 7.96 (1 H, d, J 3.3Hz), 7.60 (1 H, d, J 3.1 Hz), 3.54 (3H, s) and 3.35 (3H, s).

2-Cyanoacetylthiazole was prepared by heating a solution of 2-ethoxycarbonyl-thiazole (1.08g, 6.88mmol), acetonitrile (360mL, 7.57mmol) and sodium hydride [60% dispersion in oil] (303mg, 7.57mmol) in benzene (20mL) and DMF (2mL) at 80° for 90 min. On cooling the resulting precipitate was collected by filtration and dissolved in water (60mL). The solution was poured into cold 2M hydrochloric acid (100mL) and the resulting precipitate collected by filtration to give the desired product (200mg) as a yellow solid, m.p. 109° δH (CDCl₃) 8.06 (1 H, d, J 2.9Hz), 7.82 (1 H, d, J 2.9Hz) and 4.32 (2H, s).

### EXAMPLE 51

### 5-Cyano-4-thiazol-2-yl-N-[4-(1,2,4-triazol-1-yl)phenyl]pyrimidine-2-amine

From 2-cyano-3-dimethylamino-1-thiazol-2-ylpropen-1-one (300mg, 1.38 mmol), 4-(1,2,4-triazol-1-yl)phenylguanidinium nitrate (425mg, 1.38mmol) and sodium hydroxide (61 mg, 1.52mmol) to give the title compound (381 mg) as a yellow solid, m.p. 331°. δH (d⁶ DMSO) 10.86 (1H, bs), 9.30 (1 H, s), 9.10 (1 H, s), 8.31-8.25 (3H, m), 8.01 (2H, dapp, J 8.6Hz) and 7.93 (2H, dapp, J 8.6Hz).

### EXAMPLE 52

### 5-Cyano-N-[3,4-dimethoxyphenyl]-4-thiazol-2-ylpyrimidine-2-amine

From 2-cyano-3-dimethylamino-1-thiazol-2-ylpropen-1-one (500mg, 2.3mmol), 3,4-dimethoxyphenylguanidinium nitrate (585mg, 2.3mmol) and sodium hydroxide (100mg, 2.3mmol) to give the title compound (721 mg) as yellow solid, m.p. 258°. δH (d⁶ DMSO) 9.98 (1H, bs), 8.66 (1H, s), 7.96 (1H, d, J 3.1 Hz), 7.87 (1H, d, J 3.1 Hz), 7.20 (1 H, d, J 1.8Hz), 6.96-6.93 (1 H, m) and 6.67 (1 H, d, J 8.4Hz) and 5.79 (2H, s).

### EXAMPLE 53

### 5-Cyano-4-thiazol-2-yl-N-{4-[2-(1,2,4triazol-1-yl)ethoxy]phenyl} pyrimidine-2-amine

From 2-cyano-3-dimethylamino-1-thiazol-2-ylpropen-1-one (500mg, 2.3mmol), 4-[2-(1,2,4-triazol-1-yl)ethoxy]phenylguanidinium nitrate (714mg, 2.3mmol) and sodium hydroxide (100mg, 2.3mmol) to give the title compound (812mg) as a yellow solid, m.p. 224°. δH (d⁶ DMSO) 10.02 (1H, s), 8.70 (1 H, s), 8.33 (1 H, s), 8.01 (1 H, d, J 3.1 Hz), 7.91 (1 H, d, J 3.1 Hz), 7.77 (1 H, s), 7.47 (2H, d, J 8.8Hz), 6.79-6.76 (2H, m), 4.41 (2H, t, J 5.2Hz) and 4.23 (2H, t, J 5.2Hz).

### EXAMPLE 54

### 5-Cyano-4-thiazol-2-yl-N-{4-[2-(1,2,3-triazol-1-yl)ethoxy]phenyl} pyrimidine-2-amine

From 2-cyano-3-dimethylamino-1-thiazol-2-ylpropen-1-one (700mg, 3.2mmol), 4-[2-(1,2,3-triazol-1-yl)ethoxy]phenylguanidinium nitrate (1.0g, 3.2mmol) and sodium hydroxide (136mg, 3.2mmol) to give the title compound (0.98g) as a yellow solid, m.p. 203°. δH (d⁶ DMSO) 10.21 (1H, bs), 8.90 (1H, s), 8.21 (1 H, d, J 2.9Hz), 8.14 (1 H, s), 8.11 (1 H, d, J 2.9Hz), 7.73 (1 H, s), 7.68-7.66 (2H, m), 6.99-6.95 (2H, m), 4.81 (2H, t, J 5.1 Hz) and 4.47 (2H, t, J 5.1 Hz).

### EXAMPLE 55

### 5-Cyano-4-thiazol-2-yl-N-{4-[2-(1,2,3-triazol-2-yl)ethoxy]phenyl} pyrimidine-2-amine

From 2-cyano-3-dimethylamino-1-thiazol-2-ylpropen-1-one (700mg, 3.2mmol), 4-[2-(1,2,3-triazol-2-yl)ethoxy]phenylguanidinium nitrate (1.0g, 3.2mmol) and sodium hydroxide (136mg, 3.2mmol) to give the title compound (1.1 g) as a yellow solid, m.p. 203°. δH (d⁶ DMSO) 10.19 (1 H, s), 8.87 (1 H, s), 8.19 (1H, d, J 3.1Hz), 8.09 (1 H, d, J 3.1 Hz), 7.76 (2H, s), 7.66-7.62 (2H, m), 6.95-6.91 (2H, m), 4.80-4.78 (2H, m) and 4.52 (2H, t, J 5.5Hz).

### EXAMPLE 56

### 5-Cyano-N-[4-(2-imidazol-1-ylethoxy)phenyl]-4-thiazol-2-ylpyrimidine-2-amine

From 2-cyano-3-dimethylamino-1-thiazol-2-ylpropen-1-one (700mg, 3.2mmol), 4-(2-imidazol-1-ylethoxy)phenylguanidinium nitrate (1.0g, 3.2mmol) and sodium hydroxide (160mg, 3.8mmol) to give the title compound (981 mg) as a yellow solid, m.p. 221°. δH (d⁶ DMSO) 9.93 (1 H, s), 8.83 (1H, s), 8.16 (1H, d, J 3.12Hz), 8.03 (1 H, d, J 3.1 Hz), 7.67-7.62 (3H, m), 7.18 (1 H, d, J 1.0Hz), 6.99-6.91 (3H, m) and 4.37-4.29 (4H, m).

### EXAMPLE 57

### 5-Cyano-N-[4-fluorophenyl]-4-thiazol-2-ylpyrimidine-2-amine

From 2-cyano-3-dimethylamino-1-thiazol-2-ylpropen-1-one (700mg, 3.2 mmol), 4-fluorophenylguanidinium nitrate (700mg, 3.2mmol) and sodium hydroxide (160mg, 3.2 mmol) to give the title compound (891 mg) as a yellow solid, m.p. 263°. δH (d⁶ DMSO) 10.10 (1H, s), 8.88 (1H, s), 8.17 (1 H, d, J 3.1Hz), 8.05 (1H, d, J 3.1 Hz), 7.78-7.75 (2H, m), 7.20-7.15 (2H, m).

### EXAMPLE 58

### 5-Cyano-4-phenyl-N-{4-[2-(1,2,4-triazol-1-yl)ethoxy]phenyl}pyrimidine-2-amine

From 1-phenyl-2-cyano-3-dimethylaminopropen-1-one (470mg, 2.35mmol), 4-[2-(1,2,4-triazol-1-yl)ethoxy]phenylguanidinium nitrate (800mg, 2.6mmol) and sodium hydroxide (113mg, 2.8mmol) to give the title compound (390mg) as a yellow solid m.p. 105°. δH (d⁶ DMSO) 10.36 (1H, s), 8.89 (1H, s), 7.97 (1 H, s), 7.92 (2H, d, J 7.9Hz), 7.61-7.58 (5H, m), 6.90 (2H, d, J 9.1Hz), 4.56 (2H, t, J 5.0Hz) and 4.32 (2H, t, J 5.0Hz).

### EXAMPLE 59

### 5-Cyano-4-phenyl-N-{4-[2-(1,2,3-triazol-1-yl)ethoxy]phenyl}pyrimidine-2-amine

From 1-phenyl-2-cyano-3-dimethylaminopropen-1-one (412mg, 2.05mmol), 4-[2-(1,2,3-triazol-1-yl)ethoxy]phenylguanidinium nitrate (700mg, 2.06mmol) and sodium hydroxide (100mg, 2.5mmol) to give the title compound (300mg) as a yellow solid m.p. 178°. δH 10.37 (1H, s), 8.90 (1H, s), 7.92 (2H, d, J 7.9Hz), 7.73 (1 H, s), 7.61-7.58 (5H, m), 6.92 (2H, d, J 9.1 Hz), 4.77 (2H, t, J 5.1 Hz) and 4.38 (2H, t, J 5.1 Hz).

### EXAMPLE 60

### 5-Cyano-4-phenyl-N-{4-[2-(1,2,3-triazol-2-yl)ethoxy]phenyl}pyrimidine-2-amine

From 1-phenyl-2-cyano-3-dimethylaminopropen-1-one (470mg, 2.55mmol), 4-[2-(1,2,3-triazol-1-yl)ethoxy]phenylguanidinium nitrate (800mg, 2.56mmol) and sodium hydroxide (113mg, 2.8 mmol) to give the title compound (430mg) as a yellow solid m.p. 156°. δH (d⁶ DMSO) 10.30 (1H, s), 8.89 (1 H, s), 7.92 (2H, d, J 7.9Hz), 7.79 (2H, s), 7.61-7.58 (5H, m), 6.89 (2H, d, J 9.1 Hz), 4.78 (2H, t, J 5.1Hz) and 4.46 (2H, t, J 5.1Hz).

### EXAMPLE 61

### 5-Cyano-N-[4-hydroxyphenyl]-4-phenylpyrimidine-2-amine

From 1-phenyl-2-cyano-3-dimethylaminopropen-1-one (1.51g, 7.55 mmol), 4-hydroxyphenylguanidinium nitrate (1.78g, 8.3mmol) and sodium hydroxide (362mg, 9.06mg) to give the title compound (1.23g) as a yellow solid, m.p. 201°. δH (d⁶ DMSO) 10.23 (1 H, s), 9.26 (1H, s), 8.85 (1 H, d, J 1.0Hz), 7.92 (2H, dd, J 7.0, 1.0Hz), 7.59 (3H, m), 7.48 (2H, d, J 8.0Hz) and 6.73 (2H, d, J 8.0Hz).

### EXAMPLE 62

### 5-Cyano-N-[4-(2-hydroxyethoxy)phenyl]-4-pyridin-3-ylpyrimidine-2-amine

From 2-cyano-3-dimethylamino-1-pyridin-3-ylpropen-1-one (1.20g, 5.95mmol), 4-(2-hydroxyethoxy)phenylguanidinium nitrate (1.54g, 5.95mmol) and sodium hydroxide (262mg, 6.34mmol) to give the title compound (1.28g) as a yellow solid, m.p. 209-210°. δH (d⁶ DMSO) 10.43 (1 H, bs), 9.07 (1 H, d, J 1.9Hz), 8.93 (1H, s), 8.77 (1H, dd, J 4.8, 1.6Hz), 8.31-8.28 (1H, m), 7.65-7.60 (3H, m), 6.92 (2H, d, J 9.0Hz), 4.82 (1 H, t, J 5.6Hz), 3.96 (2H, t, J 5.0Hz) and 3.69 (2H, q, J 5.1 Hz).

### EXAMPLE 63

### 5-Cyano-4-pyridin-3-yl-N-[4-(1,2,4-triazol-1-yl)phenyl]pyrimidine-2-amine

From 2-cyano-3-dimethylamino-1-pyridin-3-ylpropen-1-one (516mg, 2.56 mmol), 4-(1,2,4-triazol-1-yl)phenylguanidinium nitrate (679mg, 2.56mmol) and sodium hydroxide (113mg, 2.82mmol) to give the title compound (425mg) as a yellow solid, m.p. 250-251°. δH 10.79 (1 H, bs), 9.20 (1 H, s), 9.12 (1 H, s), 9.04 (1 H, s), 8.81 (1 H, bm), 8.35-8.32 (1H, bm), 8.20 (1 H, s), 7.93 (2H, d, J 8.6Hz), 7.82 (2H, d, J 8.6Hz) and 7.65 (1 H, bs).

### EXAMPLE 64

### 5-Cyano-N-[4-morpholinophenyl]-4-pyridin-3-ylpyrimidine-2-amine

From 2-cyano-3-dimethylamino-1-pyridin-3-ylpropen-1-one (516mg, 2.56 mmol), 4-morpholinophenylguanidinium nitrate (725mg, 2.56mmol) and sodium hydroxide (113mg, 2.82mmol) to give the title compound (707mg) as an orange solid, m.p. 199-200°. δH (d⁶ DMSO) 10.39 (1H, bs), 9.07 (1H, d, J 1.8Hz), 8.91 (1H, s), 8.78 (1H, dd, J 4.9,1.6Hz), 8.31-8.28 (1H, m), 7.65-7.56 (3H, m), 6.93 (2H, d, J 9.1Hz), 3.74-3.70 (4H, m) and 3.07-3.04 (4H, m).

### EXAMPLE 65

### 5-Cyano-N-[4-fluorophenyl]-4-indol-3-ylpyrimidine-2-amine

From 1-indol-3-yl-2-cyano-3-dimethylaminopropen-1-one (950mg, 3.97 mmol), 4-fluorophenylguanidinium nitrate (858mg, 3.97mmol) and sodium hydroxide (176mg) to give the title compound (200mg) as an off-white solid, m.p. 256-257°. δH (d⁶ DMSO) 11.83 (1H, bs), 9.91 (1H, s), 8.73 (1H, s), 8.51 (1H, s), 8.50-8.47 (1H, m), 7.78-7.73 (2H, m), 7.53 (1H, dt, J 7.2, 0.8Hz) and 7.27-7.13 (4H, m).

### EXAMPLE 66

### 5-Cyano-N-[4-(2-imidazol-1-ylethoxy)phenyl]-4-indol-3-ylpyrimidine-2-amine

From 1-indol-3-yl-2-cyano-3-dimethylaminopropen-1-one (300mg, 1.25mmol), 4-(2-imidazol-1-ylethoxy)phenylguanidinium nitrate (465mg, 1.25mmol) and sodium hydroxide (100mg, 2.5mmol) to give the title compound (150mg) as a yellow solid, m.p. 238-239°. δH (d⁶ DMSO) 12.02 (1H, bs), 10.04 (1 H, s), 8.72 (1 H, s), 8.55 (1 H, s), 8.33 (1 H, bm), 7.68 (1 H, s), 7.63-7.55 (3H, m), 7.28-7.23 (2H, m), 7.22-7.05 (2H, bm), 6.98-6.91 (3H, m), 4.52 (2H, t, J 5.0Hz) and 4.26 (2H, t, J 5.0Hz).

### EXAMPLE 67

### 5-Cyano-4-pyridin-3-yl-N-{4-[2-(1,2,4-triazol-1-yl)ethoxy]phenyl} pyrimidine-2-amine

From 2-cyano-3-dimethylamino-1-pyridin-3-ylpropen-1-one (800mg, 3.97 mmol), 4-[2-(1,2,4-triazol-1-yl)ethoxy]phenylguanidinium nitrate (1.23g, 3.97mmol) and sodium hydroxide (176mg, 4.4mmol) to give the title compound (1.0g) as a yellow solid, m.p. 180-181°. δH (d⁶ DMSO) 10.45 (1 H, bs), 9.07 (1 H, d, J 1.8Hz), 8.93 (1 H, s), 8.77 (1 H, dd, J 4.8, 1.5Hz), 8.55 (1 H, s), 8.29 (1 H, d, J 8.1Hz), 7.97 (1 H, s), 7.64-7.60 (3H, m), 6.91 (2H, d, J 9.0Hz), 4.56 (2H, t, J 5.0Hz) and 4.32 (2H, t, J 5.0Hz).

### EXAMPLE 68

### 5-Cyano-4-pyridin-3-yl-N-{4-[2-(1,2,3-triazol-1-yl)ethoxy]phenyl} pyrimidine-2-amine

From 2-cyano-3-dimethylamino-1-pyridin-3-ylpropen-1-one (516mg, 2.56 mmol), 4-[2-(1,2,3-triazol-1-yl)ethoxy]phenylguanidinium nitrate (800mg, 2.56 mmol) and sodium hydroxide (113mg, 2.82 mmol) to give the title compound (531 mg) as a yellow solid, m.p. 177-178°. δH (d⁶ DMSO) 10.45 (1H, bs), 9.08 (1H, s), 8.93 (1H, s), 8.78 (1H, dd, J 4.8,1.5Hz), 8.31-8.28 (1H, m), 8.17 (1H, s), 7.73 (1H, s), 7.65-7.60 (3H, m), 6.92 (2H, d, J 9.0Hz), 4.77 (2H, t, J 5.0Hz).

### EXAMPLE 69

### 5-Cyano-4-pyridin-3-yl-N-{4-[2-(1,2,3-triazol-1-2-yl)ethoxy]phenyl} pyrimidine-2-amine

From 2-cyano-3-dimethylamino-1-pyridin-3-ylprope-1-one (800mg, 3.97 mmol), 4-[2-(1,2,3-triazol-2-yl)ethoxy]phenylguanidinium nitrate (1.23g, 3.97mmol) and sodium hydroxide (176mg, 4.4mmol) to give the title compound (970mg) as a yellow solid, m.p. 179-180°. δH (d⁶ DMSO) 10.45 (1 H, bs), 9.08 (1 H, d, J 1.6Hz), 8.93 (1H, s), 8.78 (1 H, dd, J 5.0, 1.6Hz), 8.32-8.27 (1H, m), 7.79 (2H, s), 7.65-7.59 (3H, m), 6.90 (2H, d, J 9.1 Hz), 4.78 (2H, t, J 5.2Hz) and 4.46 (2H, t, J 5.1Hz).

### EXAMPLE 70

### 5-Cyano-N-{4-[2-(1,2,3-triazol-1-yl)ethoxy]phenyl}-4-thien-3-ylpyrimidine-2-amine

From 2-cyano-3-dimethylamino-1-thien-3-ylpropen-1-one (557mg, 2.7 mmol), 4-[2-(1,2,3-triazol-1-yl)ethoxy]phenylguanidinium nitrate (920mg, 3.0mmol) and sodium hydroxide (130mg, 3.2mmol) to give the title compound (710mg) as a yellow solid, m.p. 177°. δH (d⁶ DMSO) 10.28 (1 H, s), 8.84 (1H,s), 8.48 (1H, s), 8.18 (1H, d, J 0.8Hz), 7.78-7.73 (3H, m), 7.61 (2H, d, J 7.6Hz), 6.91 (2H, d, J 8.9Hz) and 4.77 (2H, t, J 5.0Hz).

2-Cyano-3-dimethylamino-1-thien-3-ylpropen-1-one was prepared from 3-cyanoacetyl thiophene (7.64g,50.56mmol) and dimethylformamide dimethylacetal (20mL, 152mmol) as a yellow solid (6.6g), m.p. 134°. δH (CDCl₃) 8.27 (1H, dd, J 3.0, 1.3Hz), 8.00 (1H, s), 7.61 (1H, dd, J 5.0Hz), 7.27 (1 H, dd, J 5.0,3.0Hz), 3.48 (3H, s) and 3.29 (3H, s).

3-Cyanoacetylthiophene was prepared in a similar manner to the analogous starting material of Example 8 from 3-isoxazol-5-ylthiophene (8.15g, 53.9mmol) and a freshly prepared solution of sodium ethoxide [1.24g, 53.9mmol sodium in ethanol (100mL)] to give the desired material (7.76g) as an orange solid, δH (CDCl₃) 8.16 (1 H, dd, J 5.0, 1.3Hz), 7.55 (1 H, dd, J5.0,2.3Hz), 7.40 (1 H, dd, J5.0, 2.3Hz) and 3.96 (2H, m).

3-Isoxazol-5-ylthiophene was prepared in a similar manner to the analogous starting material of Example 8 from 3-dimethylamino-1-thien-3-ylpropen-1-one (10g, 55.2mmol) and hydroxylamine hydrochloride (4.2g, 60.7mmol) to give the desired product (8.15g) as a colourless oil, δH (CDCl₃) 8.18 (1 H, d, J 1.9Hz), 7.72 (1 H, t, J 2.1 Hz), 7.34 (2H, d, J 2.1 Hz) and 6.29 (1H, d, J 1.8Hz).

3-dimethylamino-1-thien-3-ylpropen-1-one was prepared from 3-acetylthiophene (15.0g) and dimethylformamide dimethylacetal (47.5mL, 357mmol) as a yellow solid (14g), m.p. 98°. δH (CDCl₃) 7.90 (1 H, dd, J 3.0, 1.0Hz), 7.76 (1 H, d, J 12.4Hz), 7.53 (1 H, dd, J 5.9,1.0Hz), 7.28-7.25 (1 H, m), 5.57 (1 H,d, J 12.4Hz) and 3.03 (6H, bs).

### EXAMPLE 71

### 5-Cyano-N-[4-(2-(1,2,4-triazol-1-ylethyl)phenyl]-4-thien-3-ylpyrimidine - 2-amine

From 2-cyano-3-dimethylamino-1-thien-3-ylpropen-1-one (800mg, 3.9 mmol), 4-[2-(1,2,4-triazol-1-yl)ethyl]phenylguanidinium nitrate (1.26g, 4.3mmol) and sodium hydroxide (186mg, 4.7mmol) to give the title compound (721 mg) as a light orange solid, m.p. 209°. δH (d⁶ DMSO) 10.37 (1H, s), 8.88 (1 H, s), 8.49 (1 H, s), 8.31 (1 H, s), 7.94 (1H, s), 7.80-7.76 (2H, m), 7.63 (2H, d, J 8.5Hz), 7.10 (2H, d, J 8.5Hz), 4.41 (2H, t, J 7.0Hz) and 3.07 (2H, t, J 7.0Hz).

4-[2-(1,2,4-Triazol-1-yl)ethyl]phenylguanidinium nitrate was prepared from 4-[2-(1,2,4-triazol-1-yl)ethyl]aniline (5.75g, 30.4mmol), cyanamide (2.17g, 51.7mmol in water [2mL]) and concentrated HNO₃ (2.2mL, 33.3mmol) as a pink solid, m.p. 183°. δH (d⁶ DMSO) 9.40 (1 H, bs), 8.33 (1 H, s), 7.94 (1 H, s), 7.27-7.19 (4H,m), 7.14-7.11 (2H, m), 4.42 (2H, t ,J 7.2Hz) and 3.11 (2H, t, J 7.2Hz).

4-[2-(1,2,4-triazol-1-yl)ethyl]aniline was prepared from 4-[2-(1,2,4-triazol-1-yl)ethyl]-nitro benzene (7.0g, 32.1mmol) in a manner similar to the analogous intermediate of Example 12 as an off white solid (5.8g), m.p. 79°. δH 7.93 (1H, s), 7.73 (1 H, s), 6.79 (2H, d, J 8.5Hz), 6.58 (2H, d, J 8.5Hz), 4.31 (2H, t, J 7.0Hz), 3.30 (2H,bs) and 3.03 (2H ,t, J7.0Hz).

4-[2-(1,2,4-triazol-1-yl)ethyl]nitrobenzene was prepared from 4-[2-p-toluenesulphonyloxy ethyl]nitrobenzene (2.77g, 8.6mmol) and 1,2,4-triazole sodium salt (942mg, 10.3mmol) as a pink solid (2.0g), m.p. 97°. δH (CDCl₃) 8.12 (2H, d, J 9.0Hz), 8.00 (1H, s), 7.80 (1 H, s), 7.20 (2H, m), 4.44 (2H, t, J 7.0Hz) and 3.32 (2H, t, J 7.0Hz).

4-[2-p-toluenesulphonyloxyethyl]nitrobenzene was prepared from 4-nitrophenethyl alcohol (20.0g,120mmol) and 4-toluenesulphonyl chloride (34.2g, 180mmol) as a yellow solid (3.0g), m.p. 133°. δH (CDCl₃) 8.10 (2H, d, J 9.0Hz), 7.66 (2H, d, J 9.0Hz), 7.26 (4H, m), 4.29 (2H, t, J 6.0Hz), 3.07 (2H, t, J 6.0Hz) and 2.43 (3H, s).

### EXAMPLE 72

### 4-(2-Aminopyridin-5-yl)-5-cyano-N-(4-fluorophenyl)pyrimidine-2-amine

To a suspension of 5-cyano-N-(4-fluorophenyl)-4-[2-(4-methoxybenzylamino)pyridin-5-yl]pyrimidine-2-amine (104mg, 0.24mmol) in acetonitrile (4mL), MeOH (2mL) and dichloromethane (2mL), was added dropwise a solution of ceric ammonium nitrate (133mg, 0.24mmol) in water (1mL). After. 0.5h the reaction was poured into saturated NaHCO₃ and extracted with CHCl₂. The organic phase was dried (MgSO₄) and concentrated under reduced pressure to give the title compound (42mg) as a buff solid, m.p. 289°. δH (d⁶ DMSO) 9.93 (1 H, s), 8.76 (1 H, d, J 2.3Hz), 8.74 (1 H, s), 8.09 (1 H, dd, J 8.8, 2.5Hz), 7.77-7.73 (2H, bm), 7.18-7.13 (2H, m), 6.85 (1 H, s) and 6.63 (1H, d, J 8.8Hz).

5-cyano-N-(4-fluorophenyl)-4-[2-(4-methoxybenzylamino)pyridin-5-yl]-pyrimidine-2-amine was prepared in a similar manner to the title compound of Example 7 from 4-(2-chloropyridin-5-yl)-5-cyano-N-(4-fluorophenyl)-pyrimidine-2-amine (764mg, 2.3mmol) and p-methoxybenzylamine (644mg, 4.6mmol) as a yellow solid (432mg). δH (d⁶ DMSO) 10.35 (1 H, s), 8.82 (1 H, s), 8.77 (1 H, d, J 2.3Hz), 8.04 (1 H, dd, J 8.9, 2.3Hz), 7.84 (1 H, t, J 5.6Hz), 7.77-7.74 (1 H, m), 7.28 (2H, d, J 8.6Hz), 7.21 (2H, t, J 8.9Hz), 6.90 (2H, d, J 8.6Hz), 6.66 (1 H, d, J 8.9Hz), 4.51 (2H, s) and 3.73 (3H, s).

4-[2-Chloropyridin-5-yl]-5-cyano-N-(4-fluorophenyl)pyrimidine-2-amine was prepared from 1-(2-chloropyridin-5-yl)-2-cyano-3-dimethylamino propen-1-one (8.0g, 36mmol), 4-fluorophenylguanidinium nitrate (8.23g, 37.8mmol) and sodium hydroxide (1.49g, 37.8mmol) as a yellow solid (7.23g). δH (d⁶ DMSO) 10.66 (1H, bs), 9.00 (1 H, s), 8.95 (1H, d, J 2.3Hz), 8.38 (1 H, dd, J 8.4,2.5Hz), 7.80 (1 H, d, J 8.4Hz), 7.77-7.74 (2H, m) and 7.24-7.20 (2H, m).

### EXAMPLE 73

### 5-Cyano-4-{2-([2-(diethylamino)ethyl]amino)pyridin-5-yl}-N-(4-fluorophenyl) pyrimidine-2-amine

From 4-[2-Chloropyridin-5-yl]-5-cyano-N-(4-fluorophenyl)pyrimidine-2-amine (600mg, 1.8mmol) and N,N-diethylethylenediamine (430mg) in a manner analogous to the compound of Example 7, to give the title compound (197mg) as a yellow solid, m.p. 166°. δH (d⁶ DMSO) 9.79 (1 H, bs), 8.83 (1 H, d, J 2.3Hz), 8.72 (1 H, s), 8.08 (1 H, dd, J 8.9, 2.3Hz), 7.76-7.72 (2H, m), 7.17-7.11 (2H, m), 6.73 (1H, bs), 6.65 (1H, d, J 8.9Hz), 3.53-3.48 (2H, m), 2.85 (2H, bs), 2.73-2.69 (2H, m), 2.61-2.51 (4H, m) and 1.78-1.70 (4H, m).

### EXAMPLE 74

### 4-[4-(1-Acetamido-1-methylethyl)phenyl]-5-cyano-N-4-fluorophenyl pyrimidine-2-amine

To a suspension of the compound of Example 20 (100mg 0.29mmol) in CHCl₃ (8mL) was added pyridine (0.1 mL) and acetic anhydride (0.1 mL). The reaction was stirred at ambient temperature for 6h. The reaction was then washed with 2M HCl and saturated NaHCO₃, dried (MgSO₄), and concentrated under reduced pressure to give the title compound (103mg) as a yellow solid, m.p. 212-216°. δH 8.69 (1H, s), 8.06 (2H, d, J 8.4Hz), 7.63-7.60 (2H, m), 7.56 (2H, d, J 8.4Hz), 7.12-7.08 (2H, m), 5.82 (1H, s), 2.03 (3H, s) and 1.74 (6H, s).

### EXAMPLE 75

### 5-Cyano-4-[4-(1-dimethylamino-1-methylethyl)phenyl]-N-(4-fluoro phenyl)pyrimidine-2-amine

The compound of Example 20 (500mg, 1.44mmol) was heated at reflux in formic acid (10mL) and 37% aqueous formaldehyde (10mL) for 4 days. The reaction was diluted in 100mL dichloromethane and concentrated under reduced pressure. The resulting residue was redissolved in dichloromethane (100mL) and washed with 2M NaOH, dried (MgSO₄) and again concentrated under reduced pressure. The residue was subjected to column chromatography (silica 10-15% MeOH-dichloromethane) to give the title compound (411 mg) as a yellow solid, m.p. 179°. δH (CDCl₃) 8.61 (1H, s), 7.94 (2H, d, J 8.1 Hz), 7.63 (2H, d, J 8.3Hz), 7.51-7.48 (2H, s), 7.04-7.01 (2H, m), 2.12 (6H, s) and 1.32 (6H, s).

### EXAMPLE 76

### 5-Cyano-N-[4-(1,2,4-triazol-1-yl)phenyl]-4-[4-(1-dimethylamino-1-methyl ethyl)phenyl] Pyrimidine-2-amine

In a manner analogous to the compound of Example 75, from the compound of Example 11 (750mg, 1,76mmol) to give the title compound (687mg) as a yellow solid, m.p.235-237°. δH (d⁶ DMSO) 10.68 (1 H, bs), 9.23 (1 H, s), 8.99 (1 H, J 1.9Hz), 8.21 (1 H, s), 7.99-7.96 (4H, m), 7.85 (2H, dd, J 7.1, 1.9Hz), 7.72 (2H, d, J 8.5Hz), 2.13 (6H, s) and 1.35 (6H, s).

### EXAMPLE 77

### 5-Cyano-4-[4-(1-methyl-1-pyrrolidin-1-ylethyl)phenyl]-N-(4-fluoro phenyl)pyrimidine-2-amine

To a solution of the compound (1.0g,2.88mmol) of Example 20 in DMF (20mL) was added potassium carbonate (786mg, 5.7mmol) and 1,4-dibromobutane (622mg, 2.88mmol) and the resulting mixture stirred at 60° for 12h. The reaction was partitioned between brine and ethyl acetate then the organic phase was dried (MgSO₄) and concentrated under reduced pressure. The residue was subjected to column chromatography to give the title compound (591 mg) as a yellow solid, m.p. 124°. δH (d⁶ DMSO) 8.58 (1 H, s), 7.94 (2H, dapp, J 8.4Hz), 7.63 (4H, m), 6.96 (2H, tapp, J 8.7Hz), 3.23 (4H, s), 2.61 (4H, s) and 1.47 (6H, s).

### EXAMPLE 78

### 5-Cyano-4-{[4-(imidazol-1-yl)methyl]phenyl}-N-(3,4,5-trimethoxyphenyl) pyrimidine-2-amine

From 2-cyano-3-dimethylamino-1-[(4-imidazol-1-ylmethyl)phenyl]propen-1-one (260mg,0.89mmol), 3,4,5-trimethoxyphenylguanidinium nitrate (308mg, 1.07mmol) and sodium hydroxide (46mg, 1.16mmol) in a similar manner to the compound of Example 1 to give the title compound (80mg) as a yellow solid, m.p. 253°. δH (d6 DMSO) 10.07 (1H, bs), 8.86 (1H, s), 7.99 (2H, dt, J 8.4, 1.9Hz), 7.72 (1H, s), 7.43 (2H, dt, J 8.4,1.9Hz), 7.21 (2H, s), 7.17 (1 H, t, J 1.2Hz), 6.94 (1 H, t, J 2.1 Hz), 5.31 (2H, s), 3.77 (6H, s) and 3.67 (3H, s).

2-Cyano-3-dimethylamino-1-[(4-imidazo)-1-ylmethyl)phenyl]propen-1-one was prepared by the addition of 3-hydroxy-3-(4-(imidazol-1-ylmethyl)phenyl)acrylonitrile, lithium salt (2.16g, 10.0mmol) to a solution of MeOH-acetyl chloride (15mL-1 mL) followed by dimethylformamide dimethylacetal (1.3mL). The reaction was stirred at room temperature for 1 h, then concentrated under reduced pressure. The resulting residue was partitioned between ethyl acetate and saturated Na₂CO₃. The organic phase was dried (MgSO₄) and concentrated under reduced pressure. After chromatography (silica, 5-8% MeOH-CH₂Cl₂) of the residue the desired material was obtained as a yellow oil (260mg). δH (300MHz) 7.94 (1H, s), 7.75 (2H, dt, J 8.3,1.8 Hz), 7.54 (1 H, s), 7.16 (2H, d, J 8.5Hz), 7.08 (1H, t, J 1.0Hz), 6.89 (1H, t, J 1.3Hz), 5.14 (2H, s), 3.46 (3H, s) and 3.28 (3H, s).

3-Hydroxy-3-(4-(imidazol-1-ylmethyl)phenyl)acrylonitrile, lithium salt was prepared as follows:- Acetonitrile (1.04mL, 20.0mmol) was added to a solution of lithium bistrimethylamide (20mL 1.0M in THF, 20.0mmol) at - 78° under a nitrogen atmosphere, and the resulting mixture stirred for 20 min. A solution of methyl 4-(imidazol-1-ylmethyl)benzoate (2.16g, 10mmol) in THF was added dropwise and the reaction temperature then allowed to warm to room temperature over a 3h period. The reaction was diluted with diethyl ether (30mL) and the resulting solid collected by filtration washing further with ether (3 x 30mL). The solid was dried under vacuum to give the desired material as a yellow solid (2.65g) and was used without purification. δH (d⁶ DMSO) 7.72 (1 H, s), 7.59 (2H, d, J 8.1Hz), 7.15 (1H, s), 7.10 (2H, d, J 8.2Hz), 6.88 (1H, s), 5.13 (2H, s) and 3.93 (1 H, s).

### EXAMPLE 79

### 5-Cyano-N-[3-fluorophenyl]-4-[4-(imidazol-1-yl)phenyl]pyrimidine-2-amine

From 2-cyano-3-dimethylamino-1-(4-imidazol-1-ylphenyl)propen-1-one (480mg, 1.8mmol), 3-fluorophenylguanidinium nitrate (480mg, 1.98mmol) and sodium hydroxide (87mg) in a similar manner to the compound of Example 1 to give the title compound (412mg) as a yellow solid, m.p. 300°. δH (d⁶ DMSO) 10.73 (1H, s), 9.03 (1H, s), 8.44 (1H, s), 8.12 (2H, d, J 9.0Hz), 7.96-7.90 (3H, m), 7.80 (1H, d, 1Hz), 7.55 (1H, d, J 8.0hz), 7.35 (1 H, q, J 9.0Hz), 7.16 (1 H, d, J 1.0Hz) and 6.90 (1H, m).

2-Cyano-3-dimethylamino-1-(4-imidazol-1-ylphenyl)propen-1-one was prepared in a manner similar to the analogous starting material of Example 79, as a yellow solid (970mg), m.p. 165°. δH (CDCl₃) 8.01 (1H, s), 7.96-7.92 (3H, m), 7.48-7.45 (2H, m), 7.33 (1 H, t, J 1.4hz), 7.23 (1 H, d, J 1.0Hz), 3.52 (3H, s) and 3.34 (3H, s).

3-Hydroxy-3-(4-imidazol-1-ylphenyl)acrylonitrile, sodium salt was prepared from 5-(4-imidazol-1-ylphenyl)isoxazole (1.22g, 5.78mmol) and sodium (133mg, 5.78mmol) in ethanol (15mL) in a manner silimilar to the analogous starting material of Example 8, as a beige solid (1.14g), m.p. 286°.

5-(4-Imidazol-1-ylphenyl)isoxazole was prepared by treating a solution of 3-dimethylamino-1-[(4-imidazol-1-yl)phenyl]propen-1-one (1.64g, 6.8mmol) in MeOH with hydroxylamine-O-sulphonic acid (831 mg, 7.35mmol) at ambient temperature for 16h. A further quantity of hydroxylamine-O-sulphonic acid (831mg, 7.35mmol) was added and stirring continued for 3h. The reaction was then treated with saturated NaHCO₃ solution, the resulting solution being collected by filtration and washed with water and diethyl ether to give the desired material (1.08g) as a pink solid, m.p. 168°. δH (d⁶ DMSO) 8.67 (1 H, d, J 1.8Hz), 3.89 (1 H, s), 8.03-7.99 (2H, m), 7.86-7.84 (3H, m), 7.14 (1 H, s) and 7.10 (1 H, d, J 1.9Hz).

### EXAMPLE 80

### N-[3-(5-Cyano-4-thiophen-2-ylpyrimidin-2-ylamino)phenyl]-4-(4-methyl piperazin-1-ylmethyl)benzamide

A solution of N-3-aminophenyl-5-cyano-4-thien-2-ylpyrimidine-2-amine (300mg, 1.0mmol) and 4-(4-methylpiperazin-1-yl)methylbenzoic acid, lithium salt (300mg, 1.0mmol) in DMF (10mL) was treated with 1-(3-dimethylaminopropyl)-3-ethylcarbodiimide (307mg, 1.6mmol), 1-hydroxybenzotriazole (216mg, 1.6mmol) and N-methylmorpholine (350ml, 3.2µmol) and the resulting mixture was stirred at ambient temperature for 48h. The reaction was then concentrated under reduced pressure and the resulting residue partitioned between ethyl acetate and saturated brine. The organic phase was dried (MgS04), concentrated under reduced pressure and the resulting residue recrystallised from ethyl acetate-ether-MeOH (4:4:1) to give the title compound (312mg) as a colourless solid, m.p. 208-209°. δH (d⁶ DMSO) 10.48 (1H, bs), 10.23 (1H, s), 8.89 (1h,s), 8.28 (1 H, d, J 3.5Hz), 8.13 (1 H, m), 7.98 (1 H, d, J 4.8Hz), 7.90 (2H, d, J 8.2Hz), 7.60 (1 H, bs), 7.43 (3H, d, J 8.2Hz), 7.36-7.30 (2H, s), 3.52 (2H, s), 2.37 (8H, bs) and 2.15 (3 H,s).

N-3-Aminophenyl-5-cyano-4-thien-2-ylpyrimidine-2-amine was prepared by heating a solution of 5-cyano-N-3-nitrophenyl-4-thien-2-ylpyrimidine-2-amine (2.77g, 8.57mmol) and tin(II)chloride dihydrate (7.73g, 34.05mmol) in ethanol (160mL) at reflux for 18h. On cooling the reaction was concentrated under reduced pressure and partitioned between CH₂Cl₂ and 2M NaOH. The organic phase was dried (MgSO₄) and evaporated to give the desired material (320mg) as a yellow solid, m.p. 221-222°. δH (d⁶ DMSO) 10.16 (1H, s), 8.83 (1H, s), 8.24 (1H, d, J 3.9Hz), 7.97 (1H, d, J 5.1 Hz), 7.32 (1 H, dd, J 5.0, 4.0Hz), 6.97 (3H, bm), 6.31 (1 H, m) and 5.04 (2H, bs).

5-Cyano-N-3-nitrophenyl-4-thien-2-ylpyrimidine-2-amine was prepared from 2-cyano-3-dimethylamino-1-thien-2-ylpropen-1-one (3.20g, 15.51mmol), 3-nitrophenylguanidinium nitrate (3.74g, 15.53mmol) and sodium hydroxide (652mg, 7.82mmol) to give the desired material (841 mg) as an off-white solid, m.p.275-277°. δH (d⁶ DMSO) 10.87 (1H, bs), 8.98 (1H, s), 8.90 (1H, bs), 8.31-8.29 (1H, m), 8.09-8.05 (2H, m), 7.91 (1H, dd, J 7.7,2.0Hz), 7.64 (1H, t ,J 8.1 Hz) and 7.37-7.35 (1 H, m).

4-(4-Methylpiperazin-1-yl)methylbenzoic acid, lithium salt was prepared by stirring methyl 4-(4-methylpiperazin-1-yl)methylbenzoate (845mg, 3.41 mmol) and lithium hydroxide monohydrate (300mg, 7.15mmol) in THF-H2O [50%v/v] (14mL) at room temperature for 16h. The solvent was removed under reduced pressure to give the desired material (799mg) as a white solid, m.p. 230°. δH (d⁶ DMSO) 7.80 (2H, d, J 8.1 Hz), 7.16 (2H,d, J 8.1 Hz), 3.41 (2H, s), 2.30 (8H, bs) and 2.12 (3H, s).

Methyl 4-(4-methylpiperazin-1-yl)methylbenzoate was prepared by heating methyl (4-bromomethyl)benzoate (2.3g, 10.0mmol) and 1-methylpiperazine (1.0g, 10.0mmol) in DMF (10mL) for 3h. The reaction was concentrated under reduced pressure and the residue dried under high vacuum to give the desired product (1.2g) as a white solid, m.p.152°. δH (d⁶ DMSO) 7.90 (2H, d, J 8.2Hz), 7.43 (2H, d, J 8.2Hz), 3.83 (3H, s), 2.67 (4H, bs), 2.47 (4H, bs) and 2.40 (3H, s).

### EXAMPLES 81 to 104

The compounds of Examples 81 to 104 were prepared by solution phase parallel synthesis performed on a Quest 210 Personal Synthesizer (Argonaut Technologies, San Carlos, CA,USA) employing the following intermediate:

### 2-Chloro-5-cyano-4-phenylpyrimidine

5-cyano-4-phenyl-1(H)-pyrimidin-2-one (0.83g, 4.21 mmol) was heated in phosphorous oxychloride (20mL) and DMF (~1ml) at 115° for 24h. On cooling, the reaction was concentrated under reduced pressure and the residue poured into a cooled saturated NaHCO₃ solution. This was extracted with ethyl acetate, the combined organic phases dried (MgSO₄) and evaporated to give the desired material (0.79g) as a yellow solid m.p. 79-81°.

The pyrimidin-2-one was prepared by treating a solution of 2-amino-5-cyano-4-phenylpyrimidine (2.0g, 10.2mmol) in 50% concentrated H₂SO₄-water (v/v) at room temperature, with a solution of sodium nitrite (2.82g, 40.8mmol) in water (30ml) over 1 h. The reaction was allowed to stir overnight at room temperature, before additional sodium nitrite (2.82g, 10.2mmol) was added and stirring continued for 3h. After this time ammonium hydroxide (33% aqueous) was added to pH9, and the resulting precipitate collected and dried to give the desired material (2.2g) as a white solid m.p. 220-2220.

2-Amino-5-cyano-4-phenylpyrimidine was prepared from guanidine carbonate (1.57g, 17.5mmol), 1-phenyl-2-cyano-3-dimethylaminopropen-1-one (3.5g, 17.5mmol) and sodium hydroxide (720mg, 19.3 mmol) in a similar method to the compound of Example 1, as an off-white solid, m.p. 147^{o}.

### EXAMPLE 81

### 5-Cyano-4-phenyl-N-quinol-6-ylpyrimidine-2-amine

To a Quest 5ml Teflon reaction vessel (RV), was added 6-aminoquinoline (61µmol) and a solution of 2-chloro-5-cyano-4-phenylpyrimidine (10mg, 47mmol) in 1,4-dioxan (1.5mL). The resulting mixture was heated (85°) with agitation every 10 min for 48h. After this time PS-isocyanate resin (Argonaut Technologies) (100mg, 150µmol) and PS-trisamine (Argonaut Technologies) (50mg, 150mmol) were added to the reaction vessel and the reaction was agitated every 10min at 55o for 18h. The reaction was diluted with THF (~3ml) and then filtered into a pre-weighed vial, the resins being further washed with dichloromethane. The combined filtrates were evaporated under reduced pressure overnight and the residue taken up in DMSO (500µl). The residue was purified using semi-preparitive HPLC (System; Waters HPLC Pump Module 600E, Waters 486 detector with semi-prep flow cell and Waters 717 Autosampler (250µl). Column; 150 x 10mm Luna C18 (2) 5µm. Conditions; 90% [0.1% TFA-water] 10% [0.1% TFA-acetonitrile] to 10% [0.1%TFA-water] 90% [0.1% TFA-acetonitrile] at 5.0mlmin⁻¹ with a run time of 15 min at ambient temperature) to give the title compound. HPLC-MS Retention time 4.2mins (MH)+ 324.

### HPLC-MS Conditions

HPLC-MS was performed on a Hewlett Packard Binary Pump 1100/MSD ES Single Quadropole using a Luna C18(2), 50x4.6mm column, running a gradient of 95% [20mM ammonium formate pH3.5] 5% [acetonitrile-0.1%TFA] to 5% [20mM ammonium formate pH3.5] 95% [acetonitrile-0.1%TFA] at 0.8mlmin⁻¹ with a run time of 5min. MS acquired at 70V in positive ion API-electrospray mode of ionisation, scannining from 150-750 amu.

### EXAMPLE 82

### N-(3-Chloro-4-methylphenyl)-5-cyano-4-phenylpyrimidine-2-amine

3-Chloro-4-methylaniline gave the title compound
HPLC-MS Retention time 5.13mins (MH)+ 322

### EXAMPLE 83

### N-(3-Acetylphenyl)-5-cyano-4-phenylpyrimidine-2-amine

3-Aminoacetophenone gave the title compound
HPLC-MS Retention time 4.42mins (MH)+ 315

### EXAMPLE 84

### N-(4-Chloro-3-trifluoromethylphenyl)-5-cyano-4-phenylpyrimidine-2-amine

4-Chloro-3-trifluoromethylaniline gave the title compound
HPLC-MS Retention time 5.19mins (MH)+ 376

### EXAMPLE 85

### 5-Cyano-N-(4-methoxycarbonylphenyl)-4-phenylpyrimidine-2-amine

Methyl 4-aminobenzoate gave the title compound
HPLC-MS Retention time 4.58 mins (MH)+ 331

### EXAMPLE 86

### N-(4-Carboxymethylphenyl)-5-cyano-4-phenylpyrimidine-2-amine

4-Aminophenylacetic acid gave the title compound
HPLC-MS Retention time 4.0 mins (MH)+ 331

### EXAMPLE 87

### 5-Cyano-N-[4-(2-N,N-diethylaminoethylaminocarboxy)phenyl]-4-phenylpyrimidine-2-amine

Procainamide hydrochloride gave the title compound
HPLC-MS Retention time 3.33 mins (MH)+ 415.5

### EXAMPLE 88

### N-(3-Carboxyphenyl)-5-cyano-4-phenylpyrimidine-2-amine

3-Aminobenzoic acid gave the title compound
HPLC-MS Retention time 4.03 mins (MH)+ 317

### EXAMPLE 89

### 5-Cyano-4-phenyl-N-[3-(1,1,2,2-tetrafluoroethoxyphenyl)]pyrimidine-2-amine

1,1,2,2-Tetrafluoroethoxyaniline gave the title compound
HPLC-MS Retention time 4.77 mins (MH)+ 389

### EXAMPLE 90

### 5-Cyano-N-(3-oxazol-5-ylphenyl)-4-phenylpyrimidine-2-amine

3-Oxazol-5-ylaniline gave the title compound
HPLC-MS Retention time 4.4 mins (MH)+ 340

### EXAMPLE 91

### 5-Cyano-N-[2-(4-fluorophenoxy)pyridin-5-yl)-4-phenylpyrimidine-2-amine

5-Amino-2-(4-fluorophenoxy)pyridine gave the title compound
HPLC-MS Retention time 4.72 mins (MH)+ 384

### EXAMPLE 92

### 5-Cyano-N-(4-methoxycarbonylthien-3-yl)-4-phenylpyrimidine-2-amine

Methyl 3-aminothiophene-4-carboxylate gave the title compound
HPLC-MS Retention time 5.09 min (MH)+ 337

### EXAMPLE 93

### 5-Cyano-N-(2-morpholinopyridin-5-yl)-4-phenylpyrimidine-2-amine

2-Morphilino-5-aminopyridine gave the title compound
HPLC-MS Retention time 4.1 min (MH)+ 359

### EXAMPLE 94

### 5-Cyano-N-[4-(6-methylbenzothiazol-2-yl)phenyl]]pyrimidine-2-amine

2-Amino-6-methylbenzothiazole gave the title compound
HPLC-MS Retention time 5.7min (MH)+ 420.5

### EXAMPLE 95

### 5-Cyano-N-(4-isopropyl-2-methylphenyl)-4-phenylpyrimidine-2-amine

4-isopropyl-2-methylaniline gave the title compound
HPLC-MS Retention time 5.35mins (MH)+ 329

### EXAMPLE 96

### 5-Cyano-N-(3-methanesulphonyl)phenyl-4-phenylpyrimidine-2-amine

3-(Methanesulphonyl)aniline gave the title compound
HPLC-MS Retention time 4.12mins (MH)+ 351

### EXAMPLE 97

### 5-Cyano-N-[2-(3,5-dimethylpyrazol-1-yl)pyridin-3-yl)-4-phenylpyrimidine-2-amine

3-Amino-2-(3,5-dimethylpyrazol-1-yl)pyridine gave the title compound
HPLC-MS Retention time 5.12 mins (MH)+ 368

### EXAMPLE 98

### 5-Cyano-N-(4-ethylphenyl)-4-phenylpyrimidine-2-amine

4-Ethylaniline gave title compound
HPLC-MS Retention time 4.99 mins (MH)+ 301

### EXAMPLE 99

### 5-Cyano-N-(8-methoxyquinol-6-yl)-4-phenylpyrimidine-2-amine

6-Amino-8-methoxyquinoline sgave the title compound
HPLC-MS Retention time 3.96 mins (MH)+ 354

### EXAMPLE 100

### N-(4-n-Butoxyphenyl)-5-cyano-4-phenylpyrimidine-2-amine

4-n-Butoxyaniline gave the title compound
HPLC-MS Retention time 5.19 mins (MH)+ 345.4

### EXAMPLE 101

### 5-Cyano-N-(2-oxo-2-phenylethyl)-4-phenylpyrimidine-2-amine

2-Oxo-2-phenethylamine gave the title compound
HPLC-MS Retention time 4.4 mins (MH)+ 315

### EXAMPLE 102

### 5-Cyano-N-[3-n-(4-methylpiperazin-1-yl)propyl]-4-phenylpyrimidine-2-amine

3-N-(4-Methylpiperazin-1-yl)propylamine gave the title compound
HPLC-MS Retention time 3.1 mins (MH)+ 337

### EXAMPLE 103

### N-(Adamant-1-yl)-5-cyano-4-phenylpyrimidine-2-amine

1-Adamantanamine gave the title compound
HPLC-MS Retention time 6.0 mins (MH)+ 331

### EXAMPLE 104

### 5-Cyano-N-(2-morpholinoethyl)-4-phenylpyrimidine-2-amine

2-Morpholinoethylamine gave the title compound
HPLC-MS Retention time 3.1 mins (MH)+ 310

### EXAMPLE 105

### 4-[4-(1-Amino-1-methylethyl)phenyl]-5-cyano-N-[4-(1,2,4-triazol-1-yl)phenyl]pyrimidine-2-amine citrate

The compound of Example 11 (100mg, 0.25mmol) was dissolved in acetone/methanol (25mL, 1:1 v/v) and to this citric acid (52.5mg, 0.25mmol) was added. The resulting solution was diluted with diethyl ether (20ml) to give the title compound (145mg). δH (d⁶ DMSO) 10.73 (1H, bs), 9.21 (1H, s), 9.01 (1 H, s), 8.20 (1H, s), 8.02 (2H, d, J 8.4Hz), 7.94 (2H, d, J 8.7Hz), 7.80 (2H, d, J 7.4Hz, 7.78 (2H, d, J 8.3Hz), 2.49 (4H, m) and 1.65 (6H, s)

### BIOLOGICAL ACTIVITY

The following assays were used to demonstrate the activity and selectivity of compounds according to the invention:

The activity of the comounds against KDR kinase and a FGFR Kinase [FGFR2 kinase] can be determined in the following two assays:

### KDR Kinase and FGFr2 Kinase

The activities of recombinant KDR kinase and FGFr2 kinase were determined by measuring their ability to transfer the γ-phosphate from [³³P]ATP to polyglutamic acid - tyrosine (pEY).

The assay methodology employed for both kinases is identical except that in the assay of KDR kinase the diluent used throughout was 20mM HEPES pH 7.25 containing 2mM MnCl₂, 2mM MnCl₂, 5mM DTT and 0.05% Brij 35, whereas in the FGFr2 assay 10mM MnCl₂ is used instead of 2mM MnCl₂ and 2mM MnCl₂.

The assay was conducted in a total volume of 202µl containing 1-10ng kinase, 5µg/ml pEY (4:1) (Sigma, UK), 1µM ATP (containing ~50,000cpm [³³P]ATP (Amersham International, UK) (Sigma, UK) and test inhibitors at the appropriate concentration. The test inhibitors were dissolved in DMSO and added such that the final concentration of DMSO in the assay did not exceed 2% (v/v). The assay was initiated by addition of kinase and terminated after 10 minutes incubation at room temperature by addition of 50µl of 20mM HEPES pH 7.25 containing 0.125M EDTA and 10mM ATP. A 200µl aliquot was applied to the well of a Millipore (UK) MAFC filter plate containing 100µl of 30% (w/v) trichloroacetic acid (TCA). The plate was then placed on a suitable manifold and connected to a vacuum. After complete elimination of the liquid each well was washed under vacuum using five volumes (100µl per wash) of 10% (w/v) TCA and finally two volumes (100µl per wash) of ethanol. The bottom of the filter plate was then sealed and 100µl per well of Ultima Gold (Beckham, UK) scintillant was added to each well. The readioactivity was measured using an appropiate scintillation counter such as a Wallac Trilux or Packard TopCount. The IC₅₀ value for each inhibitor was obtained from log dose inhibition curves fitted to the four-parameters logistic equation.

In this assay compounds accoding to the invention have IC₅₀ values of around 1µM and below, the most active compounds having values of 100nM and below.

The selectivity of compounds according to the invention can be determined in the following assays:

### p56^{lck} kinase assay

The tyrosine kinase activity of p56^{lck} was determined using a RR-src peptide (RRLIEDNEYTARG) and [γ-³³P]ATP as substrates. Quantitation of the ³³P-phosphorylated peptide formed by the action of p56^{lck} was achieved using an adaption of the method of Geissler *et al* (J. Biol. Chem. (1990) 265, 22255-22261).

All assays were performed in 20mM HEPES pH 7.5 containing 10mM MgCl₂, 10mM MnCl₂, 0.05% Brij, 1µM ATP (0.5µCi[γ-³³P]ATP) and 0.8mg/ml RR-src. Inhibitors in dimethylsulphoxide (DMSO) were added such that the final concentration of DMSO did not exceed 1 %, and enzyme such that the consumption of ATP was less than 10%. After incubation at 30°C for 15min, the reaction was terminated by the addition of one-third volume of stop reagent (0.25mM EDTA and 33mM ATP in dH₂O). A 15µl aliquot was removed, spotted onto a P-30 filtermat (Wallac, Milton Keynes, UK), and washed sequentially with 1% acetic acid and de-ionised water to remove ATP. The bound ³³P-RR-src was quantitated by scintillation counting of the filtermat in a Betaplate scintillation counter (Wallac, Milton Keynes, UK) after addition of Meltilex scintillant (Wallac, Milton Keynes, UK).

The dpm obtained, being directly proportional to the amount of ³³P-RR-src produced by p56^{lck}, were used to determine the IC₅₀ for each compound. The IC₅₀ was defined as the concentration of compound required to reduce the production of ³³P-RR-src by 50%.

In this test, compounds according to the invention have IC₅₀ values of 10µM and above.

### Zap-70 kinase assay

The tyrosine kinase activity of Zap-70 was determined using a capture assay based on that employed above for p56^{lck}. The RR-src peptide was replaced with polyGlu-Tyr (Sigma; Poole, UK) at a final concentration of 17 µg/ml. After addition of the stopped reaction to the filtermat, trichloroacetic acid 10% (w/v) was employed as the wash reagent instead of acetic acid and a final wash in absolute ethanol was also performed before scintillation counting. IC₅₀ values were determined as described above in the p56^{lck} assay.

In this test the compounds of the invention have IC₅₀ values of around 10µM and above.

### EGFr kinase assay

The tyrosine kinase activity of the EGF receptor (EGFr) was determined using a similar methodology to the p56^{Ick} kinase assay, except that the RR-src peptide was replaced by a peptide substrate for EGFr obtained from Amersham International plc (Little Chalfont, UK) and used at the manufacturer's recommended concentration. IC₅₀ values were determined as described previously in the p56^{lck} assay.

### Protein kinase C assay

Inhibitor activity against protein kinase C (PKC) was determined using PKC obtained from Sigma Chemical Company (Poole, UK) and a commercially available assay system (Amersham International plc, Amersham, UK). Briefly, PKC catalyses the transfer of the γ-phosphate (32p) of ATP to the threonine group on a peptide specific for PKC. Phosphorylated peptide is bound to phosphocellulose paper and subsequently quantified by scintillation counting. The inhibitor potency is expressed as either (i) the concentration required to inhibit 50% of the enzyme activity (IC₅₀) or (ii) the percentage inhibition achieved by 10µM inhibitor.

In this test the compounds of the invention have IC₅₀ values of around 10µM and above.

## Claims

1. Use of a compound of formula (1) 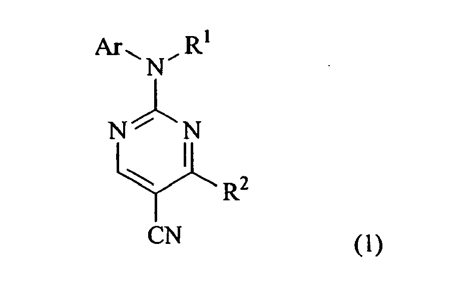 or a salt, solvate, hydrate or N-oxide thereof, for the manufacture of a medicament for the treatment of a disease state associated with angiogenesis
wherein
Ar is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R⁴ or -Alk(R⁴)ₘ;
R¹ is a hydrogen atom or a straight or branched chain C₁₋₆alkyl group;
R² is a -X¹-R³ group where X¹ is a direct bond; and
R³ is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R^{4b} or -Alk(R^{4b})ₘ;
R⁴ and R^{4b} are each a halogen atom, or an amino (-NH₂), -NHR⁵, nitro, cyano, hydroxyl (-OH), -OR⁵, formyl, carboxyl (-CO₂H), -CO₂Alk¹, thiol (-SH), -SR⁵, -COR⁵, -CSR⁵, -SO₃H, -SO₂R⁵, -SO₂NH₂, -SO₂NHR⁵ -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵ group, or a C₆₋₁₂aromatic group, a C₁₋₉heteroaromatic group containing one, two, three or four heteroatoms selected from oxygen, sulphur or nitrogen atoms, a C₃₋₁₀cycloaliphatic group, or a C₃₋₁₀heterocycloaliphatic group containing one, two, three or four heteroatoms or heteroatom-containing groups selected from -O- or -S-atoms or -N(R⁶)-, -C(O)-, -C(S)-, -S(O)- or -S(O₂)-, said aromatic or heteroaromatic groups optionally substituted with one, two or three R^{4a} atoms or groups, said cycloaliphatic or heterocycloaliphatic groups optionally substituted with one, two, three or more substituents selected from halogen atoms, hydroxyl, C₁₋₆alkoxy, thiol, C₁₋₆alkylthio, hydroxy, C₁₋₆alkyl, hydroxyethyl, -CN, -NO₂, -NHR⁵ or -N(R⁵)₂ groups, provided that two R⁴ or -Alk(R⁴)ₘ or two R^{4b} or -Alk(R^{4b})ₘ substituents may be linked together to form a C₂₋₆alkylenedioxy group;
Alk¹ is a straight or branched C₁₋₈alkyl group, a C₆₋₁₂arylC₁₋₈alkyl group, a C₆₋₁₂aryl group, a C₆₋₁₂aryloxyC₁₋₈alkyl group, a C₁₋₈alkanoyloxyC₁₋₈alkyl group, or a C₆₋₁₂aroyloxyC₁₋₈alkyl group, where said groups are optionally substituted with one or more R⁴ substituents;
R⁵ is a -Alk(R⁴)ₘ, C₆₋₁₂aromatic group, a C₁₋₉heteroaromatic group containing one, two, three or four heteroatoms selected from oxygen, sulphur or nitrogen atoms, said aromatic or heteroaromatic group optionally substituted with one, two or three R^{4a} atoms or groups;
Alk is a straight or branched C₁₋₆alkylene, C₂₋₆alkenylene or C₂₋₆alkynylene chain, optionally interrupted by one, two or three -O- or -S- atoms or groups selected from -S(O)-, -S(O)₂- or -N(R⁶)-;
R^{4a} is fluorine, chlorine, bromine or iodine atoms, or C₁₋₆alkyl, C₁₋₆alkylamino, hydroxyC₁₋₆alkyl, hydroxyC₂₋₆alkoxy, C₁₋₆alkylthiol, C₁₋₆alkoxy, C₅₋₇cycloalkoxy, haloC₁₋₆alkyl, amino (-NH₂), aminoC₁₋₆alkyl, C₁₋₆dialkylamino, aminoC₁₋₆alkoxy, C₁₋₆alkylaminoC₁₋₆alkoxy, diC₁₋₆alkylaminoC₁₋₆alkoxy, imido, 1,1,3-trioxobenzo[d]thiazolidino, nitro, cyano, hydroxyl (-OH), formyl [HC(O)-], carboxyl (-CO₂H), -CO₂Alk¹, C₁₋₆ alkanoyl, thiol (-SH), thioC₁₋₆alkyl, -SC(NH₂)NH₂, sulphonyl (-SO₃H), C₁₋₆alkylsulphonyl, aminosulphonyl (-SO₂NH₂), C₁₋₆alkylaminosulphonyl, C₁₋₆dialkylaminosulphonyl, phenylaminosulphonyl, carboxamido (-CONH₂), C₁₋₆alkylaminocarbonyl, C₁₋₆dialkylaminocarbonyl, sulphonylamino (-NHSO₂H), C₁₋₆alkylsulphonylamino, C₁₋₆dialkylsulphonylamino, phenylsulphonylamino, 2-nitrophenylsulphonylamino, aminosulphonylamino (-NHSO₂NH₂), C₁₋₆alkylaminosulphonylamino, C₁₋₆dialkylaminosulphonylamino, phenylaminosulphonylamino, aminocarbonylamino, C₁₋₆alkylaminocarbonylamino C₁₋₆dialkylaminocarbonylamino, phenylaminocarbonylamino, C₁₋₆alkanoylamino, phenylcarbonylamino, C₁₋₆alkanoylaminoC₁₋₆alkyl, C₁₋₆alkoxycarbonylamino, heteroC₃₋₆cycloalkyl, 4-(C₁₋₆alkyl)piperazinyl, heteroC₃₋₆cycloalkylC₁₋₆alkyl, 4-(C₁₋₆alkyl)piperazinylC₁₋₆alkyl, heteroC₃₋₆cycloalkylC₁₋₆alkoxy, heteroC₃₋₆alkylC₁₋₆alkylamino, heteroC₃₋₆cycloalkylamino, tetrazolyl, imidazolyl, triazolyl, imidazolylC₁₋₆alkyl, imidazolylC₁₋₆alkoxy, triazolylC₁₋₆alkoxy, imidazolylaminoC₁₋₆alkoxy, phenylamino, benzylamino, benzyloxy, or pyridylmethylamino group;
R⁶ is a hydrogen atom or a straight or branched chain C₁₋₆alkyl group;
m is zero or an integer 1, 2 or 3.

2. The use of claim 1 wherein the disease is cancer, psoriasis, rheumatoid arthritis, Kaposi's Sarcoma, ischemic heart disease, atherosclerosis or an occular disease.

3. The use of claim 1 or claim 2 wherein R³ is a phenyl, thienyl, thiazolyl, indolyl or pyridyl group optionally substituted by one, two or three -R^{4b} or -Alk(R^{4b})ₘ substituents.

4. The use of any one of claims 1 to 3 wherein Ar is a phenyl, pyridyl, indolyl, indazolyl, benzimidazolyl, benzothiazolyl, quinolyl, isoquinolyl or benzoxazolyl group each substituted by one, two or three -R⁴ or -Alk(R⁴)ₘ substituents.

5. The use of claim 4 wherein Ar is a phenyl group substituted by one, two or three -R⁴ or -Alk(R⁴)ₘ substituents.

6. The use of any one of claims 3 to 5 wherein at least one of -R⁴, -Alk(R⁴)ₘ, -R^{4b} or -Alk(R^{4b})ₘ, is a -X^{1a}(Alk^{a})ₚNR^{7a}R^{7b}) -X^{1a}(Alk^{a})ₚNHet¹ or -X^{1a}(Alk^{a})ₚAr² group, where X^{1a} is a direct bond, or a linker atom or group selected from -O-, -S-, -C(O)-, -C(S)-, -S(O)-, -S(O)₂-, -N(R⁷)-, -C(R⁷)₂-, -CON(R⁷)-, -OC(O)N(R⁷)-, -CSN(R⁷)-, -N(R⁷)CO-, -N(R⁷)C(O)O-, -N(R⁷)CS-, -SON(R⁷)-, -SO₂N(R⁷)-, -N(R⁷)SO₂-, -N(R⁷)CON(R⁷)-, -N(R⁷)CSN(R⁷)-, -N(R⁷)SON(R⁷)- or -N(R⁷)SO₂N(R⁷),
Alk^{a} is as defined for Alk,
p is zero or an integer 1,
R⁷ is a hydrogen atom or a C₁₋₆alkyl group,
R^{7a} and R^{7b} which may be the same or different is each a hydrogen atom or a straight or branched C₁₋₆alkyl group, and
-NHet¹ is an optionally substituted C₃₋₇cyclicamino group optionally containing one or more -O- or -S- atoms or -N(R⁶)-.

7. The use of claim 1 wherein the compound is:
5-Cyano-4-phenyl-N-(3,4,5-trimethoxyphenyl)pyrimidine-2-amine;
5-Cyano-N-[4-(2-imidazol-1-ylethyl)phenyl]-4-(4-methoxcarbonylphenyl)pyrimidine-2-amine;
5-Cyano-4-(4-hydroxymethylphenyl)-N-(3,4,5-trimethoxyphenyl)-pyrimidine-2-amine;
5-Cyano-4[(4-N,N-diethylaminomethyl)phenyl]-N-(3,4,5-trimethoxyphenyl)pyrimidine-2-amine;
5-Cyano-4-[2-(3(R)-dimethylaminopyrrolidin-1-yl)pyridin-5-yl]-N-(indazol-5-yl)pyrimidine-2-amine;
4-[4-(1-Amino-1-methylethyl)phenyl]-5-cyano-N-(indazol-5-yl)-pyrimidine-2-amine;
4-[4-(1-Amino-1-methylethyl)phenyl]-5-cyano-N-(3,4,5-trimethoxyphenyl)pyrimidine-2-amine;
5-Cyano-N-[4-(2-N,N-diethylaminoethylaminocarboxy)phenyl]-4-phenylpyrimidine-2-amine;
5-Cyano-4-phenyl-N-(4-[2-(2-ethylimidazol-1-yl)ethyl]phenyl)-pyrimidine-2-amine;
4-[4-(1-Amino-1-methylethyl)phenyl]-5-cyano-N-4(1,2,3-triazol-1-yl)-phenyl]pyrimidine-2-amine;
4-[4-(1-Amino-1-methylethyl)phenyl]-5-cyano-N-{4-[2-(2-ethylimidazol-1-yl)ethyl]phenyl}pyrimidine-2-amine;
N-[3-(5-Cyano-4-thiophen-2-ylpyrimidin-2-ylamino)phenyl]-4-(4-methylpiperazin-1-ylmethyl)benzamide;
4-[3-(1-Amino-1-methylethyl)phenyl]-5-cyano-N-{4-[2-(2-methyl-imidazol-1-yl)ethyl]phenyl}pyrimidine-2-amino;
5-Cyano-4-[4-(imiadzol-1-yl)methyl]phenyl-N-(3,4,5-trimethoxyphenyl)-pyrimidine-2-amino;
and the salts, solvates, hydrates and N-oxides thereof.

8. The use of claim 1 wherein the compound is:
4-[4-(1-Amino-1-methylethyl)phenyl]-5-cyano-N-[4(1,2,4-triazol-1-yl)phenyl]pyrimidine-2-amine;
5-Cyano-N-[4-(1,2,4-triazol-1-yl)phenyl]-4-[4-(1-dimethylamino-1-methylethyl)phenyl]pyrimidine-2-amine;
4-[4-(1-Amino-1-methylethyl)phenyl]-5-cyano-N-(4-fluorophenyl)pyrimidine-2-amine;
4-[4-(1-Amino-1-methylethyl)phenyl]-5-cyano-N-{4-[2-piperidin-1-ylethyl]phenyl}pyrimidine-2-amine;
4-[4-(1-Amino-1-methylethyl)phenyl]-5-cyano-N-[4-(2-imidazol-1-ylethyl)phenyl]pyrimidine-2-amine;
4-[4-(1-Amino-1-methylethyl)phenyl]-5-cyano-N-[4-(2-morpholinoethyl)phenyl]pyrimidine-2-amine;
4-[4-(1-Amino-1-methylethyl)phenyl]-5-cyano-N-[3-(2-morpholinoethyl)phenyl]pyrimidine-2-amine;
5-Cyano-4-[4-(1-methyl-1-pyrrolidin-1-ylethyl)phenyl]-N-(4-fluorophenyl)pyrimidine-2-amine;
5-Cyano-4-{2-([2-(diethylamino)ethyl]amino)pyridin-5-yl}-N-(4-fluorophenyl)pyrimidine-2-amine;
4-[4-(1-Amino-1-methylethyl)phenyl]-5-cyano-N-(3-fluorophenyl)pyrimidine-2-amine;
and the salts, solvates, hydrates and N-oxides thereof.

9. A compound of formula (1): 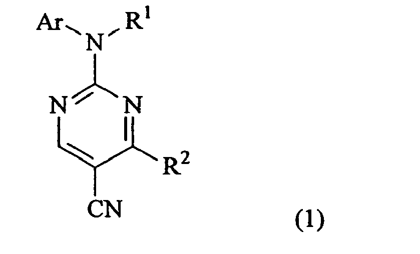 wherein
Ar is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one, two or three substituents, each represented by an atom or group -R⁴ or -Alk(R⁴)ₘ;
R¹ is a hydrogen atom or a straight or branched chain C₁₋₆alkyl group;
R² is a -X¹-R³ group where X¹ is a direct bond; and
R³ is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R^{4b} or -Alk(R^{4b})ₘ;
R⁴ and R^{4b} are each a halogen atom, or an amino (-NH₂), -NHR⁵, nitro, cyano, hydroxyl (-OH), -OR⁵, formyl, carboxyl (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵ group, or a C₆₋₁₂aromatic group, a C₁₋₉heteroaromatic group containing one, two, three or four heteroatoms selected from oxygen, sulphur or nitrogen atoms, a C₃₋₁₀cycloaliphatic group, or a C₃₋₁₀heterocycloaliphatic group containing one, two, three or four heteroatoms or heteroatom-containing groups selected from -O- or -S-atoms or -N(R⁶)-, -C(O)-, -C(S)-, -S(O)- or -S(O₂)-, said aromatic or heteroaromatic groups optionally substituted with one, two or three R^{4a} atoms or groups, said cycloaliphatic or heterocycloaliphatic groups optionally substituted with one, two, three or more substituents selected from halogen atoms, hydroxyl, C₁₋₆alkoxy, thiol, C₁₋₆alkylthio, hydroxy, C₁₋₆alkyl, hydroxyethyl, -CN, -NO₂, -NHR⁵ or -N(R⁵)₂ groups, provided that two R⁴ or -Alk(R⁴)ₘ or two R⁴⁶ or -Alk(R^{4b})ₘ substituents may be linked together to form a C₂₋₆alkylenedioxy group;
Alk¹ is a straight or branched C₁₋₈alkyl group, a C₆₋₁₂arylC₁₋₈alkyl group, a C₆₋₁₂aryl group, a C₆₋₁₂aryloxyC₁₋₈alkyl group, a C₁₋₈alkanoyloxyC₁₋₈alkyl group, or a C₆₋₁₂aroyloxyC₁₋₈alkyl group, where said groups are optionally substituted with one or more R⁴ substituents;
R⁵ is a -Alk(R⁴)ₘ, C₆₋₁₂aromatic group, a C₁₋₉heteroaromatic group containing one, two, three or four heteroatoms selected from oxygen, sulphur or nitrogen atoms, said aromatic or heteroaromatic group optionally substituted with one, two or three R^{4a} atoms or groups;
Alk is a straight or branched C₁₋₆alkylene, C₂₋₆alkenylene or C₂₋₆alkynylene chain, optionally interrupted by one, two or three -O- or -S- atoms or groups selected from -S(O)-, -S(O)₂- or -N(R⁶)-;
R^{4a} is fluorine, chlorine, bromine or iodine atoms, or C₁₋₆alkyl, C₁₋₆alkylamino, hydroxyC₁₋₆alkyl, hydroxyC₂₋₆alkoxy, C₁₋₆alkylthiol, C₁₋₆alkoxy, C₅₋₇cycloalkoxy, haloC₁₋₆alkyl, amino (-NH₂), aminoC₁₋₆alkyl, C₁₋₆dialkylamino, aminoC₁₋₆alkoxy, C₁₋₆alkylaminoC₁₋₆alkoxy, diC₁₋₆alkylaminoC₁₋₆alkoxy, imido, 1,1,3-trioxobenzo[d]thiazolidino, nitro, cyano, hydroxyl (-OH), formyl [HC(O)-], carboxyl (-CO₂H), -CO₂Alk¹, C₁₋₆ alkanoyl, thiol (-SH), thioC₁₋₆alkyl, -SC(NH₂)NH₂, sulphonyl (-SO₃H), C₁₋₆alkylsulphonyl, aminosulphonyl (-SO₂NH₂), C₁₋₆alkylaminosulphonyl, C₁₋₆dialkylaminosulphonyl, phenylaminosulphonyl, carboxamido (-CONH₂), C₁₋₆alkylaminocarbonyl, C₁₋₆dialkylaminocarbonyl, sulphonylamino (-NHSO₂H), C₁₋₆alkylsulphonylamino, C₁₋₆dialkylsulphonylamino, phenylsulphonylamino, 2-nitrophenylsulphonylamino, aminosulphonylamino (-NHSO₂NH₂), C₁₋₆alkylaminosulphonylamino, C₁₋₆dialkylaminosulphonylamino, phenylaminosulphonylamino, aminocarbonylamino, C₁₋₆alkylaminocarbonylamino C₁₋₆dialkylaminocarbonylamino, phenylaminocarbonylamino, C₁₋₆alkanoylamino, phenylcarbonylamino, C₁₋₆alkanoylaminoC₁₋₆alkyl, C₁₋₆alkoxycarbonylamino, heteroC₃₋₆cycloalkyl, 4-(C₁₋₆alkyl)piperazinyl, heteroC₃₋₆cycloalkylC₁₋₆alkyl, 4-(C₁₋₆alkyl)piperazinylC₁₋₆alkyl, heteroC₃₋₆cycloalkylC₁₋₆alkoxy, heteroC₃₋₆alkylC₁₋₆alkylamino, heteroC₃₋₆cycloalkylamino, tetrazolyl, imidazolyl, triazolyl, imidazolylC₁₋₆alkyl, imidazolylC₁₋₆alkoxy, triazolylC₁₋₆alkoxy, imidazolylaminoC₁₋₆alkoxy, phenylamino, benzylamino, benzyloxy, or pyridylmethylamino group;
R⁶ is a hydrogen atom or a straight or branched chain C₁₋₆alkyl group;
m is zero or an integer 1, 2 or 3;
and the salts, solvates, hydrates and N-oxides thereof.

10. A compound of formula (1) 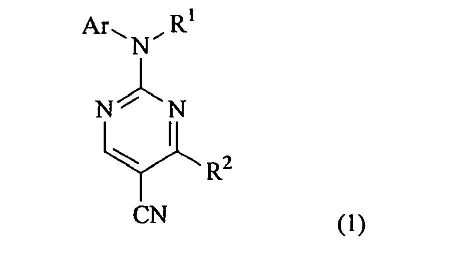 for treating a disease associated with KDR kinase and/or FGFr kinase
wherein
Ar is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R⁴ or -Alk(R⁴)ₘ;
R¹ is a hydrogen atom or a straight or branched chain C₁₋₆alkyl group;
R² is a -X¹-R³ group where X¹ is a direct bond; and
R³ is a C₆₋₁₂aromatic or C₁₋₁₃heteroaromatic group, said heteroaromatic group containing one or two ring oxygen, sulphur and/or nitrogen atoms, said aromatic or heteroaromatic group optionally substituted with one or more substituents, each represented by an atom or group -R^{4b} or -Alk(R^{4b})ₘ;
R⁴ and R^{4b} are each a halogen atom, or an amino (-NH₂), -NHR⁵, nitro, cyano, hydroxyl (-OH), -OR⁵, formyl, carboxyl (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵ group, or a C₆₋₁₂aromatic group, a C₁₋₉heteroaromatic group containing one, two, three or four heteroatoms selected from oxygen, sulphur or nitrogen atoms, a C₃₋₁₀cycloaliphatic group, or a C₃₋₁₀heterocycloaliphatic group containing one, two, three or four heteroatoms or heteroatom-containing groups selected from -O- or -S-atoms or -N(R⁶)-, -C(O)-, -C(S)-, -S(O)- or -S(O₂)-, said aromatic or heteroaromatic groups optionally substituted with one, two or three R^{4a} atoms or groups, said cycloaliphatic or heterocycloaliphatic groups optionally substituted with one, two, three or more substituents selected from halogen atoms, hydroxyl, C₁₋₆alkoxy, thiol, C₁₋₆alkylthio, hydroxy, C₁₋₆alkyl, hydroxyethyl, -CN, -NO₂, -NHR⁵ or -N(R⁵)₂ groups, provided that two R⁴ or -Alk(R⁴)ₘ or two R^{4b} or -Alk(R^{4b})ₘ substituents may be linked together to form a C₂₋₆alkylenedioxy group;
Alk¹ is a straight or branched C₁₋₈alkyl group, a C₆₋₁₂arylC₁₋₈alkyl group, a C₆₋₁₂aryl group, a C₆₋₁₂aryloxyC₁₋₈alkyl group, a C₁₋₈alkanoyloxyC₁₋₈alkyl group, or a C₆₋₁₂aroyloxyC₁₋₈alkyl group, where said groups are optionally substituted with one or more R⁴ substituents;
R⁵ is a -Alk(R⁴)ₘ, C₆₋₁₂aromatic group, a C₁₋₉heteroaromatic group containing one, two, three or four heteroatoms selected from oxygen, sulphur or nitrogen atoms, said aromatic or heteroaromatic group optionally substituted with one, two or three R^{4a} atoms or groups;
Alk is a straight or branched C₁₋₆alkylene, C₂₋₆alkenylene or C₂₋₆alkynylene chain, optionally interrupted by one, two or three -O- or -S- atoms or groups selected from -S(O)-, -S(O)₂- or -N(R⁶)-;
R^{4a} is fluorine, chlorine, bromine or iodine atoms, or C₁₋₆alkyl, C₁₋₆alkylamino, hydroxyC₁₋₆alkyl, hydroxyC₂₋₆alkoxy, C₁₋₆alkylthiol, C₁₋₆alkoxy, C₅₋₇cycloalkoxy, haloC₁₋₆alkyl, amino (-NH₂), aminoC₁₋₆alkyl, C₁₋₆dialkylamino, aminoC₁₋₆alkoxy, C₁₋₆alkylaminoC₁₋₆alkoxy, diC₁₋₆alkylaminoC₁₋₆alkoxy, imido, 1,1,3-trioxobenzo[d]thiazolidino, nitro, cyano, hydroxyl (-OH), formyl [HC(O)-], carboxyl (-CO₂H), -CO₂Alk¹, C₁₋₆ alkanoyl, thiol (-SH), thioC₁₋₆alkyl, -SC(NH₂)NH₂, sulphonyl (-SO₃H), C₁₋₆alkylsulphonyl, aminosulphonyl (-SO₂NH₂), C₁₋₆alkylaminosulphonyl, C₁₋₆dialkylaminosulphonyl, phenylaminosulphonyl, carboxamido (-CONH₂), C₁₋₆alkylaminocarbonyl, C₁₋₆dialkylaminocarbonyl, sulphonylamino (-NHSO₂H), C₁₋₆alkylsulphonylamino, C₁₋₆dialkylsulphonylamino, phenylsulphonylamino, 2-nitrophenylsulphonylamino, aminosulphonylamino (-NHSO₂NH₂), C₁₋₆alkylaminosulphonylamino, C₁₋₆dialkylaminosulphonylamino, phenylaminosulphonylamino, aminocarbonylamino, C₁₋₆alkylaminocarbonylamino C₁₋₆dialkylaminocarbonylamino, phenylaminocarbonylamino, C₁₋₆alkanoylamino, phenylcarbonylamino, C₁₋₆alkanoylaminoC₁₋₆alkyl, C₁₋₆alkoxycarbonylamino, heteroC₃₋₆cycloalkyl, 4-(C₁₋₆alkyl)piperazinyl, heteroC₃₋₆cycloalkylC₁₋₆alkyl, 4-(C₁₋₆alkyl)piperazinylC₁₋₆alkyl, heteroC₃₋₆cycloalkylC₁₋₆alkoxy, heteroC₃₋₆alkylC₁₋₆alkylamino, heteroC₃₋₆cycloalkylamino, tetrazolyl, imidazolyl, triazolyl, imidazolylC₁₋₆alkyl, imidazolylC₁₋₆alkoxy, triazolylC₁₋₆alkoxy, imidazolylaminoC₁₋₆alkoxy, phenylamino, benzylamino, benzyloxy, or pyridylmethylamino group;
R⁶ is a hydrogen atom or a straight or branched chain C₁₋₆alkyl group;
m is zero or an integer 1, 2 or 3;
and the salts, solvates, hydrates and N-oxides thereof.

11. A compound of claim 9 or claim 10 wherein R³ is a phenyl, thienyl, thiazolyl, indolyl or pyridyl group optionally substituted by one, two or three -R^{4b} or -Alk(R^{4b})ₘ substituents.

12. A compound of any one of claims 9 to 11 wherein Ar is a phenyl, pyridyl, indolyl, indazolyl, benzimidazolyl, benzothiazolyl, quinolyl, isoquinolyl or benzoxazolyl group each substituted by one, two or three -R⁴ or -Alk(R⁴)ₘ substituents.

13. A compound of claim 12 wherein Ar is a phenyl group substituted by one, two or three -R⁴ or -Alk(R⁴)ₘ substituents.

14. A compound of any one of claims 11 to 13 wherein at least one of -R⁴, -Alk(R⁴)ₘ, -R^{4b} or -Alk(R^{4b})ₘ, is a -X^{1a}(Alk^{a})ₚNR^{7a}R^{7b}) -X^{1a}(Alk^{a})ₚNHet¹ or -X^{1a}(Alk^{a})ₚAr² group, where
X^{1a} is a direct bond, or a linker atom or group selected from -O-, -S-, -C(O)-, -C(S)-, -S(O)-, -S(O)₂-, -N(R⁷)-, -C(R⁷)₂-, -CON(R⁷)-, -OC(O)N(R⁷)-, -CSN(R⁷)-, -N(R⁷)CO-, -N(R⁷)C(O)O-, -N(R⁷)CS-, -SON(R⁷)-, -SO₂N(R⁷)-, -N(R⁷)SO₂-, -N(R⁷)CON(R⁷)-, -N(R⁷)CSN(R⁷)-, -N(R⁷)SON(R⁷)- or -N(R⁷)SO₂N(R⁷),
Alk^{a} is as defined for Alk,
p is zero or an integer 1,
R⁷ is a hydrogen atom or a C₁₋₆alkyl group,
R^{7a} and R^{7b} which may be the same or different is each a hydrogen atom or a straight or branched C₁₋₆alkyl group, and
-NHet¹ is an optionally substituted C₃₋₇cyclicamino group optionally containing one or more -O- or -S- atoms or -N(R⁶)-.

15. The compound of claims 9 or claim 10 which is:
5-Cyano-4-phenyl-N-(3,4,5-trimethoxyphenyl)pyrimidine-2-amine;
5-Cyano-N-[4-(2-imidazol-1-ylethyl)phenyl]-4-(4-methoxcarbonylphenyl)pyrimidine-2-amine;
5-Cyano-4-(4-hydroxymethylphenyl)-N-(3,4,5-trimethoxyphenyl)-pyrimidine-2-amine;
5-Cyano-4[(4-N,N-diethylaminomethyl)phenyl]-N-(3,4,5-trimethoxyphenyl)pyrimidine-2-amine;
5-Cyano-4-[2-(3(R)-dimethylaminopyrrolidin-1-yl)pyridin-5-yl]-N-(indazol-5-yl)pyrimidine-2-amine;
4-[4-(1-Amino-1-methylethyl)phenyl]-5-cyano-N-(indazol-5-yl)-pyrimidine-2-amine;
4-[4-(1-Amino-1-methylethyl)phenyl]-5-cyano-N-(3,4,5-trimethoxyphenyl)pyrimidine-2-amine;
5-Cyano-N-[4-(2-N,N-diethylaminoethylaminocarboxy)phenyl]-4-phenylpyrimidine-2-amine;
5-Cyano-4-phenyl-N-{4-[2-(2-ethylimidazol-1-yl)ethyl]phenyl}-pyrimidine-2-amine;
4-[4-(1-Amino-1-methylethyl)phenyl]-5-cyano-N-4(1,2,3-triazol-1-yl)-phenyl]pyrimidine-2-amine;
4-[4-(1-Amino-1-methylethyl)phenyl]-5-cyano-N-{4-[2-(2-ethylimidazol-1-yl)ethyl]phenyl}pyrimidine-2-amine;
N-[3-(5-Cyano-4-thiophen-2-ylpyrimidin-2-ylamino)phenyl]-4-(4-methylpiperazin-1-ylmethyl)benzamide;
4-[3-(1-Amino-1-methylethyl)phenyl]-5-cyano-N-(4-[2-(2-methyl-imidazol-1-yl)ethyl]phenyl}pyrimidine-2-amino;
5-Cyano-4-[4-(imiadzol-1-yl)methyl)phenyl-N-(3,4,5-trimethoxyphenyl)-pyrimidine-2-amino;
and the salts, solvates, hydrates and N-oxides thereof.

16. The compound of claim 9 or claim 10 which is:
4-[4-(1-Amino-1-methylethyl)phenyl]-5-cyano-N-[4(1,2,4-triazol-1-yl)phenyl]pyrimidine-2-amine;
5-Cyano-N-[4-(1,2,4-triazol-1-yl)phenyl]-4-[4-(1-dimethylamino-1-methylethyl)phenyl]pyrimidine-2-amine;
4-[4-(1-Amino-1-methylethyl)phenyl]-5-cyano-N-(4-fluorophenyl)pyrimidine-2-amine;
4-[4-(1-Amino-1-methylethyl)phenyl]-5-cyano-N-{4-[2-piperidin-1-ylethyl]phenyl}pyrimidine-2-amine;
4-[4-(1-Amino-1-methylethyl)phenyl]-5-cyano-N-[4-(2-imidazol-1-ylethyl)phenyl]pyrimidine-2-amine;
4-[4-(1-Amino-1-methylethyl)phenyl]-5-cyano-N-[4-(2-morpholinoethyl)phenyl]pyrimidine-2-amine;
4-[4-(1-Amino-1-methylethyl)phenyl]-5-cyano-N-[3-(2-morpholinoethyl)phenyl]pyrimidine-2-amine;
5-Cyano-4-[4-(1-methyl-1-pyrrolidin-1-ylethyl)phenyl]-N-(4-fluorophenyl)pyrimidine-2-amine;
5-Cyano-4-{2-([2-(diethylamino)ethyl]amino)pyridin-5-yl}-N-(4-fluorophenyl)pyrimidine-2-amine;
4-[4-(1-Amino-1-methylethyl)phenyl]-5-cyano-N-(3-fluorophenyl)pyrimidine-2-amine;
and the salts, solvates, hydrates and N-oxides thereof.

17. A pharmaceutical composition comprising a compound according to any one of claims 9 to 16 together with one or more pharmaceutically acceptable carriers, excipients or diluents.

## Patentansprüche

1. Verwendung einer Verbindung der Formel (1) 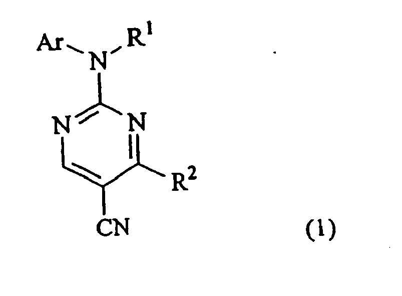 oder eines Salzes, Solvates, Hydrates oder N-Oxides davon, für die Herstellung eines Medikaments für die Behandlung eines mit Angiogenese verbundenen Krankheitszustandes,
wobei
Ar eine aromatische C₆₋₁₂- oder heteroaromatische C₁₋₁₃-Gruppe ist, wobei die heteraromatische Gruppe ein oder zwei Ringsauerstoff-, -schwefel- und/oder -stickstoffatome enthält, die aromatische oder heteroaromatische Gruppe gegebenenfalls mit einem oder mehreren Substituenten substituiert ist, wobei jeder durch ein Atom oder eine Gruppe -R⁴ oder -Alk(R⁴)ₘ dargestellt ist,
R¹ ein Wasserstoffatom oder eine gerad- oder verzweigtkettige C₁₋₆-Alkylgruppe ist,
R² eine -X¹-R³-Gruppe ist, wobei X¹ eine direkte Bindung ist, und
R³ eine aromatische C₆₋₁₂- oder heteroaromatische C₁₋₁₃-Gruppe ist, wobei die heteraromatische Gruppe ein oder zwei Ringsauerstoff-, -schwefel- und/oder -stickstoffatome enthält, die aromatische oder heteroaromatische Gruppe gegebenenfalls mit einem oder mehreren Substituenten substituiert ist, wobei jeder durch ein Atom oder eine Gruppe -R^{4b} oder -Alk(R^{4b})ₘ dargestellt ist,
R⁴ und R^{4b} jeweils ein Halogenatom oder eine Amino- (-NH₂), -NHR⁵-, Nitro-, Cyano-, Hydroxyl- (-OH), -OR⁵-, Formyl-, Carboxyl- (-CO₂H), -CO₂Alk¹-, Thiol-(-SH), -SR⁵-, -COR⁵-, -CSR⁵-, -SO₃H-, -SO₂R⁵-, -SO₂NH₂-, -SO₂NHR⁵-, -SO₂N[R⁵]₂-, -CONH₂-, -CSNH₂-, -CONHR⁵-, -CSNHR⁵-, - CON[R⁵]₂-, -CSN[R⁵]₂-, -NHSO₂H-, -NHSO₂R⁵-, -N[SO₂R⁵]₂-, -NHSO₂NH₂-, -NHSO₂NHR⁵-, -NHSO₂N[R⁵]₂-, -NHCOR⁵-, - NHCONH₂-, -NHCONHR⁵-, -NHCON[R⁵]₂-, -NHCSR⁵-, -NHC(O)OR⁵-Gruppe, oder eine aromatische C₆₋₁₂-Gruppe, eine heteroaromatische C₁₋₉-Gruppe, die ein, zwei, drei oder vier Heteroatome, ausgewählt aus Sauerstoff-, Schwefel- oder Stickstoffatomen, enthält, eine cycloaliphatische C₃₋₁₀-Gruppe, oder eine heterocylcoaliphatische C₃₋₁₀-Gruppe, die ein, zwei, drei oder vier Heteroatome oder Heteroatom-haltige Gruppen, ausgewählt aus -0- oder -S-Atomen oder -N(R⁶)-, -C(O)-, -C(S)-, -S(O)- oder -S(O₂)-, enthält, ist, wobei die aromatischen oder heteroaromatischen Gruppen gegebenenfalls mit ein, zwei oder drei R^{4a}-Atomen oder -Gruppen substituiert sind, die cycloaliphatischen oder heterocycloaliphatischen Gruppen gegebenenfalls mit einem, zwei, drei oder mehreren Substituenten, ausgewählt aus Halogenatomen, Hydroxyl-, C₁₋₆-Alkoxy-, Thiol-, C₁₋₆-Alkylthio-, Hydroxy-, C₁₋₆-Alkyl-, Hydroxyethyl-, -CN-, NO₂-, -NHR⁵- oder N(R⁵)₂-Gruppen, substituiert sind, mit der Maßgabe, dass zwei R⁴- oder -Alk(R⁴)ₘ- oder zwei R^{4b}- oder -Alk(R^{4b})ₘ-Substituenten miteinander verbunden sein können, um eine C₂₋₆-Alkylendioxy-Gruppe zu bilden,
Alk¹ eine geradkettige oder verzweigtkettige C₁₋₈-Alkylgruppe, eine C₆₋₁₂-Aryl-C₁₋₈-alkylgruppe, eine C₆₋₁₂-Arylgruppe, eine C₆₋₁₂-Aryloxy-C₁₋₈-alkylgruppe, eine C₁₋₈-Alkanoyloxy-C₁₋₈-alkylgruppe oder eine C₆₋₁₂-Aroyloxy-C₁₋₈-alkylgruppe ist, wobei die Gruppen gegebenenfalls mit einem oder mehreren R⁴-Substituenten substituiert sind,
R⁵ eine -Alk(R⁴)ₘ-, aromatische C₆₋₁₂-Gruppe, eine heteroaromatische C₁₋₉-Gruppe, die ein, zwei, drei oder vier Heteroatome, ausgewählt aus Sauerstoff-, Schwefel- oder Stickstoffatomen, enthält, ist, wobei die aromatische oder heteroaromatische Gruppe gegebenenfalls mit ein, zwei oder drei R^{4a}-Atomen oder -Gruppen substituiert ist,
Alk eine gerade oder verzweigte C₁₋₆-Alkylen-, C₂₋₆-Alkenylen- oder C₂₋₆-Alkinylenkette ist, gegebenenfalls unterbrochen durch ein, zwei oder drei -O- oder -S-Atome oder Gruppen, ausgewählt aus -S(O)-, -S(O₂)- oder -N(R⁶)-,
R^{4a} Fluor-, Chlor-, Brom- oder lodatome, oder eine C₁₋₆-Alkyl-, C₁₋₆-Alkylamino-, Hydroxy-C₁₋₆-alkyl-, Hydroxy-C₂₋₆-alkoxy-, C₁₋₆-Alkylthiol-, C₁₋₆-Alkoxy-, C₅₋₇-Cycloalkoxy-, Halogen-C₁₋₆-alkyl-, Amino- (-NH₂), Amino-C₁₋₆-alkyl-, C₁₋₆-Dialkylamino-, Amino-C₁₋₆-alkoxy-, C₁₋₆-Alkylamino-C₁₋₆-alkoxy-, Di-C₁₋₆-Alkylamino-C₁₋₆-alkoxy-, Imido-, 1,1,3-Trioxobenzo[d]thiazolidino-, Nitro-, Cyano-, Hydroxyl- (-OH), Formyl- [HC(O)-], Carboxyl- (-CO₂H), -CO₂Alk¹, C₁₋₆-Alkanoyl-, Thiol- (-SH), Thio-C₁₋₆-alkyl-, -SC(NH₂)NH₂-, Sulfonyl- (-SO₃H), C₁₋₆-Alkylsulfonyl-, Aminosulfonyl- (-SO₂NH₂), C₁₋₆-Alkylaminosulfonyl-, C₁₋₆-Dialkylaminosulfonyl, Phenylaminosulfonyl-, Carboxamido- (-CONH₂), C₁₋₆-Alkylaminocarbonyl-, C₁₋₆-Dialkylaminocarbonyl-, Sulfonylamino-(-NHSO₂H), C₁₋₆-Alkylsulfonylamino-, C₁₋₆-Dialkylsulfonylamino-, Phenylsulfonylamino-, 2-Nitrophenylsulfonylamino-, Aminosulfonylamino- (-NHSO₂NH₂), C₁₋₆-Alkylaminosulfonylamino-, C₁₋₆-Dialkylaminosulfonylamino-, Phenylaminosulfonylamino-, Aminocarbonylamino-, C₁₋₆-Alkylaminocarbonylamino-, C₁₋₆-Dialkylaminocarbonylamino-, Phenylaminocarbonylamino-, C₁₋₆-Alkanoylamino-, Phenylcarbonylamino-, C₁₋₆-Alkanoylamino-C₁₋₆-alkyl-, C₁₋₆-Alkoxycarbonylamino-, Hetero-C₃₋₆-cycloalkyl-, 4-(C₁₋₆-Alkyl)piperazinyl-, Hetero-C₃₋₆-cycloalkyl-C₁₋₆-alkyl-, 4-(C₁₋₆-Alkyl)piperazinyl-C₁₋₆-alkyl-, Hetero-C₃₋₆-cycloalkyl-C₁₋₆-alkoxy-, Hetero-C₃₋₆-alkyl-C₁₋₆-alkylamino-, Hetero-C₃₋₆-cycloalkylamino-, Tetrazolyl-, Imidazolyl-, Triazolyl-, Imidazolyl-C₁₋₆-alkyl-, Imidazolyl-C₁₋₆-alkoxy-, Triazolyl-C₁₋₆-alkoxy-, Imidazolylamino-C₁₋₆-alkoxy-, Phenylamino-, Benzylamino-, Benzyloxy- oder Pyridylmethylaminogruppe ist,
R⁶ ein Wasserstoffatom oder eine gerad- oder verzweigtkettige C₁₋₆-Alkylgruppe ist,
m Null oder eine ganze Zahl 1, 2 oder 3 ist.

2. Verwendung nach Anspruch 1, wobei die Krankheit Krebs, Psoriasis, rheumatische Arthritis, Kaposi-Sarkom, ischämische Herzerkrankung, Atherosklerose oder eine Augenerkrankung ist.

3. Verwendung nach Anspruch 1 oder Anspruch 2, wobei R³ eine Phenyl-, Thienyl-, Thiazolyl-, Indolyl- oder Pyridylgruppe ist, gegebenenfalls mit einem, zwei oder drei -R^{4b}- oder -Alk(R^{4b})ₘ-Substituenten substituiert.

4. Verwendung nach einem der Ansprüche 1 bis 3, wobei Ar eine Phenyl-, Pyridyl-, Indolyl-, Indazolyl-, Benzimidazolyl-, Benzothiazolyl-, Chinolyl-, Isochinolyl- oder Benzoxazolylgruppe ist, jeweils substituiert mit einem, zwei oder drei -R⁴- oder Alk(R⁴)ₘ-Substituenten.

5. Verwendung nach Anspruch 4, wobei Ar eine Phenylgruppe ist, die mit einem, zwei oder drei -R⁴- oder Alk(R⁴)ₘ-Substituenten substituiert ist.

6. Verwendung nach einem der Ansprüche 3 bis 5, wobei mindestens eines von -R⁴, -Alk(R⁴)ₘ, -R^{4b} oder -Alk(R^{4b})ₘ eine -X^{1a}(Alk^{a})ₚNR^{7a}R^{7b}-, -X^{1a}(Alk^{a})ₚNHet¹ oder -X^{1a}(Alk^{a})ₚAr²-Gruppe ist,
wobei X^{1a} eine direkte Bindung oder ein Linkeratom oder eine Linkergruppe, ausgewählt aus -O-, -S-, -C(O)-, -C(S)-, -S(O)-, -S(O)₂-, -N(R⁷), -C(R⁷)₂-, -CON(R⁷)-, -OC(O)N(R⁷)-, -CSN(R⁷)-, -N(R⁷)CO-, -N(R⁷)C(O)O-, -N(R⁷)CS-, -SON(R⁷)-, -SO₂N(R⁷)-, -N(R⁷)SO₂-, -N(R⁷)CON(R⁷)-, -N(R⁷)CSN(R⁷)-, -N(R⁷)SON(R⁷)- oder -N(R⁷)SO₂N(R⁷), ist,
Alk^{a} wie für Alk definiert ist,
p Null oder eine ganze Zahl 1 ist,
R⁷ ein Wasserstoffatom oder eine C₁₋₆-Alkylgruppe ist,
R^{7a} und R^{7b}, welche gleich oder verschieden sein können, jeweils ein Wasserstoffatom oder eine geradkettige oder verzweigtkettige C₁₋₆-Alkylgruppe ist, und -NHet¹ eine gegebenenfalls substituierte cyclische C₃₋₇-Amingruppe ist, die gegebenenfalls ein oder mehrere -O- oder -S-Atome oder -N(R⁶)- enthält.

7. Verwendung nach Anspruch 1, wobei die Verbindung
5-Cyano-4-phenyl-N-(3,4,5-trimethoxyphenyl)pyrimidin-2-amin,
5-Cyano-N-[4-(2-imidazol-1-ylethyl)phenyl]-4-(4-methoxcarbonylphenyl)pyrimidin-2-amin,
5-Cyano-4-(4-hydroxymethylphenyl)-N-(3,4,5-trimethoxyphenyl)pyrimidin-2-amin,
5-Cyano-4-[(4-N,N-diethylaminomethyl)phenyl]-N-(3,4,5-trimethoxyphenyl)pyrimidin-2-amin,
5-Cyano-4-[2-(3(R)-dimethylaminopyrrolidin-1-yl)pyridin-5-yl]-N-(indazol-5-yl)pyrimidin-2-amin,
4- [4-(1-Amino-1-methylethyl)phenyl]-5-cyano-N-(indazol-5-yl)-pyrimidin-2-amin,
4-[4-(1-Amino-1-methylethyl)phenyl]-5-cyano-N-(3,4,5-trimethoxyphenyl)pyrimidin-2-amin,
5-Cyano-N-[4-(2-N,N-diethylaminoethylaminocarboxy)phenyl]-4-phenylpyrimidin-2-amin,
5-Cyano-4-phenyl-N-{4-[2-(2-ethylimidazol-1-yl)ethyl]phenyl}pyrimidin-2-amin,
4-[4-(1-Amino-1-methylethyl)phenyl]-5-cyano-N-4(1,2,3-triazol-1-yl)phenyl]pyrimidin-2-amin,
4-[4-(1-Amino-1-methylethyl)phenyl]-5-cyano-N-{4-[2-(2-ethylimidazol-1-yl)ethyl]phenyl}pyrimidin-2-amin,
N-[3-(5-Cyano-4-thiophen-2-ylpyrimidin-2-ylamino)phenyl]-4-(4-methylpiperazin-1-ylmethyl)benzamid,
4-[3-(1 -Amino-1-methylethyl)phenyl]-5-cyano-N-{4-[2-(2-methylimidazol-1-yl)ethyl]phenyl}pyrimidin-2-amin;
5-Cyano-4-[4-(imiadzol-1-yl)methyl]phenyl-N-(3,4,5-trimethoxyphenyl)pyrimidin-2-amin,
und die Salze, Solvate, Hydrate und N-Oxide davon ist.

8. Verwendung nach Anspruch 1, wobei die Verbindung
4-[4-(1-Amino-1-methylethyl)phenyl]-5-cyano-N-[4(1,2,4-triazol-1-yl)phenyl]pyrimidin-2-amin,
5-Cyano-N-(1,2,4-triazol-1-yl)phenyl]-4-[4-(1-dimethylamino-1-methylethyl)phenyl]pyrimidin-2-amin,
4-[4-(1-Amino-1-methylethyl)phenyl]-5-cyano-N-(4-fluorphenyl)pyrimidin-2-amin,
4-[4-(1 -Amino-1-methylethyl)phenyl]-5-cyano-N-{4-[2-piperidin-1-ylethyl]phenyl}pyrimidin-2-amin,
4-[4-(1-Amino-1-methylethyl)phenyl]-5-cyano-N-[4-(2-imidazol-1-ylethyl)phenyl]pyrimidin-2-amin,
4-[4-(1-Amino-1-methylethyl)phenyl]-5-cyano-N-[4-(2-morpholinoethyl)phenyl]pyrimidin-2-amin,
4-[4-(1-Amino-1-methylethyl)phenyl]-5-cyano-N-[3-(2-morpholinoethyl)phenyl]pyrimidin-2-amin,
5-Cyano-4-[4-(1-methyl-1-pyrrolidin-1-ylethyl)phenyl]-N-(4-fluorphenyl)pyrimidin-2-amin,
5-Cyano-4-{2-([2-(diethylamino)ethyl]amino)pyridin-5-yl}-N-(4-fluorophenyl)pyrimidin-2-amin,
4-[4-(1-Amino-1-methylethyl)phenyl]-5-cyano-N-(3-fluorphenyl)pyrimidin-2-amin,
und die Salze, Solvate, Hydrate und N-Oxide davon ist.

9. Verbindung der Formel (1): 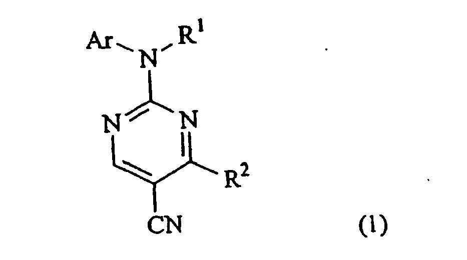 wobei
Ar eine aromatische C₆₋₁₂- oder heteroaromatische C₁₋₁₃-Gruppe ist, wobei die heteraromatische Gruppe ein oder zwei Ringsauerstoff-, -schwefel- und/oder -stickstoffatome enthält, die aromatische oder heteroaromatische Gruppe gegebenenfalls mit einem, zwei oder drei Substituenten substituiert ist, wobei jeder durch ein Atom oder eine Gruppe -R⁴ oder -Alk(R⁴)ₘ dargestellt ist,
R¹ ein Wasserstoffatom oder eine gerad- oder verzweigtkettige C₁₋₆-Alkylgruppe ist,
R² eine -X¹-R³-Gruppe ist, wobei X¹ eine direkte Bindung ist, und
R³ eine aromatische C₆₋₁₂- oder heteroaromatische C₁₋₁₃-Gruppe ist, wobei die heteraromatische Gruppe ein oder zwei Ringsauerstoff-, -schwefel- und/oder -stickstoffatome enthält, die aromatische oder heteroaromatische Gruppe gegebenenfalls mit einem oder mehreren Substituenten substituiert ist, wobei jeder durch ein Atom oder eine Gruppe -R^{4b} oder -Alk(R^{4b})ₘ dargestellt ist,
R⁴ und R^{4b} jeweils ein Halogenatom oder eine Amino- (-NH₂), -NHR⁵-, Nitro-, Cyano-, Hydroxyl- (-OH), -OR⁵-, Formyl-, Carboxyl- (-CO₂H), -CO₂Alk¹-, Thiol-(-SH), -SR⁵-, -COR⁵-, CSR⁵-, -SO₃H-, -SO₂R⁵-, -SO₂NH₂-, -SO₂NHR⁵-, -SO₂N[R⁵]₂-, -CONH₂-, -CSNH₂-, -CONHR⁵-, -CSNHR⁵-, -CON[R⁵]₂-, -CSN[R⁵]₂-, -NHSO₂H-, -NHSO₂R⁵-, -N[SO₂R⁵]₂-, -NHSO₂NH₂-, -NHSO₂NHR⁵-, -NHSO₂N[R⁵]₂-, -NHCOR⁵-, -NHCONH2-, -NHCONHR⁵-, -NHCON[R⁵]₂-, -NHCSR⁵-, -NHC(O)OR⁵-Gruppe, oder eine aromatische C₆₋₁₂-Gruppe, eine heteroaromatische C₁₋₉-Gruppe, die ein, zwei, drei oder vier Heteroatome, ausgewählt aus Sauerstoff-, Schwefel- oder Stickstoffatomen, enthält, eine cycloaliphatische C₃₋₁₀-Gruppe, oder eine heterocylcoaliphatische C₃₋₁₀-Gruppe, die ein, zwei, drei oder vier Heteroatome oder Heteroatom-haltige Gruppen, ausgewählt aus -O- oder -S-Atomen oder -N(R⁶)-, -C(O)-, -C(S)-, -S(O)- oder -S(O₂)-, enthält, ist, wobei die aromatischen oder heteroaromatischen Gruppen gegebenenfalls mit ein, zwei oder drei R^{4a}-Atomen oder -Gruppen substituiert sind, die cycloaliphatischen oder heterocycloaliphatischen Gruppen gegebenenfalls mit einem, zwei, drei oder mehreren Substituenten, ausgewählt aus Halogenatomen, Hydroxyl-, C₁₋₆-Alkoxy-, Thiol-, C₁₋₆-Alkylthio-, Hydroxy-, C₁₋₆-Alkyl-, Hydroxyethyl-, -CN-, NO₂-, -NHR⁵- oder N(R⁵)₂-Gruppen, substituiert sind, mit der Maßgabe, dass zwei R⁴- oder -Alk(R⁴)ₘ- oder zwei R^{4b}- oder -Alk(R^{4b})ₘ-Substituenten miteinander verbunden sein können, um eine C₂₋₆-Alkylendioxy-Gruppe zu bilden,
Alk¹ eine geradkettige oder verzweigtkettige C₁₋₈-Alkylgruppe, eine C₆₋₁₂-Aryl-C₁₋₈-alkylgruppe, eine C₆₋₁₂-Arylgruppe, eine C₆₋₁₂-Aryloxy-C₁₋₈-alkylgruppe, eine C₁₋₈-Alkanoyloxy-C₁₋₈-alkylgruppe oder eine C₆₋₁₂-Aroyloxy-C₁₋₈-alkylgruppe ist, wobei die Gruppen gegebenenfalls mit einem oder mehreren R⁴-Substituenten substituiert sind,
R⁵ eine -Alk(R⁴)ₘ-, aromatische C₆₋₁₂-Gruppe, eine heteroaromatische C₁₋₉-Gruppe, die ein, zwei, drei oder vier Heteroatome, ausgewählt aus Sauerstoff-, Schwefel- oder Stickstoffatomen, enthält, ist, wobei die aromatische oder heteroaromatische Gruppe gegebenenfalls mit ein, zwei oder drei R^{4a}-Atomen oder -Gruppen substituiert ist,
Alk eine gerade oder verzweigte C₁₋₆-Alkylen-, C₂₋₆-Alkenylen- oder C₂₋₆-Alkinylenkette ist, gegebenenfalls unterbrochen durch ein, zwei oder drei -O- oder -S-Atome oder Gruppen, ausgewählt aus -S(O)-, -S(O₂)- oder -N(R⁶)-,
R^{4a} Fluor-, Chlor-, Brom- oder lodatome, oder eine C₁₋₆-Alkyl-, C₁₋₆-Alkylamino-, Hydroxy-C₁₋₆-alkyl-, Hydroxy-C₂₋₆-alkoxy-, C₁₋₆-Alkylthiol-, C₁₋₆-Alkoxy-, C₅₋₇-Cycloalkoxy-, Halogen-C₁₋₆-alkyl-, Amino- (-NH₂), Amino-C₁₋₆-alkyl-, C₁₋₆-Oialkylamino-, Amino-C₁₋₆-alkoxy-, C₁₋₆-Alkylamino-C₁₋₆-alkoxy-, Di-C₁₋₆-Alkylamino-C₁₋₆-alkoxy-, Imido-, 1,1,3-Trioxobenzo[d]thiazolidino-, Nitro-, Cyano-, Hydroxyl- (-OH), Formyl- [HC(O)-], Carboxyl- (-CO₂H), -CO₂Alk¹, C₁₋₆-Alkanoyl-, Thiol- (-SH), Thio-C₁₋₆-alkyl-, -SC(NH₂)NH₂-, Sulfonyl- (-SO₃H), C₁₋₆-Alkylsulfonyl-, Aminosulfonyl- (-SO₂NH₂), C₁₋₆-Alkylaminosulfonyl-, C₁₋₆-Dialkylaminosulfonyl, Phenylaminosulfonyl-, Carboxamido- (-CONH₂), C₁₋₆-Alkylaminocarbonyl-, C₁₋₆-Dialkylaminocarbonyl-, Sulfonylamino-(-NHSO₂H), C₁₋₆-Alkylsulfonylamino-, C₁₋₆-Dialkylsulfonylamino-, Phenylsulfonylamino-, 2-Nitrophenylsulfonylamino-, Aminosulfonylamino- (-NHSO₂NH₂), C₁₋₆-Alkylaminosulfonylamino-, C₁₋₆-Dialkylaminosulfonylamino-, Phenylaminosulfonylamino-, Aminocarbonylamino-, C₁₋₆-Alkylaminocarbonylamino-, C₁₋₆-Dialkylaminocarbonylamino-, Phenylaminocarbonylamino-, C₁₋₆-Alkanoylamino-, Phenylcarbonylamino-, C₁₋₆-Alkanoylamino-C₁₋₆-alkyl-, C₁₋₆-Alkoxycarbonylamino-, Hetero-C₃₋₆-cycloalkyl-, 4-(C₁₋₆-Alkyl)piperazinyl-, Hetero-C₃₋₆-cycloalkyl-C₁₋₆-alkyl-, 4-(C₁₋₆-Alkyl)piperazinyl-C₁₋₆-alkyl-, Hetero-C₃₋₆-cycloalkyl-C₁₋₆-alkoxy-, Hetero-C₃₋₆-alkyl-C₁₋₆-alkylamino-, Hetero-C₃₋₆-cycloalkylamino-, Tetrazolyl-, Imidazolyl-, Triazolyl-, Imidazolyl-C₁₋₆-alkyl-, Imidazolyl-C₁₋₆-alkoxy-, Triazolyl-C₁₋₆-alkoxy-, Imidazolylamino-C₁₋₆-alkoxy-, Phenylamino-, Benzylamino-, Benzyloxy- oder Pyridylmethylaminogruppe ist,
R⁶ ein Wasserstoffatom oder eine gerad- oder verzweigtkettige C₁₋₆-Alkylgruppe ist,
m Null oder eine ganze Zahl 1, 2 oder 3 ist,
und die Salze, Solvate, Hydrate und N-Oxide davon.

10. Verbindung der Formel (1) 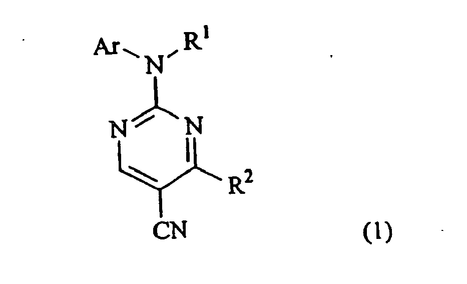 zum Behandeln einer mit KDR-Kinase und/oder FGFr-Kinase verbundenen Erkrankung,
wobei
Ar eine aromatische C₆₋₁₂- oder heteroaromatische C₁₋₁₃-Gruppe ist, wobei die heteraromatische Gruppe ein oder zwei Ringsauerstoff-, -schwefel- und/oder -stickstoffatome enthält, die aromatische oder heteroaromatische Gruppe gegebenenfalls mit einem oder mehreren Substituenten substituiert ist, wobei jeder durch ein Atom oder eine Gruppe -R⁴ oder -Alk(R⁴)ₘ dargestellt ist,
R¹ ein Wasserstoffatom oder eine gerad- oder verzweigtkettige C₁₋₆-Alkylgruppe ist,
R² eine -X¹-R³-Gruppe ist, wobei X¹ eine direkte Bindung ist, und
R³ eine aromatische C₆₋₁₂- oder heteroaromatische C₁₋₁₃-Gruppe ist, wobei die heteraromatische Gruppe ein oder zwei Ringsauerstoff-, -schwefel- und/oder -stickstoffatome enthält, die aromatische oder heteroaromatische Gruppe gegebenenfalls mit einem oder mehreren Substituenten substituiert ist, wobei jeder durch ein Atom oder eine Gruppe -R^{4b} oder -Alk(R^{4b})ₘ dargestellt ist,
R⁴ und R^{4b} jeweils ein Halogenatom oder eine Amino- (-NH₂), -NHR⁵-, Nitro-, Cyano-, Hydroxyl- (-OH), -OR⁵-, Formyl-, Carboxyl- (-CO₂H), -CO₂Alk¹-, Thiol-(-SH), -SR⁵-, -COR⁵-, -CSR⁵-, -SO₃H-, -SO₂R⁵-, -SO₂NH₂-, -SO₂NHR⁵-, -SO₂N[R⁵]₂-, -CONH₂-, -CSNH₂-, -CONHR⁵-, -CSNHR⁵-, -CON[R⁵]₂-, -CSN[R⁵]₂-, -NHSO₂H-, -NHSO₂R⁵-, -N[SO₂R⁵]₂-, -NHSO₂NH₂-, -NHSO₂NHR⁵-, -NHSO₂N[R⁵]₂-, -NHCOR⁵-, NHCONH₂-, -NHCONHR⁵-, -NHCON[R⁵]₂-, -NHCSR⁵-, -NHC(O)OR⁵-Gruppe, oder eine aromatische C₆₋₁₂-Gruppe, eine heteroaromatische C₁₋₉-Gruppe, die ein, zwei, drei oder vier Heteroatome, ausgewählt aus Sauerstoff-, Schwefel- oder Stickstoffatomen, enthält, eine cycloaliphatische C₃₋₁₀-Gruppe, oder eine heterocylcoaliphatische C₃₋₁₀-Gruppe, die ein, zwei, drei oder vier Heteroatome oder Heteroatom-haltige Gruppen, ausgewählt aus -O- oder -S-Atomen oder -N(R⁶)-, -C(O)-, -C(S)-, -S(O)- oder -S(O₂)-, enthält, ist, wobei die aromatischen oder heteroaromatischen Gruppen gegebenenfalls mit ein, zwei oder drei R^{4a}-Atomen oder -Gruppen substituiert sind, die cycloaliphatischen oder heterocycloaliphatischen Gruppen gegebenenfalls mit einem, zwei, drei oder mehreren Substituenten, ausgewählt aus Halogenatomen, Hydroxyl-, C₁₋₆-Alkoxy-, Thiol-, C₁₋₆-Alkylthio-, Hydroxy-, C₁₋₆-Alkyl-, Hydroxyethyl-, -CN-, NO₂-, -NHR⁵- oder N(R⁵)₂-Gruppen, substituiert sind, mit der Maßgabe, dass zwei R⁴- oder -Alk(R⁴)ₘ- oder zwei R^{4b}- oder -Alk(R^{4b})ₘ-Substituenten miteinander verbunden sein können, um eine C₂₋₆-Alkylendioxy-Gruppe zu bilden,
Alk¹ eine geradkettige oder verzweigtkettige C₁₋₈-Alkylgruppe, eine C₆₋₁₂-Aryl-C₁₋₈-alkylgruppe, eine C₆₋₁₂-Arylgruppe, eine C₆₋₁₂-Ary)oxy-C₁₋₈-alkytgruppe, eine C₁₋₈-Alkanoyloxy-C₁₋₈-alkylgruppe oder eine C₆₋₁₂-Aroyloxy-C₁₋₈-alkylgruppe ist, wobei die Gruppen gegebenenfalls mit einem oder mehreren R⁴-Substituenten substituiert sind,
R⁵ eine -Alk(R⁴)ₘ-, aromatische C₆₋₁₂-Gruppe, eine heteroaromatische C₁₋₉-Gruppe, die ein, zwei, drei oder vier Heteroatome, ausgewählt aus Sauerstoff-, Schwefel- oder Stickstoffatomen, enthält, ist, wobei die aromatische oder heteroaromatische Gruppe gegebenenfalls mit ein, zwei oder drei R^{4a}-Atomen oder -Gruppen substituiert ist,
Alk eine gerade oder verzweigte C₁₋₆-Alkylen-, C₂₋₆-Alkenylen- oder C₂₋₆-Alkinylenkette ist, gegebenenfalls unterbrochen durch ein, zwei oder drei -O- oder -S-Atome oder Gruppen, ausgewählt aus -S(O)-, -S(O₂)- oder -N(R⁶)-,
R^{4a} Fluor-, Chlor-, Brom- oder lodatome, oder eine C₁₋₆-Alkyl-, C₁₋₆-Alkylamino-, Hydroxy-C₁₋₆-alkyl-, Hydroxy-C₂₋₆-alkoxy-, C₁₋₆-Alkylthiol-, C₁₋₆-Alkoxy-, C₅₋₇-Cycloalkoxy-, Halogen-C₁₋₆-alkyl-, Amino- (-NH₂), Amino-C₁₋₆-alkyl-, C₁₋₆-Dialkylamino-, Amino-C₁₋₆-alkoxy-, C₁₋₆-Alkylamino-C₁₋₆-alkoxy-, Di-C₁₋₆-Alkylamino-C₁₋₆-alkoxy-, Imido-, 1,1,3-Trioxobenzo[d]thiazolidino-, Nitro-, Cyano-, Hydroxyl- (-OH), Formyl- [HC(O)-], Carboxyl- (-CO₂H), -CO₂Alk¹, C₁₋₆-Alkanoyl-, Thiol- (-SH), Thio-C₁₋₆-alkyl-, -SC(NH₂)NH₂-, Sulfonyl- (-SO₃H), C₁₋₆-Alkylsulfonyl-, Aminosulfonyl- (-SO₂NH₂), C₁₋₆-Alkylaminosulfonyl-, C₁₋₆-Dialkylaminosulfonyl, Phenylaminosulfonyl-, Carboxamido- (-CONH₂), C₁₋₆-Alkylaminocarbonyl-, C₁₋₆-Dialkylaminocarbonyl-, Sulfonylamino-(-NHSO₂H), C₁₋₆-Alkylsulfonylamino-, C₁₋₆-Dialkylsulfonylamino-, Phenylsulfonylamino-, 2-Nitrophenylsulfonylamino-, Aminosulfonylamino- (-NHSO₂NH₂), C₁₋₆-Alkylaminosulfonylamino-, C₁₋₆-Dialkylaminosulfonylamino-, Phenylaminosulfonylamino-, Aminocarbonylamino-, C₁₋₆-Alkylaminocarbonylamino-, C₁₋₆-Dialkylaminocarbonylamino-, Phenylaminocarbonylamino-, C₁₋₆-Alkanoylamino-, Phenylcarbonylamino-, C₁₋₆-Alkanoylamino-C₁₋₆-alkyl-, C₁₋₆-Alkoxycarbonylamino-, Hetero-C₃₋₆-cycloalkyl-, 4-(C₁₋₆-Alkyl)piperazinyl-, Hetero-C₃₋₆-cycloalkyl-C₁₋₆-alkyl-, 4-(C₁₋₆-Alkyl)piperazinyl-C₁₋₆-alkyl-, Hetero-C₃₋₆-cycloalkyl-C₁₋₆-alkoxy-, Hetero-C₃₋₆-alkyl-C₁₋₆-alkylamino-, Hetero-C₃₋₆-cycloalkylamino-, Tetrazolyl-, Imidazolyl-, Triazolyl-, Imidazolyl-C₁₋₆-alkyl-, Imidazolyl-C₁₋₆-alkoxy-, Triazolyl-C₁₋₆-alkoxy-, Imidazolylamino-C₁₋₆-alkoxy-, Phenylamino-, Benzylamino-, Benzyloxy- oder Pyridylmethylaminogruppe ist,
R⁶ ein Wasserstoffatom oder eine gerad- oder verzweigtkettige C₁₋₆-Alkylgruppe ist,
m Null oder eine ganze Zahl 1, 2 oder 3 ist,
oder die Salze, Solvate, Hydrate oder N-Oxide davon.

11. Verbindung nach Anspruch 9 oder Anspruch 10, wobei R³ eine Phenyl-, Thienyl-, Thiazolyl-, Indolyl- oder Pyridylgruppe ist, gegebenenfalls mit einem, zwei oder drei -R^{4b}- oder -Alk(R^{4b})ₘ-Substituenten substituiert.

12. Verbindung nach einem der Ansprüche 9 bis 11, wobei Ar eine Phenyl-, Pyridyl-, Indolyl-, Indazolyl-, Benzimidazolyl-, Benzothiazolyl-, Chinolyl-, Isochinolyl- oder Benzoxazolylgruppe ist, jeweils substituiert mit einem, zwei oder drei -R⁴- oder Alk(R⁴)ₘ-Substituenten.

13. Verbindung nach Anspruch 12, wobei Ar eine Phenylgruppe ist, die mit einem, zwei oder drei -R⁴- oder Alk(R⁴)ₘ-Substituenten substituiert ist.

14. Verbindung nach einem der Ansprüche 11 bis 13, wobei mindestens eines von -R⁴, -Alk(R⁴)ₘ, -R^{4b} oder -Alk(R^{4b})ₘ eine -X^{1a}(Alk^{a})ₚNR^{7a}R^{7b}-, -X^{1a}(Alk^{a})ₚNHet¹ oder -X^{1a}(Alk^{a})ₚAr²-Gruppe ist,
wobei X^{1a} eine direkte Bindung oder ein Linkeratom oder eine Linkergruppe, ausgewählt aus -O-, -S-, -C(O)-, -C(S)-, -S(O)-, -S(O)₂-, -N(R⁷), -C(R⁷)₂-, -CON(R⁷)-, -OC(O)N(R⁷)-, -CSN(R⁷)-, -N(R⁷)CO-, -N(R⁷)C(O)O-, -N(R⁷)CS-, -SON(R⁷)-, -SO₂N(R⁷)-, -N(R⁷)SO₂-, -N(R⁷)CON(R⁷)-, -N(R⁷)CSN(R⁷)-, -N(R⁷)SON(R⁷)- oder -N(R⁷)SO₂N(R⁷), ist,
Alk^{a} wie für Alk definiert ist,
p Null oder eine ganze Zahl 1 ist,
R⁷ ein Wasserstoffatom oder eine C₁₋₆-Alkylgruppe ist,
R^{7a} und R^{7b}, welche gleich oder verschieden sein können, jeweils ein Wasserstoffatom oder eine geradkettige oder verzweigtkettige C₁₋₆-Alkylgruppe ist, und -NHet¹ eine gegebenenfalls substituierte cyclische C₃₋₇-Amingruppe ist, die gegebenenfalls ein oder mehrere -O- oder -S-Atome oder -N(R⁶)- enthält.

15. Verbindung nach Anspruch 9 oder Anspruch 10, welche
5-Cyano-4-phenyl-N-(3,4,5-trimethoxyphenyl)pyrimidin-2-amin,
5-Cyano-N-[4-(2-imidazol-1-ylethyl)phenyl]-4-(4-methoxcarbonylphenyl)pyrimidin-2-amin,
5-Cyano-4-(4-hydroxymethylphenyl)-N-(3,4,5-trimethoxyphenyl)pyrimidin-2-amin,
5-Cyano-4-[(4-N,N-diethylaminomethyl)phenyl]-N-(3,4,5-trimethoxyphenyl)pyrimidin-2-amin,
5-Cyano-4-[2-(3(R)-dimethylaminopyrrolidin-1-yl)pyridin-5-yl]-N-(indazol-5-yl)pyrimidin-2-amin,
4- [4-(1-Amino-1-methylethyl)phenyl]-5-cyano-N-(indazol-5-yl)-pyrimidin-2-amin,
4-[4-(1-Amino-1-methylethyl)phenyl]-5-cyano-N-(3,4,5-trimethoxyphenyl)pyrimidin-2-amin,
5-Cyano-N-[4-(2-N,N-diethylaminoethylaminocarboxy)phenyl]-4-phenylpyrimidin-2-amin,
5-Cyano-4-phenyl-N-{4-[2-(2-ethylimidazol-1-yl)ethyl]phenyl}pyrimidin-2-amin,
4-[4-(1-Amino-1-methylethyl)phenyl]-5-cyano-N-4(1,2,3-triazol-1-yl)phenyl]pyrimidin-2-amin,
4-[4-(1-Amino-1-methylethyl)phenyl]-5-cyano-N-{4-[2-(2-ethylimidazol-1-yl)ethyl]phenyl}pyrimidin-2-amin,
N-[3-(5-Cyano-4-thiophen-2-ylpyrimidin-2-ylamino)phenyl]-4-(4-methylpiperazin-1-ylmethyl)benzamid,
4-[3-(1-Amino-1-methylethyl)phenyl]-5-cyano-N-{4-[2-(2-methylimidazol-1-yl)ethyl]phenyl}pyrimidin-2-amin;
5-Cyano-4-[4-(imiadzol-1-yl)methyl]phenyl-N-(3,4,5-trimethoxyphenyl)pyrimidin-2-amin,
und die Salze, Solvate, Hydrate und N-Oxide davon ist.

16. Verbindung nach Anspruch 9 oder Anspruch 10, welche
4-[4-(1 -Amino-1-methylethyl)phenyl]-5-cyano-N-[4(1,2,4-triazo)-1-yl)phenyl]pyrimidin-2-amin,
5-Cyano-N-(1,2,4-triazol-1-yl)phenyl]-4-[4-(1-dimethylamino-1-methylethyl)phenyl]pyrimidin-2-amin,
4-[4-(1-Amino-1-methylethyl)phenyl]-5-cyano-N-(4-fluorphenyl)pyrimidin-2-amin,
4-[4-(1-Amino-1-methylethyl)phenyl]-5-cyano-N-{4-[2-piperidin-1-ylethyl]phenyl}pyrimidin-2-amin,
4-[4-(1-Amino-1-methylethyl)phenyl]-5-cyano-N-[4-(2-imidazol-1-ylethyl)phenyl]pyrimidin-2-amin,
4-[4-(1-Amino-1-methylethyl)phenyl]-5-cyano-N-[4-(2-morpholinoethyl)phenyl]pyrimidin-2-amin,
4-[4-(1 -Amino-1-methylethyl)phenyl]-5-cyano-N-[3-(2-morpholinoethyl)phenyl]pyrimidin-2-amin,
5-Cyano-4-[4-(1-methyl-1-pyrrolidin-1-ylethyl)phenyl]-N-(4-fluorphenyl)pyrimidin-2-amin,
5-Cyano-4-{2-([2-(diethylamino)ethyl]amino)pyridin-5-yl}-N-(4-fluorophenyl)pyrimidin-2-amin,
4-[4-(1-Amino-1-methylethyl)phenyl]-5-cyano-N-(3-fluorphenyl)pyrimidin-2-amin,
und die Salze, Solvate, Hydrate und N-Oxide davon ist.

17. Pharmazeutische Zusammensetzung, welche eine Verbindung nach einem der Ansprüche 9 bis 16 zusammen mit einem oder mehreren pharmazeutisch verträglichen Trägern, Bindemitteln oder Verdünnungsmitteln umfasst.

## Revendications

1. Utilisation d'un composé de formule (1) 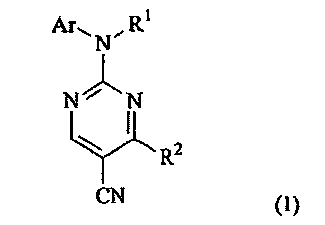 ou d'un sel, produit de solvatation, hydrate, ou N-oxyde de ce dernier, pour préparer un médicament destiné au traitement d'un état pathologique associé à l'angiogenèse,
pour laquelle
Ar est un groupe aromatique en C₆₋₁₂ ou hétéroaromatique en C₁₋₁₃, ledit groupe hétéroaromatique contenant un ou deux atomes dans le cycle d'oxygène, de soufre et/ou d'azote, ledit groupe aromatique ou hétéroaromatique pouvant éventuellement être substitué par un ou plusieurs substituants, dont chacun est représenté par un atome ou un groupe -R⁴ ou -Alk(R⁴)ₘ ;
R¹ est un atome d'hydrogène ou un groupe alkyle en C₁₋₆ à chaîne droite ou ramifiée ;
R² est un groupe -X¹-R³, où X¹ est une liaison directe ; et
R³ est un groupe aromatique en C₆₋₁₂ ou hétéroaromatique en C₁₋₁₃, ledit groupe hétéroaromatique contenant un ou deux atomes dans le cycle d'oxygène, de soufre et/ou d'azote, ledit groupe aromatique ou hétéroaromatique étant éventuellement substitué par un ou plusieurs substituants, dont chacun est représenté par un atome ou un groupe -R^{4b} ou -Alk(R^{4b})ₘ ;
R⁴ et R^{4b} représentent chacun un atome d'halogène ou un groupe amino (-NH₂), -NHR⁵, nitro, cyano, hydroxyle (-OH), -OR⁵, formyle, carboxyle (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵, ou un groupe aromatique en C₆₋₁₂, un groupe hétéroaromatique en C₁₋₉ contenant un, deux, trois ou quatre hétéroatomes choisis parmi les atomes d'oxygène, de soufre ou d'azote, un groupe cycloaliphatique en C₃₋₁₀, ou un groupe hétérocycloaliphatique en C₃₋₁₀ contenant un, deux, trois ou quatre hétéroatomes ou groupes contenant des hétéroatomes choisis parmi les atomes -O- ou -S-, ou -N(R⁶)-, -C(O)-, -C(S)-, -S(O)- ou -S(O₂)-, lesdits groupes aromatiques ou hétéroaromatiques étant éventuellement substitués par un, deux ou trois atomes ou groupes R^{4a}, lesdits groupes cycloaliphatiques ou hétérocycloaliphatiques étant éventuellement substitués par un, deux, trois ou plusieurs substituants choisis parmi les atomes d'halogène et les groupes hydroxyle, alcoxy en C₁₋₆, thiol, alkylthio en C₁₋₆, hydroxy, alkyle en C₁₋₆, hydroxyéthyle, -CN, -NO₂, -NHR⁵ ou -N(R⁵)₂, à la condition que deux substituants R⁴ ou -Alk(R⁴)ₘ ou deux substituants R^{4b} ou -Alk(R^{4b})ₘ puissent être liés l'un à l'autre pour former un groupe alkylènedioxy en C₂₋₆ ;
Alk¹ est un groupe alkyle en C₁₋₈ à chaîne droite ou ramifiée, un groupe (aryle en C₆₋₁₂)(alkyle en C₁₋₈), un groupe aryle en C₆₋₁₂, un groupe (aryloxy en C₆₋₁₂)(alkyle en C₁₋₈), un groupe (alcanoyloxy en C₁₋₈)(alkyle en C₁₋₈) ou un groupe (aroyloxy en C₆₋₁₂)(alkyle en C₁₋₈), ces groupes étant éventuellement substitués par un ou plusieurs substituants R⁴ ;
R⁵ est un -Alk(R⁴)ₘ, un groupe aromatique en C₆₋₁₂, un groupe hétéroaromatique en C₁₋₉ contenant un, deux, trois ou quatre hétéroatomes choisis parmi les atomes d'oxygène, de soufre ou d'azote, ledit groupe aromatique ou hétéroaromatique étant éventuellement substitué par un, deux ou trois atomes ou groupes R^{4a};
Alk est un alkylène en C₁₋₆, alcénylène en C₂₋₆ ou alcynylène en C₂₋₆ à chaîne droite ou ramifiée, éventuellement interrompu par un, deux ou trois atomes -O- ou -S- ou groupes choisis parmi -S(O)-, -S(O)₂- ou -N(R⁶)- ;
R^{4a} désigne un atome de fluor, de chlore, de brome ou d'iode, ou un groupe alkyle en C₁₋₆, alkylamino en C₁₋₆, hydroxyalkyle en C₁₋₆, hydroxyalcoxy en C₂₋₆, alkylthiol en C₁₋₆, alcoxy en C₁₋₆, cycloalcoxy en C₅₋₇, halogènoalkyle en C₁₋₆, amino(-NH₂), aminoalkyle en C₁₋₆, di(alkyle en C₁₋₆)amino, aminoalcoxy en C₁₋₆, (alkylamino en C₁₋₆)(alcoxy en C₁₋₆), di(alkyle en C₁₋₆)amino(alcoxy en C₁₋₆), imido, 1,1,3-trioxobenzo[d]-thiazolidino, nitro, cyano, hydroxyle (-OH), formyle [HC(O)-], carboxyle (-CO₂H), -CO₂Alk¹, alcanoyle en C₁₋₆, thiol (-SH), thioalkyle en C₁₋₆, -SC(NH₂)NH₂, sulfonyle (-SO₃H), alkylsulfonyle en C₁₋₆, aminosulfonyle (-SO₂NH₂), (alkyle en C₁₋₆)aminosulfonyle, di(alkyle en C₁₋₆)aminosulfonyle, phénylaminosulfonyle, carboxamido (-CONH₂), (alkyle en C₁₋₆)aminocarbonyle, di(alkyle en C₁₋₆)aminocarbonyle, sulfonylamino(-NHSO₂H), (alkyle en C₁₋₆)sulfonylamino, di(alkyle en C₁₋₆)sulfonylamino, phénylsulfonylamino, 2-nitrophénylsulfonylamino, aminosulfonylamino (-NHSO₂NH₂), (alkyle en C₁₋₆)aminosulfonylamino, di(alkyle en C₁₋₆)aminosulfonylamino, phénylaminosulfonylamino, aminocarbonylamino, (alkyle en C₁₋₆)aminocarbonylamino, di(alkyle en C₁₋₆)aminocarbonylamino, phénylaminocarbonylamino, alcanoylamino en C₁₋₆, phénylcarbonylamino, (alcanoyle en C₁₋₆)amino(alkyle en C₁₋₆), (alcoxy en C₁₋₆)carbonylamino, hétérocycloalkyle en C₃₋₆, 4-(alkyle en C₁₋₆)pipérazinyle, hétéro(cycloalkyle en C₃₋₆)(alkyle en C₁₋₆), 4-(alkyle en C₁₋₆)pipérazinyl(alkyle en C₁₋₆), hétéro(cycloalkyle en C₃₋₆)(alcoxy en C₁₋₆), hétéro(alkyle en C₃₋₆)(alkyle en C₁₋₆)amino, hétéro(cycloalkyle en C₃₋₆)amino, tetrazolyle, imidazolyle, triazolyle, imidazolyl(alkyle en C₁₋₆), imidazolyl(alcoxy en C₁₋₆), triazolyl(alcoxy en C₁₋₆), imidazolylamino(alcoxy en C₁₋₆), phénylamino, benzylamino, benzyloxy ou pyridylméthylamino ;
R⁶ est un atome d'hydrogène ou un groupe alkyle en C₁₋₆ à chaîne droite ou ramifiée ;
m vaut zéro ou est un entier valant 1, 2 ou 3.

2. Utilisation selon la revendication 1, pour laquelle la maladie est un cancer, un psoriasis, une polyarthrite rhumatoïde, un sarcome de Kaposi, une cardiopathie ischémique, une athérosclérose ou une maladie oculaire.

3. Utilisation selon la revendication 1 ou 2, pour laquelle R³ est un groupe phényle, thiényle, thiazolyle, indolyle ou pyridyle éventuellement substitué par un, deux ou trois substituants -R^{4b} ou -Alk(R^{4b})ₘ.

4. Utilisation selon l'une quelconque des revendications 1 à 3, pour laquelle Ar est un groupe phényle, pyridyle, indolyle, indazolyle, benzimidazolyle, benzothiazolyle, quinoléyle, isoquinoléyle ou benzoxazolyle, dont chacun est substitué par un, deux ou trois substituants -R⁴ ou -Alk(R⁴)ₘ.

5. Utilisation selon la revendication 4, pour laquelle Ar est un groupe phényle substitué par un, deux ou trois substituants -R⁴ ou -Alk(R⁴)ₘ.

6. Utilisation selon l'une quelconque des revendications 3 à 5, pour laquelle au moins l'un de -R⁴, -Alk(R⁴)ₘ, -R^{4b} ou -Alk(R^{4b})ₘ est un groupe -X^{1a}(Alk^{a})ₚNR^{7a}R^{7b})-X^{1a}(Alk^{a})pNHet¹ ou -X^{1a}(Alk^{a})ₚAr², où X^{1a} est une liaison directe, est un atome ou un groupe de liaison choisi parmi -O-, -S-, -C(O)-, -C(S)-, -S(O)-, -S(O)₂-, -N(R⁷)-, -C(R⁷)₂-, -CON(R⁷), -OC(O)N(R⁷)-, -CSN(R⁷)-, -N(R⁷)CO-, -N(R⁷)C(O)O-, -N(R⁷)CS-, -SON(R⁷)-, SO₂N(R⁷)-, -N(R⁷)SO₂-, -N(R⁷)CON(R⁷)-, -N(R⁷)CSN(R⁷)-, N(R⁷)SON(R⁷)- ou -N(R⁷)SO₂N(R⁷),
Alk^{a} est tel que défini pour Alk,
p vaut zéro ou vaut l'entier 1,
R⁷ est un atome d'hydrogène ou un groupe alkyle en C₁₋₆,
R^{7a} et R^{7b}, qui peuvent être identiques ou différents, représentent chacun un atome d'hydrogène ou un groupe alkyle en C₁₋₆ à chaîne droite ou ramifiée, et
-NHet¹ est un groupe aminé cyclique en C₃₋₇ éventuellement substitué, contenant éventuellement un ou plusieurs atomes -O- ou -S- ou groupes -N(R⁶)-.

7. Utilisation selon la revendication 1, pour laquelle le composé est l'un des composés suivants,
5-cyano-4-phényl-N-(3,4,5-triméthoxyphényl)pyrimidine-2-amine;
5-cyano-N-[4-(2-imidazole-1-yléthyl)phényl]-4-(4-méthoxycarbonylphényl)pyrimidine-2-amine ;
5-cyano-4-(4-hydroxyméthylphényl)-N-(3,4,5-triméthoxyphényl)-pyrimidine-2-amine ;
5-cyano-4[(4-N,N-diéthylaminométhyl)phényl]-N-(3,4,5-triméthoxyphényl)pyrimidine-2-amine ;
5-cyano-4-[2-(3(R)-diméthylaminopyrrolidine-1-yl)pyridine-5-yl]-N-(indazole-5-yl)pyrimidine-2-amine ;
4-[4-(1-amino-1-méthyléthyl)phényl]-5-cyano-N-(indazole-5-yl)-pyrimidine-2-amine ;
4-[4-(1-amino-1-méthyléthyl)phényl]-5-cyano-N-(3,4,5-triméthoxyphényl)pyrimidine-2-amine ;
5-cyano-N-[4-(2,N,N-diéthylaminoéthylaminocarboxy)phényl]-4-phénylpyrimidine-2-amine ;
5-cyano-4-phényl-N-{4-[2-(2-éthylimidazole-1-yl)éthyl]phényl}pyrimidine-2-amine ;
4-[4-(1-amino-1-méthyléthyl)phényl]-5-cyano-N-4(1,2,3-triazole-1-yl)-phényl]pyrimidine-2-amine ;
4-[4-(1-amino-1-méthyléthyl)phényl]-5-cyano-N-{4-[2-(2-éthylimidazole-1-yl)éthyl]phényl}pyrimidine-2-amine ;
N-[3-(5-cyano-4-thiophène-2-ylpyrimidine-2-ylamino)phényl]-4-(4-méthylpipérazine-1-ylméthyl)benzamide ;
4-[3-(1-amino-1-méthyléthyl)phényl]-5-cyano-N-{4-[2-(2-méthylimidazole-1-yl)éthyl]phényl}pyrimidine-2-amino ;
5-cyano-4-[4-(imidazole-1-yl)méthyl]phényl-N (3,4,5-triméthoxyphényl)-pyrimidine-2-amino ;
et les sels, produits de solvatation, hydrates et N-oxydes de ce composé.

8. Utilisation selon la revendication 1, pour laquelle le composé est l'un des composés suivants :
4-[4-(1-amino-1-méthyléthyl)phényl]-5-cyano-N-[4(1,2,4-triazole-1-yl)phényl]pyrimidine-2-amine ;
5-cyano-N-[4-(1,2,4-triazole-1-yl)phényl]-4-[4-(1-diméthylamino-1-méthyléthyl)phényl]pyrimidine-2-amine;
4-[4-(1-amino-1-méthyléthyl)phényl]-5-cyano-N-(4-fluorophényl)-pyrimidine-2-amine ;
4-[4-(1-amino-1-méthyléthyl)phényl]-5-cyano-N-{4-[2-pipéridine-1-yléthyl]phényl}pyrimidine-2-amine ;
4-[4-(1-amino-1-méthyléthyl)phényl]-5-cyano-N-[4-(2-imidazole-1-yléthyl)phényl]pyrimidine-2-amine ;
4-[4-(1-amino-1-méthyléthyl)phényl]-5-cyano-N-[4-(2-morpholinoéthyl)phényl]pyrimidine-2-amine ;
4-[4-(1-amino-1-méthyléthyl)phényl]-5-cyano-N-[3-(2-morpholinoéthyl)phényl]pyrimidine-2-amine ;
5-cyano-4-[4-(1-méthyl-1-pyrrolidine-1-yléthyl)phényl]-N-(4-fluorophényl)pyrimidine-2-amine ;
5-cyano-4-{2-([2-diéthylamino)éthyl]amino]pyridine-5-yl}-N-(4-fluorophényl)pyrimidine-2-amine ;
4-[4-(1-amino-1-méthyléthyl)phényl]-5-cyano-N-(3-fluorophényl)-pyrimidine-2-amine ;
et les sels, produits de solvatation, hydrates et N-oxydes de ce composé.

9. Composé de formule (1) 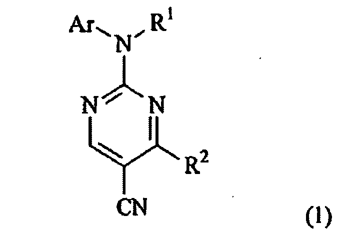 dans laquelle
Ar est un groupe aromatique en C₆₋₁₂ ou hétéroaromatique en C₁₋₁₃, ledit groupe hétéroaromatique contenant un ou deux atomes dans le cycle d'oxygène, de soufre et/d'azote, ledit groupe aromatique ou hétéroaromatique pouvant éventuellement être substitué par un ou deux ou trois substituants, dont chacun est représenté par un atome ou un groupe -R⁴ ou -Alk(R⁴)ₘ ;
R¹ est un atome d'hydrogène ou un groupe alkyle en C₁₋₆ à chaîne droite ou ramifiée ;
R² est un groupe -X¹-R³, où X¹ est une liaison directe ; et
R³ est un groupe aromatique en C₆₋₁₂ ou hétéroaromatique en C₁₋₁₃, ledit groupe hétéroaromatique contenant un ou deux atomes dans le cycle d'oxygène, de soufre et/ou d'azote, ledit groupe aromatique ou hétéroaromatique étant éventuellement substitué par un ou plusieurs substituants, dont chacun est représenté par un atome ou un groupe -R^{4b} ou -Alk(R^{4b})ₘ;
R⁴ et R^{4b} représentent chacun un atome d'halogène ou un groupe amino (-NH₂), -NHR⁵, nitro, cyano, hydroxyle (-OH), -OR⁵, formyle, carboxyle (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]2, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, -NHC(O)OR⁵, ou un groupe aromatique en C₆₋₁₂, un groupe hétéroaromatique en C₁₋₉ contenant un, deux, trois ou quatre hétéroatomes choisis parmi les atomes d'oxygène, de soufre ou d'azote, un groupe cycloaliphatique en C₃₋₁₀, ou un groupe hétérocycloaliphatique en C₃₋₁₀ contenant un, deux, trois ou quatre hétéroatomes ou groupes contenant des hétéroatomes choisis parmi les atomes -O- ou -S-, ou -N(R⁶)-, -C(O)-, -C(S)-, -S(O)- ou -S(O₂)-, lesdits groupes aromatiques ou hétéroaromatiques étant éventuellement substitués par un, deux ou trois atomes ou groupes R^{4a}, lesdits groupes cycloaliphatiques ou hétérocycloaliphatiques étant éventuellement substitués par un, deux, trois ou plusieurs substituants choisis parmi les atomes d'halogène et les groupes hydroxyle, alcoxy en C₁₋₆, thiol, alkylthio en C₁₋₆, hydroxy, alkyle en C₁₋₆, hydroxyéthyle, -CN, -NO₂, -NHR⁵ ou -N(R⁵)₂, à la condition que deux substituants R⁴ ou -Alk(R⁴)ₘ ou deux substituants R^{4b} ou -Alk(R^{4b})ₘ puissent être liés l'un à l'autre pour former un groupe alkylènedioxy en C₂₋₆ ;
Alk¹ est un groupe alkyle en C₁₋₈ à chaîne droite ou ramifiée, un groupe (aryle en C₆₋₁₂)(alkyle en C₁₋₈), un groupe aryle en C₆₋₁₂, un groupe (aryloxy en C₆₋₁₂)(alkyle en C₁₋₈), un groupe (alcanoyloxy en C₁₋₈)(alkyle en C₁₋₈) ou un groupe (aroyloxy en C₆₋₁₂)(alkyle en C₁₋₈), ces groupes étant éventuellement substitués par un ou plusieurs substituants R⁴ ;
R⁵ est -Alk(R⁴)ₘ, un groupe aromatique en C₆₋₁₂, un groupe hétéroaromatique en C₁₋₉ contenant un, deux, trois ou quatre hétéroatomes choisis parmi les atomes d'oxygène, de soufre ou d'azote, ledit groupe aromatique ou hétéroaromatique étant éventuellement substitué par un, deux ou trois atomes ou groupes R^{4a} ;
Alk est un alkylène en C₁₋₆, alcénylène en C₂₋₆ ou alcynylène en C₂₋₆ à chaîne droite ou ramifiée, éventuellement interrompu par un, deux ou trois atomes -O- ou -S- ou groupes choisis parmi -S(O)-, -S(O)₂- ou -N(R⁶)- ;
R^{4a} désigne un atome de fluor, de chlore, de brome ou d'iode, ou un groupe alkyle en C₁₋₆, alkylamino en C₁₋₆, hydroxyalkyle en C₁₋₆, hydroxyalcoxy en C₂₋₆, alkylthiol en C₁₋₆, alcoxy en C₁₋₆, cycloalcoxy en C₅₋₇, halogènoalkyle en C₁₋₆, amino(-NH₂), aminoalkyle en C₁₋₆, di(alkyle en C₁₋₆)amino, aminoalcoxy en C₁₋₆, (alkylamino en C₁₋₆)(alcoxy en C₁₋₆), di(alkyle en C₁₋₆)amino(alcoxy en C₁₋₆), imido, 1,1,3-trioxobenzo[d]-thiazolidino, nitro, cyano, hydroxyle (-OH), formyle [HC(O)-], carboxyle (-CO₂H), -CO₂Alk¹, alcanoyle en C₁₋₆, thiol (-SH), thioalkyle en C₁₋₆, -SC(NH₂)NH₂, sulfonyle (-SO₃H), alkylsulfonyle en C₁₋₆, aminosulfonyle (-SO₂NH₂), (alkyle en C₁₋₆)aminosulfonyle, di(alkyle en C₁₋₆)aminosulfonyle, phénylaminosulfonyle, carboxamido (-CONH₂), (alkyle en C₁₋₆)aminocarbonyle, di(alkyle en C₁₋₆)aminocarbonyle, sulfonylamino(-NHSO₂H), (alkyle en C₁₋₆)sulfonylamino, di(alkyle en C₁₋₆)sulfonylamino, phénylsulfonylamino, 2-nitrophénylsulfonylamino, aminosulfonylamino (-NHSO₂NH₂), (alkyle en C₁₋₆)aminosulfonylamino, di(alkyle en C₁₋₆)aminosulfonylamino, phénylaminosulfonylamino, aminocarbonylamino, (alkyle en C₁₋₆)aminocarbonylamino, di(alkyle en C₁₋₆)aminocarbonylamino, phénylaminocarbonylamino, alcanoylamino en C₁₋₆, phénylcarbonylamino, (alcanoyle en C₁₋₆)amino(alkyle en C₁₋₆), (alcoxy en C₁₋₆)carbonylamino, hétérocycloalkyle en C₃₋₆, 4-(alkyle en C₁₋₆)pipérazinyle, hétéro(cycloalkyle en C₃₋₆)(alkyle en C₁₋₆), 4-(alkyle en C₁₋₆)pipérazinyl(alkyle en C₁₋₆), hétéro(cycloalkyle en C₃₋₆)(alcoxy en C₁₋₆), hétéro(alkyle en C₃₋₆)(alkyle en C₁₋₆)amino, hétéro(cycloalkyle en C₃₋₆)amino, tetrazolyle, imidazolyle, triazolyle, imidazolyl(alkyle en C₁₋₆), imidazolyl(alcoxy en C₁₋₆), triazolyl(alcoxy en C₁₋₆), imidazolylamino(alcoxy en C₁₋₆), phénylamino, benzylamino, benzyloxy ou pyridylméthylamino ;
R⁶ est un atome d'hydrogène ou un groupe alkyle en C₁₋₆ à chaîne droite ou ramifiée ;
m vaut zéro ou est un entier valant 1, 2 ou 3 ;
et les sels, produits de solvatation, hydrates et N-oxydes de ce composé.

10. Composé de formule (1) 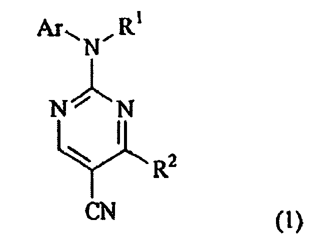 pour le traitement d'une maladie associée à la kinase KDR et/ou à la kinase FGFr,
dans laquelle
Ar est un groupe aromatique en C₆₋₁₂ ou hétéroaromatique en C₁₋₁₃, ledit groupe hétéroaromatique contenant un ou deux atomes dans le cycle d'oxygène, de soufre et/d'azote, ledit groupe aromatique ou hétéroaromatique pouvant éventuellement être substitué par un ou plusieurs substituants, dont chacun est représenté par un atome ou un groupe -R⁴ ou -Alk(R⁴)ₘ ;
R¹ est un atome d'hydrogène ou un groupe alkyle en C₁₋₆ à chaîne droite ou ramifiée ;
R² est un groupe -X¹-R³, où X¹ est une liaison directe ; et
R³ est un groupe aromatique en C₆₋₁₂ ou hétéroaromatique en C₁₋₁₃, ledit groupe hétéroaromatique contenant un ou deux atomes dans le cycle d'oxygène, de soufre et/ou d'azote, ledit groupe aromatique ou hétéroaromatique étant éventuellement substitué par un ou plusieurs substituants, dont chacun est représenté par un atome ou un groupe -R^{4b} ou -Alk(R^{4b})ₘ ;
R⁴ et R^{4b} représentent chacun un atome d'halogène ou un groupe amino (-NH₂), -NHR⁵, nitro, cyano, hydroxyle (-OH), -OR⁵, formyle, carboxyle (-CO₂H), -CO₂Alk¹, thiol (-SH), -SR⁵, -COR⁵, -CSR⁵, -SO₃H, -SO₂R⁵, -SO₂NH₂, -SO₂NHR⁵, -SO₂N[R⁵]₂, -CONH₂, -CSNH₂, -CONHR⁵, -CSNHR⁵, -CON[R⁵]₂, -CSN[R⁵]₂, -NHSO₂H, -NHSO₂R⁵, -N[SO₂R⁵]₂, -NHSO₂NH₂, -NHSO₂NHR⁵, -NHSO₂N[R⁵]₂, -NHCOR⁵, -NHCONH₂, -NHCONHR⁵, -NHCON[R⁵]₂, -NHCSR⁵, NHC(O)OR⁵, ou un groupe aromatique en C₆₋₁₂, un groupe hétéroaromatique en C₁₋₉ contenant un, deux, trois ou quatre hétéroatomes choisis parmi les atomes d'oxygène, de soufre ou d'azote, un groupe cycloaliphatique en C₃₋₁₀, ou un groupe hétérocycloaliphatique en C₃₋₁₀ contenant un, deux, trois ou quatre hétéroatomes ou groupes contenant des hétéroatomes choisis parmi les atomes -O- ou -S-, ou -N(R⁶)-, -C(O)-, -C(S)-, -S(O)- ou -S(O₂)-, lesdits groupes aromatiques ou hétéroaromatiques étant éventuellement substitués par un, deux ou trois atomes ou groupes R^{4a}, lesdits groupes cycloaliphatiques ou hétérocycloaliphatiques étant éventuellement substitués par un, deux, trois ou plusieurs substituants choisis parmi les atomes d'halogène et les groupes hydroxyle, alcoxy en C₁₋₆, thiol, alkylthio en C₁₋₆, hydroxy, alkyle en C₁₋₆, hydroxyéthyle, -CN, -NO₂, -NHR⁵ ou -N(R⁵)₂, à la condition que deux substituants R⁴ ou -Alk(R⁴)ₘ ou deux substituants R^{4b} ou -Alk(R^{4b})ₘ puissent être liés l'un à l'autre pour former un groupe alkylènedioxy en C₂₋₆ ;
Alk¹ est un groupe alkyle en C₁₋₈ à chaîne droite ou ramifiée, un groupe (aryle en C₆₋₁₂)(alkyle en C₁₋₈), un groupe aryle en C₆₋₁₂, un groupe (aryloxy en C₆₋₁₂)(alkyle en C₁₋₈), un groupe (alcanoyloxy en C₁₋₈)(alkyle en C₁₋₈) ou un groupe (aroyloxy en C₆₋₁₂)(alkyle en C₁₋₈), ces groupes étant éventuellement substitués par un ou plusieurs substituants R⁴ ;
R⁵ est -Alk(R⁴)ₘ, un groupe aromatique en C₆₋₁₂, un groupe hétéroaromatique en C₁₋₉ contenant un, deux, trois ou quatre hétéroatomes choisis parmi les atomes d'oxygène, de soufre ou d'azote, ledit groupe aromatique ou hétéroaromatique étant éventuellement substitué par un, deux ou trois atomes ou groupes R^{4a};
Alk est un alkylène en C₁₋₆, alcénylène en C₂₋₆ ou alcynylène en C₂₋₆ à chaîne droite ou ramifiée, éventuellement interrompu par un, deux ou trois atomes -O- ou -S- ou groupes choisis parmi -S(O)-, -S(O)₂- ou -N(R⁶)- ;
R^{4a} désigne un atome de fluor, de chlore, de brome ou d'iode, ou un groupe alkyle en C₁₋₆, alkylamino en C₁₋₆, hydroxyalkyle en C₁₋₆, hydroxyalcoxy en C₂₋₆, alkylthiol en C₁₋₆, alcoxy en C₁₋₆, cycloalcoxy en C₅₋₇, halogènoalkyle en C₁₋₆, amino(-NH₂), aminoalkyle en C₁₋₆, di(alkyle en C₁₋₆)amino, aminoalcoxy en C₁₋₆, (alkylamino en C₁₋₆)(alcoxy en C₁₋₆), di(alkyle en C₁₋₆)amino(alcoxy en C₁₋₆), imido, 1,1,3-trioxobenzo[d]-thiazolidino, nitro, cyano, hydroxyle (-OH), formyle [HC(O)-], carboxyle (-CO₂H), -CO₂Alk¹, alcanoyle en C₁₋₆, thiol (-SH), thioalkyle en C₁₋₆, -SC(NH₂)NH₂, sulfonyle (-SO₃H), alkylsulfonyle en C₁₋₆, aminosulfonyle (-SO₂NH₂), (alkyle en C₁₋₆)aminosulfonyle, di(alkyle en C₁₋₆)aminosulfonyle, phénylaminosulfonyle, carboxamido (-CONH₂), (alkyle en C₁₋₆)aminocarbonyle, di(alkyle en C₁₋₆)aminocarbonyle, sulfonylamino(-NHSO₂H), (alkyle en C₁₋₆)sulfonylamino, di(alkyle en C₁₋₆)sulfonylamino, phénylsulfonylamino, 2-nitrophénylsulfonylamino, aminosulfonylamino (-NHSO₂NH₂), (alkyle en C₁₋₆)aminosulfonylamino, di(alkyle en C₁₋₆)aminosulfonylamino, phénylaminosulfonylamino, aminocarbonylamino, (alkyle en C₁₋₆)aminocarbonylamino, di(alkyle en C₁₋₆)aminocarbonylamino, phénylaminocarbonylamino, alcanoylamino en C₁₋₆, phénylcarbonylamino, (alcanoyle en C₁₋₆)amino(alkyle en C₁₋₆), (alcoxy en C₁₋₆)carbonylamino, hétérocycloalkyle en C₃₋₆, 4-(alkyle en C₁₋₆)pipérazinyle, hétéro(cycloalkyle en C₃₋₆)(alkyle en C₁₋₆), 4-(alkyle en C₁₋₆)pipérazinyl(alkyle en C₁₋₆), hétéro(cycloalkyle en C₃₋₆)(alcoxy en C₁₋₆), hétéro(alkyle en C₃₋₆)(alkyle en C₁₋₆)amino, hétéro(cycloalkyle en C₃₋₆)amino, tetrazolyle, imidazolyle, triazolyle, imidazolyl(alkyle en C₁₋₆), imidazolyl(alcoxy en C₁₋₆), triazolyl(alcoxy en C₁₋₆), imidazolylamino(alcoxy en C₁₋₆), phénylamino, benzylamino, benzyloxy ou pyridylméthylamino ;
R⁶ est un atome d'hydrogène ou un groupe alkyle en C₁₋₆ à chaîne droite ou ramifiée;
m vaut zéro ou est un entier valant 1, 2 ou 3 ;
et les sels, produits de solvatation, hydrates et N-oxydes de ce composé.

11. Composé selon la revendication 9 ou 10, dans lequel R³ est un groupe phényle, thiényle, thiazolyle, indolyle ou pyridyle éventuellement substitué par un, deux ou trois substituants -R^{4b} ou -Alk(R^{4b})ₘ.

12. Composé selon l'une quelconque des revendications 9 à 11, dans lequel Ar est un groupe phényle, pyridyle, indolyle, indazolyle, benzimidazolyle, benzothiazolyle, quinoléyle, isoquinoléyle ou benzoxazolyle, dont chacun est substitué par un, deux ou trois substituants -R⁴ ou -Alk(R⁴)ₘ.

13. composé selon la revendication 12, dans lequel Ar est un groupe phényle substitué par un, deux ou trois substituants -R⁴ ou -Alk(R⁴)ₘ.

14. Composé selon l'une quelconque des revendications 11 à 13, dans lequel au moins l'un de -R⁴, -Alk(R⁴)ₘ, -R^{4b} ou -Alk(R^{4b})ₘ est un groupe -X^{1a}(Alk^{a})ₚNR^{7a}R^{7b})-X^{1a}(Alk^{a})pNHet¹ ou -X^{1a}(Alk^{a})pAr², où
X^{1a} est une liaison directe, est un atome ou un groupe de liaison choisi parmi -O-, -S-, -C(O)-, -C(S)-, -S(O)-, -S(O)₂-, -N(R⁷)-, -C(R⁷)₂-, -CON(R⁷), -OC(O)N(R⁷)-, -CSN(R⁷)-, -N(R⁷)CO-, -N(R⁷)C(O)O-, -N(R⁷)CS-, -SON(R⁷)-, SO₂N(R⁷)-, -N(R⁷)SO₂-, -N(R⁷)CON(R⁷)-, -N(R⁷)CSN(R⁷)-, N(R⁷)SON(R⁷)- ou -N(R⁷)SO₂N(R⁷),
Alk^{a} est tel que défini pour Alk,
p vaut zéro ou vaut l'entier 1,
R⁷ est un atome d'hydrogène ou un groupe alkyle en C₁₋₆,
R^{7a} et R^{7b}, qui peuvent être identiques ou différents, représentent chacun un atome d'hydrogène ou un groupe alkyle en C₁₋₆ à chaîne droite ou ramifiée, et
-NHet¹ est un groupe aminé cyclique en C₃₋₇ éventuellement substitué, contenant éventuellement un ou plusieurs atomes -O- ou -S- ou groupes -N(R⁶)-.

15. Composé selon la revendication 9 ou 10, qui est l'un des composés suivants :
5-cyano-4-phényl-N-(3,4,5-triméthoxyphényl)pyrimidine-2-amine ;
5-cyano-N-[4-(2-imidazole-1-yléthyl)phényl]-4-(4-méthoxycarbonylphényl)pyrimidine-2-amine ;
5-cyano-4-(4-hydroxyméthylphényl)-N-(3,4,5-triméthoxyphényl)-pyrimidine2-amine ;
5-cyano-4[(4-N,N-diéthylaminométhyl)phényl]-N-(3,4,5-triméthoxyphényl)pyrimidine-2-amine ;
5-cyano-4-[2-(3(R)-diméthylaminopyrrolidine-1-yl)pyridine-5-yl]-N-(indazole-5-yl)pyrimidine-2-amine ;
4-[4-(1-amino-1-méthyléthyl)phényl]-5-cyano-N-(indazole-5-yl)-pyrimidine-2-amine ;
4-[4-(1-amino-1-méthyléthyl)phényl]-5-cyano-N-(3,4,5-triméthoxyphényl)pyrimidine-2-amine ;
5-cyano-N-[4-(2,N,N-diéthylaminoéthylaminocarboxy)phényl]-4-phénylpyrimidine-2-amine ;
5-cyano-4-phényl-N- {4-[2-(2-éthylimidazole-1-yl)éthyl]phényl } - pyrimidine-2-amine ;
4-[4-(1-amino-1-méthyléthyl)phényl]-5-cyano-N-4(1,2,3-triazole-1-yl)-phényl]pyrimidine-2-amine ;
4-[4-(1-amino-1-méthyléthyl)phényl]-5-cyano-N-{4-[2-(2-éthylimidazole-1-yl)éthyl]phényl}pyrimidine-2-amine ;
N-[3-(5-cyano-4-thiophène-2-ylpyrimidine-2-ylamino)phényl]-4-(4-méthylpipérazine-1-ylméthyl)benzamide ;
4-[3-(1-amino-1-méthyléthyl)phényl]-5-cyano-N-{4-[2-(2-méthylimidazole-1-yl)éthyl]phényl}pyrimidine-2-amino ;
5-cyano-4-[4-(imidazole-1-yl)méthyl]phényl-*N*-(3,4,5-triméthoxyphényl)-pyrimidine-2-amino ;
et les sels, produits de solvatation, hydrates et N-oxydes de ce composé.

16. Composé selon la revendication 9 ou 10, qui est l'un des composé suivants :
4-[4-(1-amino-1-méthyléthyl)phényl]-5-cyano-N-[4(1,2,4-triazole-1-yl)phényl]pyrimidine-2-amine ;
5-cyano-N-[4-(1,2,4-triazole-1-yl)phényl]-4-[4-(1-diméthylamino-1-méthyléthyl)phényl]pyrimidine-2-amine ;
4-[4-(1-amino-1-méthyléthyl)phényl]-5-cyano-N-(4-fluorophényl)-pyrimidine-2-amine ;
4-[4-(1-amino-1-méthyléthyl)phényl]-5-cyano-N-{4-[2-pipéridine-1-yléthyl]phényl}pyrimidine-2-amine ;
4-[4-(1-amino-1-méthyléthyl)phényl]-5-cyano-N-[4-(2-imidazole-1-yléthyl)phényl]pyrimidine-2-amine ;
4-[4-(1-amino-1-méthyléthyl)phényl]-5-cyano-N-[4-(2-morpholinoéthyl)phényl]pyrimidine-2-amine ;
4-[4-(1-amino-1-méthyléthyl)phényl]-5-cyano-N-[3-(2-morpholinoéthyl)phényl]pyrimidine-2-amine ;
5-cyano-4-[4-(1-méthyl-1-pyrrolidine-1-yléthyl)phényl]-N-(4-fluorophényl)pyrimidine-2-amine ;
5-cyano-4-{2-([2-diéthylamino)éthyl]amino]pyridine-5-yl}-N-(4-fluorophényl)pyrimidine-2-amine ;
4-[4-(1-amino-1-méthyléthyl)phényl]-5-cyano-N-(3-fluorophényl)-pyrimidine-2-amine ;
et les sels, produits de solvatation, hydrates et N-oxydes de ce composé.

17. Composition pharmaceutique comprenant un composé selon l'une quelconque des revendications 9 à 16, en même temps qu'un ou plusieurs supports, excipients ou diluants acceptables d'un point de vue pharmaceutique.
